# EUROPEAN PATENT APPLICATION

(11) **EP 2 431 480 A2**
(43) Date of publication of application: **21.03.2012**
(21) Application number: 11179088.7
(22) Date of filing: 08.08.2003
(51) Int. Cl.: C12Q 1/34, C12Q 1/48

(54) **Methods and compositions for increasing the stress resistance of cells and organisms**

(30) Priority: 09.08.2002 US 402254 P; 22.11.2002 US 428614 P
(62) Divisional of application: 03788377.4
(71) Applicant: The President and Fellows of Harvard College, Cambridge, MA 02114 (US)
(72) Inventor: Sinclair, David A., West Roxbury, MA 02132 (US); Bitterman, Kevin J., Boston, MA 02115 (US)
(74) Representative: Crooks, Elizabeth Caroline

(57) **Abstract**

The invention provides methods and compositions for modulating the life span of eukaryotic and prokaryotic cells and for protecting cells against certain stresses, e.g., heatshock. One method comprises modulating the flux of the NAD+ salvage pathway in the cell, e.g., by modulating the level or activity of one or more proteins selected from the group consisting of NPT1, PNC1, NMA1 and NMA2. Another method comprises modulating the level of nicotinamide in the cell.

SEQUENCE LISTING

<110> PRESIDENT AND FELLOWS OF HARVARD COLLEGE
      SINCLAIR, David,A.

<120> METHODS AND COMPOSITIONS FOR EXTENDING THE LIFE SPAN AND
      INCREASING THE STRESS RESISTANCE OF CELLS AND ORGANISMS

<130> P38363EP1

<140>
<141> 2003-08-08

<150> 60/428,614
<151> 2002-11-22

<150> 60/402,254
<151> 2002-08-09

<160> 38

<170> PatentIn version 2.1

<210> 1
<211> 1290
<212> DNA
<213> Saccharomyces cerevisiae

<220>
<221> CDS
<222> (1)..(1290)

<400> 1
atg tca gaa cca gtg ata aag tct ctt ttg gac aca gac atg tac aag      48
Met Ser Glu Pro Val Ile Lys Ser Leu Leu Asp Thr Asp Met Tyr Lys
1               5                   10                  15

att acg atg cat gct gct gtc ttc act aat ttt cca gat gtt aca gtt      96
Ile Thr Met His Ala Ala Val Phe Thr Asn Phe Pro Asp Val Thr Val
            20                  25                  30

act tat aaa tat acc aac agg tcg tcc caa ttg acc ttc aat aag gaa      144
Thr Tyr Lys Tyr Thr Asn Arg Ser Ser Gln Leu Thr Phe Asn Lys Glu
        35                  40                  45

gcc att aat tgg ttg aaa gag caa ttt tcg tat ttg gga aat ttg agg      192
Ala Ile Asn Trp Leu Lys Glu Gln Phe Ser Tyr Leu Gly Asn Leu Arg
    50                  55                  60

ttc aca gaa gag gaa att gaa tac tta aaa cag gaa atc cca tat ttg      240
Phe Thr Glu Glu Glu Ile Glu Tyr Leu Lys Gln Glu Ile Pro Tyr Leu
65                  70                  75                  80

cca tcg gca tat att aag tat att agc agt tct aat tac aaa cta cac      288
Pro Ser Ala Tyr Ile Lys Tyr Ile Ser Ser Ser Asn Tyr Lys Leu His
                85                  90                  95

cct gaa gag cag att tcc ttc act tca gaa gaa atc gag ggc aag ccc      336
Pro Glu Glu Gln Ile Ser Phe Thr Ser Glu Glu Ile Glu Gly Lys Pro
            100                 105                 110

acc cac tac aaa ttg aaa att tta gtc agt ggt agt tgg aag gat act      384
Thr His Tyr Lys Leu Lys Ile Leu Val Ser Gly Ser Trp Lys Asp Thr
        115                 120                 125

atc ctt tat gag atc ccc tta ctg tcc cta ata tca gaa gcg tat ttt 432
Ile Leu Tyr Glu Ile Pro Leu Leu Ser Leu Ile Ser Glu Ala Tyr Phe
    130                 135                 140

aaa ttt gtt gac atc gac tgg gac tac gaa aac caa tta gaa caa gct 480
Lys Phe Val Asp Ile Asp Trp Asp Tyr Glu Asn Gln Leu Glu Gln Ala
145                 150                 155                 160

gag aag aag gcg gaa act ttg ttt gat aat ggt att aga ttc agt gaa 528
Glu Lys Lys Ala Glu Thr Leu Phe Asp Asn Gly Ile Arg Phe Ser Glu
                165                 170                 175

ttt ggt aca aga cgt cgt aga tct ctg aag gct caa gat cta att atg 576
Phe Gly Thr Arg Arg Arg Arg Ser Leu Lys Ala Gln Asp Leu Ile Met
            180                 185                 190

caa gga atc atg aaa gct gtg aac ggt aac cca gac aga aac aaa tcg 624
Gln Gly Ile Met Lys Ala Val Asn Gly Asn Pro Asp Arg Asn Lys Ser
        195                 200                 205

cta tta tta ggc aca tca aat att tta ttt gcc aag aaa tat gga gtc 672
Leu Leu Leu Gly Thr Ser Asn Ile Leu Phe Ala Lys Lys Tyr Gly Val
    210                 215                 220

aag cca atc ggt act gtg gct cac gag tgg gtt atg gga gtc gct tct 720
Lys Pro Ile Gly Thr Val Ala His Glu Trp Val Met Gly Val Ala Ser
225                 230                 235                 240

att agt gaa gat tat ttg cat gcc aat aaa aat gca atg gat tgt tgg 768
Ile Ser Glu Asp Tyr Leu His Ala Asn Lys Asn Ala Met Asp Cys Trp
                245                 250                 255

atc aat act ttt ggt gca aaa aat gct ggt tta gca tta acg gat act 816
Ile Asn Thr Phe Gly Ala Lys Asn Ala Gly Leu Ala Leu Thr Asp Thr
            260                 265                 270

ttt gga act gat gac ttt tta aaa tca ttc cgt cca cca tat tct gat 864
Phe Gly Thr Asp Asp Phe Leu Lys Ser Phe Arg Pro Pro Tyr Ser Asp
        275                 280                 285

gct tac gtc ggt gtt aga caa gat tct gga gac cca gtt gag tat acc 912
Ala Tyr Val Gly Val Arg Gln Asp Ser Gly Asp Pro Val Glu Tyr Thr
    290                 295                 300

aaa aag att tcc cac cat tac cat gac gtg ttg aaa ttg cct aaa ttc 960
Lys Lys Ile Ser His His Tyr His Asp Val Leu Lys Leu Pro Lys Phe
305                 310                 315                 320

tcg aag att atc tgt tat tcc gat tct ttg aac gtc gaa aag gca ata 1008
Ser Lys Ile Ile Cys Tyr Ser Asp Ser Leu Asn Val Glu Lys Ala Ile
                325                 330                 335

act tac tcc cat gca gct aaa gag aat gga atg cta gcc aca ttc ggt 1056
Thr Tyr Ser His Ala Ala Lys Glu Asn Gly Met Leu Ala Thr Phe Gly
            340                 345                 350

att ggc aca aac ttt act aat gat ttt cgt aag aag tca gaa ccc cag 1104
Ile Gly Thr Asn Phe Thr Asn Asp Phe Arg Lys Lys Ser Glu Pro Gln
        355                 360                 365

gtt aaa agt gag ccg tta aac atc gtt atc aaa cta tta gaa gta aat 1152
Val Lys Ser Glu Pro Leu Asn Ile Val Ile Lys Leu Leu Glu Val Asn
    370                 375                 380

ggt aat cac gct atc aaa att tct gat aac tta ggt aaa aat atg gga 1200

Gly Asn His Ala Ile Lys Ile Ser Asp Asn Leu Gly Lys Asn Met Gly
385                 390                 395                 400

gat cct gcc act gtg aag aga gtg aaa gag gaa ttg gga tat act gaa      1248
Asp Pro Ala Thr Val Lys Arg Val Lys Glu Glu Leu Gly Tyr Thr Glu
                405                 410                 415

cga agt tgg agt ggt gat aac gaa gcg cac aga tgg acc taa              1290
Arg Ser Trp Ser Gly Asp Asn Glu Ala His Arg Trp Thr
            420                 425

<210> 2
<211> 429
<212> PRT
<213> Saccharomyces cerevisiae

<400> 2
Met Ser Glu Pro Val Ile Lys Ser Leu Leu Asp Thr Asp Met Tyr Lys
1               5                   10                  15

Ile Thr Met His Ala Ala Val Phe Thr Asn Phe Pro Asp Val Thr Val
            20                  25                  30

Thr Tyr Lys Tyr Thr Asn Arg Ser Ser Gln Leu Thr Phe Asn Lys Glu
        35                  40                  45

Ala Ile Asn Trp Leu Lys Glu Gln Phe Ser Tyr Leu Gly Asn Leu Arg
    50                  55                  60

Phe Thr Glu Glu Glu Ile Glu Tyr Leu Lys Gln Glu Ile Pro Tyr Leu
65                  70                  75                  80

Pro Ser Ala Tyr Ile Lys Tyr Ile Ser Ser Ser Asn Tyr Lys Leu His
                85                  90                  95

Pro Glu Glu Gln Ile Ser Phe Thr Ser Glu Glu Ile Glu Gly Lys Pro
            100                 105                 110

Thr His Tyr Lys Leu Lys Ile Leu Val Ser Gly Ser Trp Lys Asp Thr
        115                 120                 125

Ile Leu Tyr Glu Ile Pro Leu Leu Ser Leu Ile Ser Glu Ala Tyr Phe
    130                 135                 140

Lys Phe Val Asp Ile Asp Trp Asp Tyr Glu Asn Gln Leu Glu Gln Ala
145                 150                 155                 160

Glu Lys Lys Ala Glu Thr Leu Phe Asp Asn Gly Ile Arg Phe Ser Glu
                165                 170                 175

Phe Gly Thr Arg Arg Arg Arg Ser Leu Lys Ala Gln Asp Leu Ile Met
            180                 185                 190

Gln Gly Ile Met Lys Ala Val Asn Gly Asn Pro Asp Arg Asn Lys Ser
        195                 200                 205

Leu Leu Leu Gly Thr Ser Asn Ile Leu Phe Ala Lys Lys Tyr Gly Val
    210                 215                 220

Lys Pro Ile Gly Thr Val Ala His Glu Trp Val Met Gly Val Ala Ser
225                 230                 235                 240

Ile Ser Glu Asp Tyr Leu His Ala Asn Lys Asn Ala Met Asp Cys Trp
                245                 250                 255

Ile Asn Thr Phe Gly Ala Lys Asn Ala Gly Leu Ala Leu Thr Asp Thr
            260                 265                 270

Phe Gly Thr Asp Asp Phe Leu Lys Ser Phe Arg Pro Pro Tyr Ser Asp
        275                 280                 285

Ala Tyr Val Gly Val Arg Gln Asp Ser Gly Asp Pro Val Glu Tyr Thr
    290                 295                 300

Lys Lys Ile Ser His His Tyr His Asp Val Leu Lys Leu Pro Lys Phe
305                 310                 315                 320

Ser Lys Ile Ile Cys Tyr Ser Asp Ser Leu Asn Val Glu Lys Ala Ile
                325                 330                 335

Thr Tyr Ser His Ala Ala Lys Glu Asn Gly Met Leu Ala Thr Phe Gly
            340                 345                 350

Ile Gly Thr Asn Phe Thr Asn Asp Phe Arg Lys Lys Ser Glu Pro Gln
        355                 360                 365

Val Lys Ser Glu Pro Leu Asn Ile Val Ile Lys Leu Leu Glu Val Asn
    370                 375                 380

Gly Asn His Ala Ile Lys Ile Ser Asp Asn Leu Gly Lys Asn Met Gly
385                 390                 395                 400

Asp Pro Ala Thr Val Lys Arg Val Lys Glu Glu Leu Gly Tyr Thr Glu
                405                 410                 415

Arg Ser Trp Ser Gly Asp Asn Glu Ala His Arg Trp Thr
            420                 425

<210> 3
<211> 651
<212> DNA
<213> Saccharomyces cerevisiae

<220>
<221> CDS
<222> (1)..(651)

<400> 3
atg aag act tta att gtt gtt gat atg caa aat gat ttt att tca cct 48
Met Lys Thr Leu Ile Val Val Asp Met Gln Asn Asp Phe Ile Ser Pro
1               5                   10                  15

tta ggt tcc ttg act gtt cca aaa ggt gag gaa tta atc aat cct atc 96
Leu Gly Ser Leu Thr Val Pro Lys Gly Glu Glu Leu Ile Asn Pro Ile
            20                  25                  30

tcg gat ttg atg caa gat gct gat aga gac tgg cac agg att gtg gtc 144
Ser Asp Leu Met Gln Asp Ala Asp Arg Asp Trp His Arg Ile Val Val
        35                  40                  45

acc aga gat tgg cac cct tcc aga cat att tcg ttc gca aag aac cat 192
Thr Arg Asp Trp His Pro Ser Arg His Ile Ser Phe Ala Lys Asn His
    50                  55                  60

aaa gat aaa gaa ccc tat tca aca tac acc tac cac tct cca agg cca 240
Lys Asp Lys Glu Pro Tyr Ser Thr Tyr Thr Tyr His Ser Pro Arg Pro
65                  70                  75                  80

ggc gat gat tcc acg caa gag ggt att ttg tgg ccc gta cac tgt gtg 288
Gly Asp Asp Ser Thr Gln Glu Gly Ile Leu Trp Pro Val His Cys Val
                85                  90                  95

aaa aac acc tgg ggt agt caa ttg gtt gac caa ata atg gac caa gtg 336
Lys Asn Thr Trp Gly Ser Gln Leu Val Asp Gln Ile Met Asp Gln Val
            100                 105                 110

gtc act aag cat att aag att gtc gac aag ggt ttc ttg act gac cgt 384
Val Thr Lys His Ile Lys Ile Val Asp Lys Gly Phe Leu Thr Asp Arg
        115                 120                 125

gaa tac tac tcc gcc ttc cac gac atc tgg aac ttc cat aag acc gac 432
Glu Tyr Tyr Ser Ala Phe His Asp Ile Trp Asn Phe His Lys Thr Asp
    130                 135                 140

atg aac aag tac tta gaa aag cat cat aca gac gag gtt tac att gtc 480
Met Asn Lys Tyr Leu Glu Lys His His Thr Asp Glu Val Tyr Ile Val
145                 150                 155                 160

ggt gta gct ttg gag tat tgt gtc aaa gcc acc gcc att tcc gct gca 528
Gly Val Ala Leu Glu Tyr Cys Val Lys Ala Thr Ala Ile Ser Ala Ala
                165                 170                 175

gaa cta ggt tat aag acc act gtc ctg ctg gat tac aca aga ccc atc 576
Glu Leu Gly Tyr Lys Thr Thr Val Leu Leu Asp Tyr Thr Arg Pro Ile
            180                 185                 190

agc gat gat ccc gaa gtc atc aat aag gtt aag gaa gag ttg aag gcc 624
Ser Asp Asp Pro Glu Val Ile Asn Lys Val Lys Glu Glu Leu Lys Ala
        195                 200                 205

cac aac atc aat gtc gtg gat aaa taa 651
His Asn Ile Asn Val Val Asp Lys
    210                 215


<210> 4
<211> 216

<212> PRT
<213> Saccharomyces cerevisiae

<400> 4
Met Lys Thr Leu Ile Val Val Asp Met Gln Asn Asp Phe Ile Ser Pro
1               5                   10                  15

Leu Gly Ser Leu Thr Val Pro Lys Gly Glu Glu Leu Ile Asn Pro Ile
            20                  25                  30

Ser Asp Leu Met Gln Asp Ala Asp Arg Asp Trp His Arg Ile Val Val
        35                  40                  45

Thr Arg Asp Trp His Pro Ser Arg His Ile Ser Phe Ala Lys Asn His
    50                  55                  60

Lys Asp Lys Glu Pro Tyr Ser Thr Tyr Thr Tyr His Ser Pro Arg Pro
65                  70                  75                  80

Gly Asp Asp Ser Thr Gln Glu Gly Ile Leu Trp Pro Val His Cys Val
                85                  90                  95

Lys Asn Thr Trp Gly Ser Gln Leu Val Asp Gln Ile Met Asp Gln Val
            100                 105                 110

Val Thr Lys His Ile Lys Ile Val Asp Lys Gly Phe Leu Thr Asp Arg
        115                 120                 125

Glu Tyr Tyr Ser Ala Phe His Asp Ile Trp Asn Phe His Lys Thr Asp
    130                 135                 140

Met Asn Lys Tyr Leu Glu Lys His His Thr Asp Glu Val Tyr Ile Val
145                 150                 155                 160

Gly Val Ala Leu Glu Tyr Cys Val Lys Ala Thr Ala Ile Ser Ala Ala
                165                 170                 175

Glu Leu Gly Tyr Lys Thr Thr Val Leu Leu Asp Tyr Thr Arg Pro Ile
            180                 185                 190

Ser Asp Asp Pro Glu Val Ile Asn Lys Val Lys Glu Glu Leu Lys Ala
        195                 200                 205

His Asn Ile Asn Val Val Asp Lys
    210                 215

<210> 5
<211> 1206
<212> DNA
<213> Saccharomyces cerevisiae

<220>
<221> CDS
<222> (1)..(1206)

<400> 5
atg gat ccc aca aga gct ccg gat ttc aaa ccg cca tct gca gac gag      48
Met Asp Pro Thr Arg Ala Pro Asp Phe Lys Pro Pro Ser Ala Asp Glu
1               5                   10                  15

gaa ttg att cct cca ccc gac ccg gaa tct aaa att ccc aaa tct att      96
Glu Leu Ile Pro Pro Pro Asp Pro Glu Ser Lys Ile Pro Lys Ser Ile
            20                  25                  30

cca att att cca tac gtc tta gcc gat gcg aat tcc tct ata gat gca      144
Pro Ile Ile Pro Tyr Val Leu Ala Asp Ala Asn Ser Ser Ile Asp Ala
        35                  40                  45

cct ttt aat att aag agg aag aaa aag cat cct aag cat cat cat cac      192
Pro Phe Asn Ile Lys Arg Lys Lys Lys His Pro Lys His His His His
    50                  55                  60

cat cat cac agt cgt aaa gaa ggc aat gat aaa aaa cat cag cat att      240
His His His Ser Arg Lys Glu Gly Asn Asp Lys Lys His Gln His Ile
65                  70                  75                  80

cca ttg aac caa gac gac ttt caa cca ctt tcc gca gaa gtg tct tcc      288
Pro Leu Asn Gln Asp Asp Phe Gln Pro Leu Ser Ala Glu Val Ser Ser
                85                  90                  95

gaa gat gat gac gcg gat ttt aga tcc aag gag aga tac ggt tca gat      336
Glu Asp Asp Asp Ala Asp Phe Arg Ser Lys Glu Arg Tyr Gly Ser Asp
            100                 105                 110

tca acc aca gaa tca gaa act aga ggt gtt cag aaa tat cag att gct      384
Ser Thr Thr Glu Ser Glu Thr Arg Gly Val Gln Lys Tyr Gln Ile Ala
        115                 120                 125

gat tta gaa gaa gtt cca cat gga atc gtt cgt caa gca aga acc ttg      432
Asp Leu Glu Glu Val Pro His Gly Ile Val Arg Gln Ala Arg Thr Leu
    130                 135                 140

gaa gac tac gaa ttc ccc tca cac aga tta tcg aaa aaa tta ctg gat      480
Glu Asp Tyr Glu Phe Pro Ser His Arg Leu Ser Lys Lys Leu Leu Asp
145                 150                 155                 160

cca aat aaa ctg ccg tta gta ata gta gca tgt ggg tct ttt tca cca      528
Pro Asn Lys Leu Pro Leu Val Ile Val Ala Cys Gly Ser Phe Ser Pro
                165                 170                 175

atc acc tac ttg cat cta aga atg ttt gaa atg gct tta gat gca atc      576
Ile Thr Tyr Leu His Leu Arg Met Phe Glu Met Ala Leu Asp Ala Ile
            180                 185                 190

tct gaa caa aca agg ttt gaa gtc ata ggt gga tat tac tcc cct gtt      624
Ser Glu Gln Thr Arg Phe Glu Val Ile Gly Gly Tyr Tyr Ser Pro Val
        195                 200                 205

agt gat aac tat caa aag caa ggc ttg gcc cca tcc tac cat aga gta      672
Ser Asp Asn Tyr Gln Lys Gln Gly Leu Ala Pro Ser Tyr His Arg Val
    210                 215                 220

cgt atg tgt gaa ttg gcc tgc gaa aga acc tca tct tgg ttg atg gtg      720
Arg Met Cys Glu Leu Ala Cys Glu Arg Thr Ser Ser Trp Leu Met Val
225                 230                 235                 240

gat gca tgg gag tca ttg caa cct tca tac aca aga act gcc aag gtc      768
Asp Ala Trp Glu Ser Leu Gln Pro Ser Tyr Thr Arg Thr Ala Lys Val
                245                 250                 255

ttg gat cat ttc aat cac gaa atc aat att aag aga ggt ggt gta gct      816
Leu Asp His Phe Asn His Glu Ile Asn Ile Lys Arg Gly Gly Val Ala
            260                 265                 270

act gtt act gga gaa aaa att ggt gtg aaa ata atg ttg ctg gct ggt      864
Thr Val Thr Gly Glu Lys Ile Gly Val Lys Ile Met Leu Leu Ala Gly
        275                 280                 285

ggt gac cta ata gag tca atg ggt gaa cca aac gtt tgg gcg gac gcc      912
Gly Asp Leu Ile Glu Ser Met Gly Glu Pro Asn Val Trp Ala Asp Ala
    290                 295                 300

gat tta cat cac att ctc ggt aat tac ggt tgt ttg att gtc gaa cgt      960
Asp Leu His His Ile Leu Gly Asn Tyr Gly Cys Leu Ile Val Glu Arg
305                 310                 315                 320

act ggt tct gat gta agg tct ttt ttg tta tcc cat gat att atg tat      1008
Thr Gly Ser Asp Val Arg Ser Phe Leu Leu Ser His Asp Ile Met Tyr
                325                 330                 335

gaa cat aga agg aat att ctt atc atc aag caa ctc atc tat aat gat      1056
Glu His Arg Arg Asn Ile Leu Ile Ile Lys Gln Leu Ile Tyr Asn Asp
            340                 345                 350

att tct tcc acg aaa gtt cgt cta ttt atc aga cgc gcc atg tct gta      1104
Ile Ser Ser Thr Lys Val Arg Leu Phe Ile Arg Arg Ala Met Ser Val
        355                 360                 365

caa tat ttg tta cct aat tcg gtc atc agg tat atc caa gaa cat aga      1152
Gln Tyr Leu Leu Pro Asn Ser Val Ile Arg Tyr Ile Gln Glu His Arg
    370                 375                 380

cta tat gtg gac caa acc gaa cct gtt aag caa gtt ctt gga aac aaa      1200
Leu Tyr Val Asp Gln Thr Glu Pro Val Lys Gln Val Leu Gly Asn Lys
385                 390                 395                 400

gaa tga                                                              1206
Glu

<210> 6
<211> 401
<212> PRT
<213> Saccharomyces cerevisiae

<400> 6
Met Asp Pro Thr Arg Ala Pro Asp Phe Lys Pro Pro Ser Ala Asp Glu
1               5                   10                  15

Glu Leu Ile Pro Pro Pro Asp Pro Glu Ser Lys Ile Pro Lys Ser Ile
            20                  25                  30

Pro Ile Ile Pro Tyr Val Leu Ala Asp Ala Asn Ser Ser Ile Asp Ala
        35                  40                  45

Pro Phe Asn Ile Lys Arg Lys Lys Lys His Pro Lys His His His His
    50                  55                  60

His His His Ser Arg Lys Glu Gly Asn Asp Lys Lys His Gln His Ile
65                  70                  75                  80

Pro Leu Asn Gln Asp Asp Phe Gln Pro Leu Ser Ala Glu Val Ser Ser
                85                  90                  95

Glu Asp Asp Asp Ala Asp Phe Arg Ser Lys Glu Arg Tyr Gly Ser Asp
            100                 105                 110

Ser Thr Thr Glu Ser Glu Thr Arg Gly Val Gln Lys Tyr Gln Ile Ala
        115                 120                 125

Asp Leu Glu Glu Val Pro His Gly Ile Val Arg Gln Ala Arg Thr Leu
    130                 135                 140

Glu Asp Tyr Glu Phe Pro Ser His Arg Leu Ser Lys Lys Leu Leu Asp
145                 150                 155                 160

Pro Asn Lys Leu Pro Leu Val Ile Val Ala Cys Gly Ser Phe Ser Pro
                165                 170                 175

Ile Thr Tyr Leu His Leu Arg Met Phe Glu Met Ala Leu Asp Ala Ile
            180                 185                 190

Ser Glu Gln Thr Arg Phe Glu Val Ile Gly Gly Tyr Tyr Ser Pro Val
        195                 200                 205

Ser Asp Asn Tyr Gln Lys Gln Gly Leu Ala Pro Ser Tyr His Arg Val
    210                 215                 220

Arg Met Cys Glu Leu Ala Cys Glu Arg Thr Ser Ser Trp Leu Met Val
225                 230                 235                 240

Asp Ala Trp Glu Ser Leu Gln Pro Ser Tyr Thr Arg Thr Ala Lys Val
                245                 250                 255

Leu Asp His Phe Asn His Glu Ile Asn Ile Lys Arg Gly Gly Val Ala
            260                 265                 270

Thr Val Thr Gly Glu Lys Ile Gly Val Lys Ile Met Leu Leu Ala Gly
        275                 280                 285

Gly Asp Leu Ile Glu Ser Met Gly Glu Pro Asn Val Trp Ala Asp Ala
    290                 295                 300

Asp Leu His His Ile Leu Gly Asn Tyr Gly Cys Leu Ile Val Glu Arg
305                 310                 315                 320

Thr Gly Ser Asp Val Arg Ser Phe Leu Leu Ser His Asp Ile Met Tyr
                325                 330                 335

Glu His Arg Arg Asn Ile Leu Ile Ile Lys Gln Leu Ile Tyr Asn Asp
            340                 345                 350

Ile Ser Ser Thr Lys Val Arg Leu Phe Ile Arg Arg Ala Met Ser Val
        355                 360                 365

Gln Tyr Leu Leu Pro Asn Ser Val Ile Arg Tyr Ile Gln Glu His Arg
    370                 375                 380

Leu Tyr Val Asp Gln Thr Glu Pro Val Lys Gln Val Leu Gly Asn Lys
385                 390                 395                 400

Glu

<210> 7
<211> 1188
<212> DNA
<213> Saccharomyces cerevisiae

<220>
<221> CDS
<222> (1)..(1188)

<400> 7
atg gat ccc acc aaa gca ccc gat ttt aaa ccg cca cag cca aat gaa      48
Met Asp Pro Thr Lys Ala Pro Asp Phe Lys Pro Pro Gln Pro Asn Glu
1               5                   10                  15

gaa cta caa cca ccg cca gat cca aca cat acg ata cca aaa tct gga      96
Glu Leu Gln Pro Pro Pro Asp Pro Thr His Thr Ile Pro Lys Ser Gly
            20                  25                  30

ccc ata gtt cca tat gtt tta gct gat tat aat tct tcg atc gat gct      144
Pro Ile Val Pro Tyr Val Leu Ala Asp Tyr Asn Ser Ser Ile Asp Ala
        35                  40                  45

cct ttc aat ctc gac att tac aaa acc ctg tcg tca agg aaa aaa aac      192
Pro Phe Asn Leu Asp Ile Tyr Lys Thr Leu Ser Ser Arg Lys Lys Asn
    50                  55                  60

gcc aac tca agc aac cga atg gac cat att cca tta aat act agt gac      240
Ala Asn Ser Ser Asn Arg Met Asp His Ile Pro Leu Asn Thr Ser Asp
65                  70                  75                  80

ttc cag cca cta tct cgg gat gta tca tcg gag gag gaa agt gaa ggg      288
Phe Gln Pro Leu Ser Arg Asp Val Ser Ser Glu Glu Glu Ser Glu Gly
                85                  90                  95

caa tcg aat gga att gac gct act cta cag gat gtt acg atg act ggg      336
Gln Ser Asn Gly Ile Asp Ala Thr Leu Gln Asp Val Thr Met Thr Gly
            100                 105                 110

aat ttg ggg gta ctg aag agc caa att gct gat ttg gaa gaa gtt cct      384
Asn Leu Gly Val Leu Lys Ser Gln Ile Ala Asp Leu Glu Glu Val Pro

115                 120                 125

cac aca att gta aga caa gcc aga act att gaa gat tac gaa ttt cct      432
His Thr Ile Val Arg Gln Ala Arg Thr Ile Glu Asp Tyr Glu Phe Pro
    130                 135                 140

gta cac aga ttg acg aaa aag tta caa gat cct gaa aaa ctg cct ctg      480
Val His Arg Leu Thr Lys Lys Leu Gln Asp Pro Glu Lys Leu Pro Leu
145                 150                 155                 160

atc atc gtt gct tgt gga tca ttt tct ccc ata aca tac cta cat ttg      528
Ile Ile Val Ala Cys Gly Ser Phe Ser Pro Ile Thr Tyr Leu His Leu
                165                 170                 175

aga atg ttt gaa atg gct tta gat gat atc aat gag caa acg cgt ttt      576
Arg Met Phe Glu Met Ala Leu Asp Asp Ile Asn Glu Gln Thr Arg Phe
            180                 185                 190

gaa gtg gtt ggt ggt tat ttt tct cca gta agt gat aac tat caa aag      624
Glu Val Val Gly Gly Tyr Phe Ser Pro Val Ser Asp Asn Tyr Gln Lys
        195                 200                 205

cga ggg tta gcc cca gct tat cat cgt gtc cgc atg tgc gaa tta gca      672
Arg Gly Leu Ala Pro Ala Tyr His Arg Val Arg Met Cys Glu Leu Ala
    210                 215                 220

tgc gag cgg aca tca tct tgg tta atg gtt gat gcc tgg gaa tct tta      720
Cys Glu Arg Thr Ser Ser Trp Leu Met Val Asp Ala Trp Glu Ser Leu
225                 230                 235                 240

caa tca agt tat aca agg aca gca aaa gtc ttg gac cat ttc aat cat      768
Gln Ser Ser Tyr Thr Arg Thr Ala Lys Val Leu Asp His Phe Asn His
                245                 250                 255

gaa ata aat atc aag aga ggt gga atc atg act gta gat ggt gaa aaa      816
Glu Ile Asn Ile Lys Arg Gly Gly Ile Met Thr Val Asp Gly Glu Lys
            260                 265                 270

atg ggc gta aaa atc atg tta ttg gca ggc ggt gat ctt atc gaa tcc      864
Met Gly Val Lys Ile Met Leu Leu Ala Gly Gly Asp Leu Ile Glu Ser
        275                 280                 285

atg ggc gag cct cat gtg tgg gct gat tca gac ctg cac cat att ttg      912
Met Gly Glu Pro His Val Trp Ala Asp Ser Asp Leu His His Ile Leu
    290                 295                 300

ggt aat tat gga tgt ttg atc gtg gaa agg act ggt tct gat gtt agg      960
Gly Asn Tyr Gly Cys Leu Ile Val Glu Arg Thr Gly Ser Asp Val Arg
305                 310                 315                 320

tcc ttc ttg ctt tcc cat gat atc atg tat gaa cac aga aga aat atc     1008
Ser Phe Leu Leu Ser His Asp Ile Met Tyr Glu His Arg Arg Asn Ile
                325                 330                 335

ctt att atc aaa caa ctt att tac aat gat att tcc tct acg aaa gtg     1056
Leu Ile Ile Lys Gln Leu Ile Tyr Asn Asp Ile Ser Ser Thr Lys Val
            340                 345                 350

cgg ctt ttc atc aga cgt gga atg tca gtt caa tat ctt ctt cca aac     1104
Arg Leu Phe Ile Arg Arg Gly Met Ser Val Gln Tyr Leu Leu Pro Asn
        355                 360                 365

tct gtc atc cgt tac atc caa gag tat aat cta tac att aat caa agt     1152
Ser Val Ile Arg Tyr Ile Gln Glu Tyr Asn Leu Tyr Ile Asn Gln Ser
    370                 375                 380

gaa ccg gtc aag cag gtc ttg gat agc aaa gag tga                    1188
Glu Pro Val Lys Gln Val Leu Asp Ser Lys Glu
385                 390                 395

<210> 8
<211> 395
<212> PRT
<213> Saccharomyces cerevisiae

<400> 8
Met Asp Pro Thr Lys Ala Pro Asp Phe Lys Pro Pro Gln Pro Asn Glu
1               5                   10                  15

Glu Leu Gln Pro Pro Pro Asp Pro Thr His Thr Ile Pro Lys Ser Gly
            20                  25                  30

Pro Ile Val Pro Tyr Val Leu Ala Asp Tyr Asn Ser Ser Ile Asp Ala
        35                  40                  45

Pro Phe Asn Leu Asp Ile Tyr Lys Thr Leu Ser Ser Arg Lys Lys Asn
    50                  55                  60

Ala Asn Ser Ser Asn Arg Met Asp His Ile Pro Leu Asn Thr Ser Asp
65                  70                  75                  80

Phe Gln Pro Leu Ser Arg Asp Val Ser Ser Glu Glu Glu Ser Glu Gly
                85                  90                  95

Gln Ser Asn Gly Ile Asp Ala Thr Leu Gln Asp Val Thr Met Thr Gly
            100                 105                 110

Asn Leu Gly Val Leu Lys Ser Gln Ile Ala Asp Leu Glu Glu Val Pro
        115                 120                 125

His Thr Ile Val Arg Gln Ala Arg Thr Ile Glu Asp Tyr Glu Phe Pro
    130                 135                 140

Val His Arg Leu Thr Lys Lys Leu Gln Asp Pro Glu Lys Leu Pro Leu
145                 150                 155                 160

Ile Ile Val Ala Cys Gly Ser Phe Ser Pro Ile Thr Tyr Leu His Leu
                165                 170                 175

Arg Met Phe Glu Met Ala Leu Asp Asp Ile Asn Glu Gln Thr Arg Phe
            180                 185                 190

Glu Val Val Gly Gly Tyr Phe Ser Pro Val Ser Asp Asn Tyr Gln Lys
        195                 200                 205

Arg Gly Leu Ala Pro Ala Tyr His Arg Val Arg Met Cys Glu Leu Ala

210                 215                 220

Cys Glu Arg Thr Ser Ser Trp Leu Met Val Asp Ala Trp Glu Ser Leu
225                 230                 235                 240

Gln Ser Ser Tyr Thr Arg Thr Ala Lys Val Leu Asp His Phe Asn His
                245                 250                 255

Glu Ile Asn Ile Lys Arg Gly Gly Ile Met Thr Val Asp Gly Glu Lys
            260                 265                 270

Met Gly Val Lys Ile Met Leu Leu Ala Gly Gly Asp Leu Ile Glu Ser
        275                 280                 285

Met Gly Glu Pro His Val Trp Ala Asp Ser Asp Leu His His Ile Leu
    290                 295                 300

Gly Asn Tyr Gly Cys Leu Ile Val Glu Arg Thr Gly Ser Asp Val Arg
305                 310                 315                 320

Ser Phe Leu Leu Ser His Asp Ile Met Tyr Glu His Arg Arg Asn Ile
                325                 330                 335

Leu Ile Ile Lys Gln Leu Ile Tyr Asn Asp Ile Ser Ser Thr Lys Val
            340                 345                 350

Arg Leu Phe Ile Arg Arg Gly Met Ser Val Gln Tyr Leu Leu Pro Asn
        355                 360                 365

Ser Val Ile Arg Tyr Ile Gln Glu Tyr Asn Leu Tyr Ile Asn Gln Ser
    370                 375                 380

Glu Pro Val Lys Gln Val Leu Asp Ser Lys Glu
385                 390                 395

<210> 9
<211> 952
<212> DNA
<213> Homo sapiens

<220>
<221> CDS
<222> (118)..(912)

<400> 9
tgaactctgg atgctgttag cctgagactc aggaagacaa cttctgcagg gtcactccct      60

ggcttctgga ggaaagagaa ggagggcagt gctccagtgg tacagaagtg agacata        117

atg gaa tca ggc ttc acc tcc aag gac acc tat cta agc cat ttt aac       165
Met Glu Ser Gly Phe Thr Ser Lys Asp Thr Tyr Leu Ser His Phe Asn
1               5                   10                  15

cct cgg gat tac cta gaa aaa tat tac aag ttt ggt tct agg cac tct     213
Pro Arg Asp Tyr Leu Glu Lys Tyr Tyr Lys Phe Gly Ser Arg His Ser
            20                  25                  30

gca gaa agc cag att ctt aag cac ctt ctg aaa aat ctt ttc aag ata     261
Ala Glu Ser Gln Ile Leu Lys His Leu Leu Lys Asn Leu Phe Lys Ile
        35                  40                  45

ttc tgc cta gac ggt gtg aag gga gac ctg ctg att gac atc ggc tct     309
Phe Cys Leu Asp Gly Val Lys Gly Asp Leu Leu Ile Asp Ile Gly Ser
    50                  55                  60

ggc ccc act atc tat cag ctc ctc tct gct tgt gaa tcc ttt aag gag     357
Gly Pro Thr Ile Tyr Gln Leu Leu Ser Ala Cys Glu Ser Phe Lys Glu
65                  70                  75                  80

atc gtc gtc act gac tac tca gac cag aac ctg cag gag ctg gag aag     405
Ile Val Val Thr Asp Tyr Ser Asp Gln Asn Leu Gln Glu Leu Glu Lys
                85                  90                  95

tgg ctg aag aaa gag cca gag gcc ttt gac tgg tcc cca gtg gtg acc     453
Trp Leu Lys Lys Glu Pro Glu Ala Phe Asp Trp Ser Pro Val Val Thr
            100                 105                 110

tat gtg tgt gat ctt gaa ggg aac aga gtc aag ggt cca gag aag gag     501
Tyr Val Cys Asp Leu Glu Gly Asn Arg Val Lys Gly Pro Glu Lys Glu
        115                 120                 125

gag aag ttg aga cag gcg gtc aag cag gtg ctg aag tgt gat gtg act     549
Glu Lys Leu Arg Gln Ala Val Lys Gln Val Leu Lys Cys Asp Val Thr
    130                 135                 140

cag agc cag cca ctg ggg gcc gtc ccc tta ccc ccg gct gac tgc gtg     597
Gln Ser Gln Pro Leu Gly Ala Val Pro Leu Pro Pro Ala Asp Cys Val
145                 150                 155                 160

ctc agc aca ctg tgt ctg gat gcc gcc tgc cca gac ctc ccc acc tac     645
Leu Ser Thr Leu Cys Leu Asp Ala Ala Cys Pro Asp Leu Pro Thr Tyr
                165                 170                 175

tgc agg gcg ctc agg aac ctc ggc agc cta ctg aag cca ggg ggc ttc     693
Cys Arg Ala Leu Arg Asn Leu Gly Ser Leu Leu Lys Pro Gly Gly Phe
            180                 185                 190

ctg gtg atc atg gat gcg ctc aag agc agc tac tac atg att ggt gag     741
Leu Val Ile Met Asp Ala Leu Lys Ser Ser Tyr Tyr Met Ile Gly Glu
        195                 200                 205

cag aag ttc tcc agc ctc ccc ctg ggc cgg gag gca gta gag gct gct     789
Gln Lys Phe Ser Ser Leu Pro Leu Gly Arg Glu Ala Val Glu Ala Ala
    210                 215                 220

gtg aaa gag gct ggc tac aca atc gaa tgg ttt gag gtg atc tcg caa     837
Val Lys Glu Ala Gly Tyr Thr Ile Glu Trp Phe Glu Val Ile Ser Gln
225                 230                 235                 240

agt tat tct tcc acc atg gcc aac aac gaa gga ctt ttc tcc ctg gtg     885
Ser Tyr Ser Ser Thr Met Ala Asn Asn Glu Gly Leu Phe Ser Leu Val
                245                 250                 255

gcg agg aag ctg agc aga ccc ctg tga tgcctgtgac ctcaattaaa           932
Ala Arg Lys Leu Ser Arg Pro Leu
            260

gcaattcctt tgacctgtca                                               952

<210> 10
<211> 264
<212> PRT
<213> Homo sapiens

<400> 10
Met Glu Ser Gly Phe Thr Ser Lys Asp Thr Tyr Leu Ser His Phe Asn
1               5                   10                  15

Pro Arg Asp Tyr Leu Glu Lys Tyr Tyr Lys Phe Gly Ser Arg His Ser
            20                  25                  30

Ala Glu Ser Gln Ile Leu Lys His Leu Leu Lys Asn Leu Phe Lys Ile
        35                  40                  45

Phe Cys Leu Asp Gly Val Lys Gly Asp Leu Leu Ile Asp Ile Gly Ser
    50                  55                  60

Gly Pro Thr Ile Tyr Gln Leu Leu Ser Ala Cys Glu Ser Phe Lys Glu
65                  70                  75                  80

Ile Val Val Thr Asp Tyr Ser Asp Gln Asn Leu Gln Glu Leu Glu Lys
                85                  90                  95

Trp Leu Lys Lys Glu Pro Glu Ala Phe Asp Trp Ser Pro Val Val Thr
            100                 105                 110

Tyr Val Cys Asp Leu Glu Gly Asn Arg Val Lys Gly Pro Glu Lys Glu
        115                 120                 125

Glu Lys Leu Arg Gln Ala Val Lys Gln Val Leu Lys Cys Asp Val Thr
    130                 135                 140

Gln Ser Gln Pro Leu Gly Ala Val Pro Leu Pro Pro Ala Asp Cys Val
145                 150                 155                 160

Leu Ser Thr Leu Cys Leu Asp Ala Ala Cys Pro Asp Leu Pro Thr Tyr
                165                 170                 175

Cys Arg Ala Leu Arg Asn Leu Gly Ser Leu Leu Lys Pro Gly Gly Phe
            180                 185                 190

Leu Val Ile Met Asp Ala Leu Lys Ser Ser Tyr Tyr Met Ile Gly Glu
        195                 200                 205

Gln Lys Phe Ser Ser Leu Pro Leu Gly Arg Glu Ala Val Glu Ala Ala
    210                 215                 220

Val Lys Glu Ala Gly Tyr Thr Ile Glu Trp Phe Glu Val Ile Ser Gln

225                 230                 235                 240

Ser Tyr Ser Ser Thr Met Ala Asn Asn Glu Gly Leu Phe Ser Leu Val
                245                 250                 255

Ala Arg Lys Leu Ser Arg Pro Leu
            260

<210> 11
<211> 1240
<212> DNA
<213> Homo sapiens

<220>
<221> CDS
<222> (38)..(1144)

<400> 11
   gagctcgcag cgcgcggccc ctgtcctccg gcccgag atg aat cct gcg gca gaa      55
                                            Met Asn Pro Ala Ala Glu
                                            1               5
   gcc gag ttc aac atc ctc ctg gcc acc gac tcc tac aag gtt act cac      103
   Ala Glu Phe Asn Ile Leu Leu Ala Thr Asp Ser Tyr Lys Val Thr His
               10                  15                  20
   tat aaa caa tat cca ccc aac aca agc aaa gtt tat tcc tac ttt gaa      151
   Tyr Lys Gln Tyr Pro Pro Asn Thr Ser Lys Val Tyr Ser Tyr Phe Glu
           25                  30                  35
   tgc cgt gaa aag aag aca gaa aac tcc aaa tta agg aag gtg aaa tat      199
   Cys Arg Glu Lys Lys Thr Glu Asn Ser Lys Leu Arg Lys Val Lys Tyr
       40                  45                  50
   gag gaa aca gta ttt tat ggg ttg cag tac att ctt aat aag tac tta      247
   Glu Glu Thr Val Phe Tyr Gly Leu Gln Tyr Ile Leu Asn Lys Tyr Leu
   55                  60                  65                  70
   aaa ggt aaa gta gta acc aaa gag aaa atc cag gaa gcc aaa gat gtc      295
   Lys Gly Lys Val Val Thr Lys Glu Lys Ile Gln Glu Ala Lys Asp Val
                   75                  80                  85
   tac aaa gaa cat ttc caa gat gat gtc ttt aat gaa aag gga tgg aac      343
   Tyr Lys Glu His Phe Gln Asp Asp Val Phe Asn Glu Lys Gly Trp Asn
               90                  95                  100
   tac att ctt gag aag tat gat ggg cat ctt cca ata gaa ata aaa gct      391
   Tyr Ile Leu Glu Lys Tyr Asp Gly His Leu Pro Ile Glu Ile Lys Ala
           105                 110                 115
   gtt cct gag ggc ttt gtc att ccc aga gga aat gtt ctc ttc acg gtg      439
   Val Pro Glu Gly Phe Val Ile Pro Arg Gly Asn Val Leu Phe Thr Val
       120                 125                 130
   gaa aac aca gat cca gag tgt tac tgg ctt aca aat tgg att gag act      487
   Glu Asn Thr Asp Pro Glu Cys Tyr Trp Leu Thr Asn Trp Ile Glu Thr
   135                 140                 145                 150
   att ctt gtt cag tcc tgg tat cca atc aca gtg gcc aca aat tct aga      535
   Ile Leu Val Gln Ser Trp Tyr Pro Ile Thr Val Ala Thr Asn Ser Arg
                   155                 160                 165
   gag cag aag aaa ata ttg gcc aaa tat ttg tta gaa act tct ggt aac 583
   Glu Gln Lys Lys Ile Leu Ala Lys Tyr Leu Leu Glu Thr Ser Gly Asn
   170 175 180
   tta gat ggt ctg gaa tac aag tta cat gat ttt ggc tac aga gga gtc 631
   Leu Asp Gly Leu Glu Tyr Lys Leu His Asp Phe Gly Tyr Arg Gly Val
   185 190 195
   tct tcc caa gag act gct ggc ata gga gca tct gct cac ttg gtt aac 679
   Ser Ser Gln Glu Thr Ala Gly Ile Gly Ala Ser Ala His Leu Val Asn
   200 205 210
   ttc aaa gga aca gat aca gta gca gga ctt gct cta att aaa aaa tat 727
   Phe Lys Gly Thr Asp Thr Val Ala Gly Leu Ala Leu Ile Lys Lys Tyr
   215 220 225 230
   tat gga acg aaa gat cct gtt cca ggc tat tct gtt cca gca gca gaa 775
   Tyr Gly Thr Lys Asp Pro Val Pro Gly Tyr Ser Val Pro Ala Ala Glu
   235 240 245
   cac agt acc ata aca gct tgg ggg aaa gac cat gaa aaa gat gct ttt 823
   His Ser Thr Ile Thr Ala Trp Gly Lys Asp His Glu Lys Asp Ala Phe
   250 255 260
   gaa cat att gta aca cag ttt tca tca gtg cct gta tct gtg gtc agc 871
   Glu His Ile Val Thr Gln Phe Ser Ser Val Pro Val Ser Val Val Ser
   265 270 275
   gat agc tat gac att tat aat gcg tgt gag aaa ata tgg ggt gaa gat 919
   Asp Ser Tyr Asp Ile Tyr Asn Ala Cys Glu Lys Ile Trp Gly Glu Asp
   280 285 290
   cta aga cat tta ata gta tcg aga agt aca cag gca cca cta ata atc 967
   Leu Arg His Leu Ile Val Ser Arg Ser Thr Gln Ala Pro Leu Ile Ile
   295 300 305 310
   aga cct gat tct gga aac cct ctt gac act gtg tta aag gtt ttg gag 1015
   Arg Pro Asp Ser Gly Asn Pro Leu Asp Thr Val Leu Lys Val Leu Glu
   315 320 325
   att tta ggt aag aag ttt cct gtt act gag aac tca aag ggt tac aag 1063
   Ile Leu Gly Lys Lys Phe Pro Val Thr Glu Asn Ser Lys Gly Tyr Lys
   330 335 340
   ttg ctg cca cct tat ctt aga gtt att caa ggg gat gga gta gat att 1111
   Leu Leu Pro Pro Tyr Leu Arg Val Ile Gln Gly Asp Gly Val Asp Ile
   345 350 355
   aat acc tta caa gag gta tgt gtt tta tat taa aagtttcaat aaggcatttc 1164
   Asn Thr Leu Gln Glu Val Cys Val Leu Tyr
   360 365
   ttataattaa gtttgtttat gtttgataaa gaacacaata taaatacaaa aaaaaaaaaa 1224
   aaaaaaaaaa aaaaaa 1240

<210> 12
<211> 368
<212> PRT
<213> Homo sapiens

<400> 12
Met Asn Pro Ala Ala Glu Ala Glu Phe Asn Ile Leu Leu Ala Thr Asp
1               5                   10                  15

Ser Tyr Lys Val Thr His Tyr Lys Gln Tyr Pro Pro Asn Thr Ser Lys
            20                  25                  30

Val Tyr Ser Tyr Phe Glu Cys Arg Glu Lys Lys Thr Glu Asn Ser Lys
        35                  40                  45

Leu Arg Lys Val Lys Tyr Glu Glu Thr Val Phe Tyr Gly Leu Gln Tyr
    50                  55                  60

Ile Leu Asn Lys Tyr Leu Lys Gly Lys Val Val Thr Lys Glu Lys Ile
65                  70                  75                  80

Gln Glu Ala Lys Asp Val Tyr Lys Glu His Phe Gln Asp Asp Val Phe
                85                  90                  95

Asn Glu Lys Gly Trp Asn Tyr Ile Leu Glu Lys Tyr Asp Gly His Leu
            100                 105                 110

Pro Ile Glu Ile Lys Ala Val Pro Glu Gly Phe Val Ile Pro Arg Gly
        115                 120                 125

Asn Val Leu Phe Thr Val Glu Asn Thr Asp Pro Glu Cys Tyr Trp Leu
    130                 135                 140

Thr Asn Trp Ile Glu Thr Ile Leu Val Gln Ser Trp Tyr Pro Ile Thr
145                 150                 155                 160

Val Ala Thr Asn Ser Arg Glu Gln Lys Lys Ile Leu Ala Lys Tyr Leu
                165                 170                 175

Leu Glu Thr Ser Gly Asn Leu Asp Gly Leu Glu Tyr Lys Leu His Asp
            180                 185                 190

Phe Gly Tyr Arg Gly Val Ser Ser Gln Glu Thr Ala Gly Ile Gly Ala
        195                 200                 205

Ser Ala His Leu Val Asn Phe Lys Gly Thr Asp Thr Val Ala Gly Leu
    210                 215                 220

Ala Leu Ile Lys Lys Tyr Tyr Gly Thr Lys Asp Pro Val Pro Gly Tyr
225                 230                 235                 240

Ser Val Pro Ala Ala Glu His Ser Thr Ile Thr Ala Trp Gly Lys Asp
                245                 250                 255

His Glu Lys Asp Ala Phe Glu His Ile Val Thr Gln Phe Ser Ser Val
            260                 265                 270

Pro Val Ser Val Val Ser Asp Ser Tyr Asp Ile Tyr Asn Ala Cys Glu
        275                 280                 285

Lys Ile Trp Gly Glu Asp Leu Arg His Leu Ile Val Ser Arg Ser Thr
    290                 295                 300

Gln Ala Pro Leu Ile Ile Arg Pro Asp Ser Gly Asn Pro Leu Asp Thr
305                 310                 315                 320

Val Leu Lys Val Leu Glu Ile Leu Gly Lys Lys Phe Pro Val Thr Glu
                325                 330                 335

Asn Ser Lys Gly Tyr Lys Leu Leu Pro Pro Tyr Leu Arg Val Ile Gln
            340                 345                 350

Gly Asp Gly Val Asp Ile Asn Thr Leu Gln Glu Val Cys Val Leu Tyr
        355                 360                 365

<210> 13
<211> 1011
<212> DNA
<213> Homo sapiens

<220>
<221> CDS
<222> (4)..(936)

<400> 13
ccg atg ttg gcg cca gca gct ggt gag ggc cct ggg gtg gac ctg gcg      48
    Met Leu Ala Pro Ala Ala Gly Glu Gly Pro Gly Val Asp Leu Ala
    1               5                   10                  15

gcc aaa gcc cag gtg tgg ctg gag cag gtg tgt gcc cac ctg ggg ctg      96
Ala Lys Ala Gln Val Trp Leu Glu Gln Val Cys Ala His Leu Gly Leu
                20                  25                  30

ggg gtg cag gag cca cat cca ggc gag cgg gca gcc ttt gtg gcc tat      144
Gly Val Gln Glu Pro His Pro Gly Glu Arg Ala Ala Phe Val Ala Tyr
            35                  40                  45

gcc ttg gct ttt ccc cgg gcc ttc cag ggc ctc ctg gac acc tac agc      192
Ala Leu Ala Phe Pro Arg Ala Phe Gln Gly Leu Leu Asp Thr Tyr Ser
        50                  55                  60

gtg tgg agg agt ggt ctc ccc aac ttc cta gca gtc gcc ttg gcc ctg      240
Val Trp Arg Ser Gly Leu Pro Asn Phe Leu Ala Val Ala Leu Ala Leu
    65                  70                  75

gga gag ctg ggc tac cgg gca gtg ggc gtg agg ctg gac agt ggt gac      288
Gly Glu Leu Gly Tyr Arg Ala Val Gly Val Arg Leu Asp Ser Gly Asp
80                  85                  90                  95

ctg cta cag cag gct cag gag atc cgc aag gtc ttc cga gct gct gca      336
Leu Leu Gln Gln Ala Gln Glu Ile Arg Lys Val Phe Arg Ala Ala Ala
                100                 105                 110

gcc cag ttc cag gtg ccc tgg ctg gag tca gtc ctc atc gta gtc agc      384
Ala Gln Phe Gln Val Pro Trp Leu Glu Ser Val Leu Ile Val Val Ser

                115                 120                 125

aac aac att gac gag gag gcg ctg gcc cga ctg gcc cag gag ggc agt      432
Asn Asn Ile Asp Glu Glu Ala Leu Ala Arg Leu Ala Gln Glu Gly Ser
        130                 135                 140

gag gtg aat gtc att ggc att ggc acc agt gtg gtc acc tgc ccc caa      480
Glu Val Asn Val Ile Gly Ile Gly Thr Ser Val Val Thr Cys Pro Gln
    145                 150                 155

cag cct tcc ctg ggt ggc gtc tat aag ctg gtg gcc gtg ggg ggc cag      528
Gln Pro Ser Leu Gly Gly Val Tyr Lys Leu Val Ala Val Gly Gly Gln
160                 165                 170                 175

cca cga atg aag ctg acc gag gac ccc gag aag cag acg ttg cct ggg      576
Pro Arg Met Lys Leu Thr Glu Asp Pro Glu Lys Gln Thr Leu Pro Gly
                180                 185                 190

agc aag gct gct ttc cgg ctc ctg ggc tct gac ggg tct cca ctc atg      624
Ser Lys Ala Ala Phe Arg Leu Leu Gly Ser Asp Gly Ser Pro Leu Met
            195                 200                 205

gac atg ctg cag tta gca gaa gag cca gtg cca cag gct ggg cag gag      672
Asp Met Leu Gln Leu Ala Glu Glu Pro Val Pro Gln Ala Gly Gln Glu
        210                 215                 220

ctg agg gtg tgg cct cca ggg gcc cag gag ccc tgc acc gtg agg cca      720
Leu Arg Val Trp Pro Pro Gly Ala Gln Glu Pro Cys Thr Val Arg Pro
    225                 230                 235

gcc cag gtg gag cca cta ctg cgg ctc tgc ctc cag cag gga cag ctg      768
Ala Gln Val Glu Pro Leu Leu Arg Leu Cys Leu Gln Gln Gly Gln Leu
240                 245                 250                 255

tgt gag ccg ctc cca tcc ctg gca gag tct aga gcc ttg gcc cag ctg      816
Cys Glu Pro Leu Pro Ser Leu Ala Glu Ser Arg Ala Leu Ala Gln Leu
                260                 265                 270

tcc ctg agc cga ctc agc cct gag cac agg cgg ctg cgg agc cct gca      864
Ser Leu Ser Arg Leu Ser Pro Glu His Arg Arg Leu Arg Ser Pro Ala
            275                 280                 285

cag tac cag gtg gtg ctg tcc gag agg ctg cag gcc ctg gtg aac agt      912
Gln Tyr Gln Val Val Leu Ser Glu Arg Leu Gln Ala Leu Val Asn Ser
        290                 295                 300

ctg tgt gcg ggg cag tcc ccc tga gactcggagc ggggctgact ggaaacaaca      966
Leu Cys Ala Gly Gln Ser Pro
    305                 310

cgaatcactc acttttcccc aaaaaaaaaa aaaaaaaaaa aaaaa                    1011


<210> 14
<211> 310
<212> PRT
<213> Homo sapiens

<400> 14
Met Leu Ala Pro Ala Ala Gly Glu Gly Pro Gly Val Asp Leu Ala Ala
1               5                   10                  15


Lys Ala Gln Val Trp Leu Glu Gln Val Cys Ala His Leu Gly Leu Gly
            20                  25                  30

Val Gln Glu Pro His Pro Gly Glu Arg Ala Ala Phe Val Ala Tyr Ala
        35                  40                  45

Leu Ala Phe Pro Arg Ala Phe Gln Gly Leu Leu Asp Thr Tyr Ser Val
    50                  55                  60

Trp Arg Ser Gly Leu Pro Asn Phe Leu Ala Val Ala Leu Ala Leu Gly
65                  70                  75                  80

Glu Leu Gly Tyr Arg Ala Val Gly Val Arg Leu Asp Ser Gly Asp Leu
                85                  90                  95

Leu Gln Gln Ala Gln Glu Ile Arg Lys Val Phe Arg Ala Ala Ala Ala
            100                 105                 110

Gln Phe Gln Val Pro Trp Leu Glu Ser Val Leu Ile Val Val Ser Asn
        115                 120                 125

Asn Ile Asp Glu Glu Ala Leu Ala Arg Leu Ala Gln Glu Gly Ser Glu
    130                 135                 140

Val Asn Val Ile Gly Ile Gly Thr Ser Val Val Thr Cys Pro Gln Gln
145                 150                 155                 160

Pro Ser Leu Gly Gly Val Tyr Lys Leu Val Ala Val Gly Gly Gln Pro
                165                 170                 175

Arg Met Lys Leu Thr Glu Asp Pro Glu Lys Gln Thr Leu Pro Gly Ser
            180                 185                 190

Lys Ala Ala Phe Arg Leu Leu Gly Ser Asp Gly Ser Pro Leu Met Asp
        195                 200                 205

Met Leu Gln Leu Ala Glu Glu Pro Val Pro Gln Ala Gly Gln Glu Leu
    210                 215                 220

Arg Val Trp Pro Pro Gly Ala Gln Glu Pro Cys Thr Val Arg Pro Ala
225                 230                 235                 240

Gln Val Glu Pro Leu Leu Arg Leu Cys Leu Gln Gln Gly Gln Leu Cys
                245                 250                 255

Glu Pro Leu Pro Ser Leu Ala Glu Ser Arg Ala Leu Ala Gln Leu Ser
            260                 265                 270

Leu Ser Arg Leu Ser Pro Glu His Arg Arg Leu Arg Ser Pro Ala Gln
        275                 280                 285

Tyr Gln Val Val Leu Ser Glu Arg Leu Gln Ala Leu Val Asn Ser Leu
    290                 295                 300

Cys Ala Gly Gln Ser Pro
305                 310

<210> 15
<211> 1073
<212> DNA
<213> Homo sapiens

<220>
<221> CDS
<222> (71)..(688)

<400> 15
   ggcacgaggg gtgcccccgc ctcacctgca gaggggccgt tccgggctcg aacccggcac      60
   cttccggaaa atg gcg gct gcc agg ccc agc ctg ggc cga gtc ctc cca      109
              Met Ala Ala Ala Arg Pro Ser Leu Gly Arg Val Leu Pro
              1               5                   10
   gga tcc tct gtc ctg ttc ctg tgt gac atg cag gag aag ttc cgc cac      157
   Gly Ser Ser Val Leu Phe Leu Cys Asp Met Gln Glu Lys Phe Arg His
       15                  20                  25
   aac atc gcc tac ttc cca cag atc gtc tca gtg gct gcc cgc atg ctc      205
   Asn Ile Ala Tyr Phe Pro Gln Ile Val Ser Val Ala Ala Arg Met Leu
   30                  35                  40                  45
   aag gtg gcc cgg ctg ctt gag gtg cca gtc atg ctg acg gag cag tac      253
   Lys Val Ala Arg Leu Leu Glu Val Pro Val Met Leu Thr Glu Gln Tyr
                   50                  55                  60
   cca caa ggc ctg ggc ccc acg gtg ccc gag ctg ggg act gag ggc ctt      301
   Pro Gln Gly Leu Gly Pro Thr Val Pro Glu Leu Gly Thr Glu Gly Leu
               65                  70                  75
   cgg ccg ctg gcc aag acc tgc ttc agc atg gtg cct gcc ctg cag cag      349
   Arg Pro Leu Ala Lys Thr Cys Phe Ser Met Val Pro Ala Leu Gln Gln
           80                  85                  90
   gag ctg gac agt cgg ccc cag ctg cgc tct gtg ctg ctc tgt ggc att      397
   Glu Leu Asp Ser Arg Pro Gln Leu Arg Ser Val Leu Leu Cys Gly Ile
       95                  100                 105
   gag gca cag gcc tgc atc ttg aac acg acc ctg gac ctc cta gac cgg      445
   Glu Ala Gln Ala Cys Ile Leu Asn Thr Thr Leu Asp Leu Leu Asp Arg
   110                 115                 120                 125
   ggg ctg cag gtc cat gtg gtg gtg gac gcc tgc tcc tca cgc agc cag      493
   Gly Leu Gln Val His Val Val Val Asp Ala Cys Ser Ser Arg Ser Gln
                   130                 135                 140
   gtg gac cgg ctg gtg gct ctg gcc cgc atg aga cag agt ggt gcc ttc      541
   Val Asp Arg Leu Val Ala Leu Ala Arg Met Arg Gln Ser Gly Ala Phe
               145                 150                 155
   ctc tcc acc agc gaa ggg ctc att ctg cag ctt gtg ggc gat gcc gtc      589
   Leu Ser Thr Ser Glu Gly Leu Ile Leu Gln Leu Val Gly Asp Ala Val
           160                 165                 170
   cac ccc cag ttc aag gag atc cag aaa ctc atc aag gag ccc gcc cca 637
   His Pro Gln Phe Lys Glu Ile Gln Lys Leu Ile Lys Glu Pro Ala Pro
   175 180 185
   gac agc gga ctg ctg ggc ctc ttc caa ggc cag aac tcc ctc ctc cac 685
   Asp Ser Gly Leu Leu Gly Leu Phe Gln Gly Gln Asn Ser Leu Leu His
   190 195 200 205
   tga actccaaccc tgccttgagg gaagaccacc ctcctgtcac ccggacctca 738
   gtggaagccc gttcccccca tccctggatc ccaagagtgg tgcgatccac caggagtgcc 798
   gcccccttgt gggggggggc agggtgctgc cttcccattg gacagctgct cccggaaatg 858
   caaatgagac tcctggaaac tgggtgggaa ttggctgagc caagatggag gcggggctcg 918
   gccccgggcc acttcacggg gcgggaaggg gaggggaaga agagtctcag actgtgggac 978
   acggactcgc agaataaaca tatatgtggc aaaaaaaaaa aaaaaaaaaa aaaaaaaaaa 1038
   aaaaaaaaaa aaaaaaaaaa aaaaaaaaaa aaaaa 1073

<210> 16
<211> 205
<212> PRT
<213> Homo sapiens

<400> 16
Met Ala Ala Ala Arg Pro Ser Leu Gly Arg Val Leu Pro Gly Ser Ser
1               5                   10                  15

Val Leu Phe Leu Cys Asp Met Gln Glu Lys Phe Arg His Asn Ile Ala
            20                  25                  30

Tyr Phe Pro Gln Ile Val Ser Val Ala Ala Arg Met Leu Lys Val Ala
        35                  40                  45

Arg Leu Leu Glu Val Pro Val Met Leu Thr Glu Gln Tyr Pro Gln Gly
    50                  55                  60

Leu Gly Pro Thr Val Pro Glu Leu Gly Thr Glu Gly Leu Arg Pro Leu
65                  70                  75                  80

Ala Lys Thr Cys Phe Ser Met Val Pro Ala Leu Gln Gln Glu Leu Asp
                85                  90                  95

Ser Arg Pro Gln Leu Arg Ser Val Leu Leu Cys Gly Ile Glu Ala Gln
            100                 105                 110

Ala Cys Ile Leu Asn Thr Thr Leu Asp Leu Leu Asp Arg Gly Leu Gln
        115                 120                 125

Val His Val Val Val Asp Ala Cys Ser Ser Arg Ser Gln Val Asp Arg
    130                 135                 140

Leu Val Ala Leu Ala Arg Met Arg Gln Ser Gly Ala Phe Leu Ser Thr
145                 150                 155                 160

Ser Glu Gly Leu Ile Leu Gln Leu Val Gly Asp Ala Val His Pro Gln
                165                 170                 175

Phe Lys Glu Ile Gln Lys Leu Ile Lys Glu Pro Ala Pro Asp Ser Gly
            180                 185                 190

Leu Leu Gly Leu Phe Gln Gly Gln Asn Ser Leu Leu His
        195                 200                 205

<210> 17
<211> 1825
<212> DNA
<213> Homo sapiens

<220>
<221> CDS
<222> (144)..(983)

<400> 17
agagtgcgac cgagatgttc cactcgctgg cgtccgggcc gctggtgatc tccggtagca      60
ctcgggccgg cggacagtga gggcgcgaca acaaggaggg tgtcacagtt ttccatttag     120
atcaacaact tcaagttctt acc atg gaa aat tcc gag aag act gaa gtg gtt     173
ctc ctt gct tgt ggt tca ttc aat ccc atc acc aac atg cac ctc agg       221
ttg ttt gag ctg gcc aag gac tac atg aat gga aca gga agg tac aca       269
gtt gtc aaa ggc atc atc tct cct gtt ggt gat gcc tac aag aag aaa       317
gga ctc att cct gcc tat cac cgg gtc atc atg gca gaa ctt gct acc       365
aag aat tct aaa tgg gtg gaa gtt gat aca tgg gaa agt ctt cag aag       413
gag tgg aaa gag act ctg aag gtg cta aga cac cat caa gag aaa ttg       461
gag gct agt gac tgt gat cac cag cag aac tca cct act cta gaa agg       509
cct gga agg aag agg aag tgg act gaa aca caa gat tct agt caa aag       557
aaa tcc cta gag cca aaa aca aaa gct gtg cca aag gtc aag ctg ctg     605
tgt ggg gca gat tta ttg gag tcc ttt gct gtt ccc aat ttg tgg aag     653
agt gaa gac atc acc caa atc gtg gcc aac tat ggg ctc ata tgt gtt     701
act cgg gct gga aat gat gct cag aag ttt atc tat gaa tcg gat gtg     749
ctg tgg aaa cac cgg agc aac att cac gtg gtg aat gaa tgg atc gct     797
aat gac atc tca tcc aca aaa atc cgg aga gcc ctc aga agg ggc cag     845
agc att cgc tac ttg gta cca gat ctt gtc caa gaa tac att gaa aag     893
cat aat ttg tac agc tct gag agt gaa gac agg aat gct ggg gtc atc     941
ctg gcc cct ttg cag aga aac act gca gaa gct aag aca tag             983
gaattctaca gcatgatatt tcagacttcc catttgggga tctgaaacaa tctgggagtt    1043
aataactggg gaaagaagtt gtgatctgtt gcctaaacta aagcttaaaa gtttagtaaa    1103
aatcgtctgg gcacagtggc tcacgcctgt agtcccagct acttgggagg ctgaggcagg    1163
agaatcactt gaccccaggt ggtggaggtt gcagtgagcc aagattgcac cattgcactc    1223
cagcctggcg acagagcaag actctgtctc aaaaaaaaaa aaaaaattta gtaaaaatca    1283
atggtaagct aaaataagtt tttgtttgtt tatttgtttt tgagatggag tctctactaa    1343
aaatacaaaa aattagccag gcatggtgcc gcataactat aatcccagct acttgggagg    1403
ctgaggcagg agaatcgctt gaacccggga ggcacaggtt ccagtgggcc aaggttgtgc    1463
cactgcactc cagcctgggc aaaaaagcaa aactccatct caaagagaaa aaaaaaaaag    1523
accgggtgtg gtggctcaca cctgtaatcc cagcactttg ggaggcctaa gtgggtggat    1583
cacgtgaggt caagagttca agaccagcct ggccaatatg gtgaaacccc atctctacta    1643
agaatacaaa aaattagctg agcatggtgg tgggctcctg tagtcccagc tacttgggag    1703
gctgaggcag gagaatcgct tgaacctggg aggcagaggt tgcagtaagc caagatcgtg    1763
ccattgcact ccagcctggg tgacagagcg agactccatc tcaaaaaaaa aaaaaaaaaa    1823
aa                                                                    1825

<210> 18
<211> 279
<212> PRT
<213> Homo sapiens

<400> 18

<210> 19
<211> 5690
<212> DNA
<213> Homo sapiens

<220>
<221> CDS
<222> (338)..(1261)

<400> 19
atataaactc taaggaagac agtgatggag tgaagtgggc tgggggcgat agagaggatg      60

gggtggggca ccaggcgaga gatgcgaagg aagccagaac gaaaagagag cgaccgagga     120

gagaagagag cagagcaata caaaagcagc ctcggatcta gccggagctg caagcgttaa     180

ggggaggcgg agagtgacgc ggtttgcgtc tggagcggct ccttggagtc cacagcatcc     240

accgccggag cctcgccttc ctttctccct ctgcagacac aacgagacac aaaaagagag     300

gcaaccccta gaccaccgcg aaggacccat ctgcacc atg acc gag acc acc aag     355
                                         Met Thr Glu Thr Thr Lys
                                         1               5

acc cac gtt atc ttg ctc gcc tgc ggc agc ttc aat ccc atc acc aaa     403
Thr His Val Ile Leu Leu Ala Cys Gly Ser Phe Asn Pro Ile Thr Lys
            10                  15                  20

ggg cac att cag atg ttt gaa aga gcc agg gat tat ctg cac aaa act     451
Gly His Ile Gln Met Phe Glu Arg Ala Arg Asp Tyr Leu His Lys Thr
        25                  30                  35

gga agg ttt att gtg att ggc ggg att gtc tcc cct gtc cac gac tcc     499
Gly Arg Phe Ile Val Ile Gly Gly Ile Val Ser Pro Val His Asp Ser
    40                  45                  50

tat gga aaa cag ggc ctc gtg tca agc cgg cac cgt ctc atc atg tgt     547
Tyr Gly Lys Gln Gly Leu Val Ser Ser Arg His Arg Leu Ile Met Cys
55                  60                  65                  70

cag ctg gcc gtc cag aat tct gat tgg atc agg gtg gac cct tgg gag     595
Gln Leu Ala Val Gln Asn Ser Asp Trp Ile Arg Val Asp Pro Trp Glu
                75                  80                  85

tgc tac cag gac acc tgg cag acg acc tgc agc gtg ttg gaa cac cac     643
Cys Tyr Gln Asp Thr Trp Gln Thr Thr Cys Ser Val Leu Glu His His
            90                  95                  100

cgg gac ctc atg aag agg gtg act ggc tgc atc ctc tcc aat gtc aac     691

Arg Asp Leu Met Lys Arg Val Thr Gly Cys Ile Leu Ser Asn Val Asn
        105                 110                 115

aca cct tcc atg aca cct gtg atc gga cag cca caa aac gag acc ccc     739
Thr Pro Ser Met Thr Pro Val Ile Gly Gln Pro Gln Asn Glu Thr Pro
    120                 125                 130

cag ccc att tac cag aac agc aac gtg gcc acc aag ccc act gca gcc     787
Gln Pro Ile Tyr Gln Asn Ser Asn Val Ala Thr Lys Pro Thr Ala Ala
135                 140                 145                 150

aag atc ttg ggg aag gtg gga gaa agc ctc agc cgg atc tgc tgt gtc     835
Lys Ile Leu Gly Lys Val Gly Glu Ser Leu Ser Arg Ile Cys Cys Val
                155                 160                 165

cgc ccg ccg gtg gag cgt ttc acc ttt gta gat gag aat gcc aat ctg     883
Arg Pro Pro Val Glu Arg Phe Thr Phe Val Asp Glu Asn Ala Asn Leu
            170                 175                 180

ggc acg gtg atg cgg tat gaa gag att gag cta cgg atc ctg ctg ctg     931
Gly Thr Val Met Arg Tyr Glu Glu Ile Glu Leu Arg Ile Leu Leu Leu
        185                 190                 195

tgt ggt agt gac ctg ctg gag tcc ttc tgc atc cca ggg ctc tgg aac     979
Cys Gly Ser Asp Leu Leu Glu Ser Phe Cys Ile Pro Gly Leu Trp Asn
    200                 205                 210

gag gca gat atg gag gtg att gtt ggt gac ttt ggg att gtg gtg gtg     1027
Glu Ala Asp Met Glu Val Ile Val Gly Asp Phe Gly Ile Val Val Val
215                 220                 225                 230

ccc cgg gat gca gcc gac aca gac cga atc atg aat cac tcc tca ata     1075
Pro Arg Asp Ala Ala Asp Thr Asp Arg Ile Met Asn His Ser Ser Ile
                235                 240                 245

ctc cgc aaa tac aaa aac aac atc atg gtg gtg aag gat gac atc aac     1123
Leu Arg Lys Tyr Lys Asn Asn Ile Met Val Val Lys Asp Asp Ile Asn
            250                 255                 260

cat ccc atg tct gtt gtc agc tca acc aag agc agg ctg gcc ctg cag     1171
His Pro Met Ser Val Val Ser Ser Thr Lys Ser Arg Leu Ala Leu Gln
        265                 270                 275

cat ggg gac ggc cat gtt gtg gat tac ctg tcc cag ccg gtc atc gac     1219
His Gly Asp Gly His Val Val Asp Tyr Leu Ser Gln Pro Val Ile Asp
    280                 285                 290

tac atc ctc aaa agc cag ctg tac atc aat gcc tcc ggc tag             1261
Tyr Ile Leu Lys Ser Gln Leu Tyr Ile Asn Ala Ser Gly
295                 300                 305

cagcccctcg tcctccggca acacaatggc ccctccatct ttgtcagccc cctgtttctc     1321

tcctgcctct ctgtttctcc atctcctcgt cttgactgtt ttccctactt gctgacttaa     1381

ccccccatag tgtgggggac ctgcagagaa ccatggcatt ccctattcca cagtcatctt     1441

tggacagact ttcctctagt ctccgggttg ggggtgggtg agggaatggg gtgggagtcg     1501

ggggaagtgc agtccttgga gatgtactgg tgtccgtctc ccagcatgct ctagagaggc     1561

ggctctggtg cccatcctcc cagcacgctc tggggaggcg gctctggtgc ccatcctccc     1621

agcatgctct agagaggcgg ctctggtgcc cctcctccca gcatgctctg gggaggcggc     1681

tctggctctt gccttcccag catgcccttt actacaaagg gctatttttc ttttctttct 1741

tttgtttatt tatttttctt tgttcactcc ctgtagaact tggatgaaat cagtgtccat 1801

ggttctttat gtttgtagtc ttgatgtgct cctgtggtat tacttcccct ctgataggac 1861

attgtagcca gcctcagcac tcagtgagtt catcagggcc acacccagta gagaaggcca 1921

agcaacctcc acttcttcag caccacacac acgcacacac acacacacgc acacatgcgt 1981

gtgcacccgc gcacgcacat acacacacac atatagcagt agcagcagca gcagcagcag 2041

cagcaacctt tgatcaggag tgagattttc gggttctgaa acctgggaca cgagtctgtg 2101

aatagtcggt tttctcagaa taatttgaat ctgttttctt agtttcaaat gaccatttcc 2161

ctgatgctct gagcttatga tcacacagag ccagtccatc ctcatttcct ggtggcatct 2221

gttcatttac ctttgtggac tgtagctgat ggcacagtgc gggttcccta ccagccaggg 2281

gtttccaagg gacctttgga ggccatgctt agacacattc ctgtacctga gaacaaccac 2341

ataggcagga ccagatccac atcgtgcagt cgtgtcataa aaaaacaaaa caaaacaaaa 2401

aaacactagg agtccactca accctggagg tctttgctaa ttggaattat gtattgtctg 2461

ttgggctggg aaatgtctct ttcatattgt aagtccagga tgaactagga gaaagcaatt 2521

tgttgccctg atgataactg atgattttca ccctctctag ctgaggtaac tcagacagtg 2581

catgaggtca gtttcttctt gagaagcagt gccttggtct tgtttctgtg gttggttcta 2641

gcccctgcag agcctgggag ctgcaggaac tgtctgagaa aatctcccta ataggggagt 2701

gggttcccag aagggagatc tgggaggggt caggagccac taagttgctt cactcctttt 2761

ttctctaatt ttctaccttc ctctctgttc ctgcagacag ttttgccagc tttgcttctg 2821

gttactaggg tctcatgcgt gtcctgcttg gagagccata aggaaattgc tgtcttgtgc 2881

tttgtgtctc tcatccagtc tctggctctt gggattctgg tctttgagaa atagtccctg 2941

agtattagga tacttttatc aaaatctagt accagctacg gccagaaagg gccaggtggg 3001

acctgaaagc aaagacaatg ttctttacca cacgtttcac atctgcaaca tccttcaatt 3061

gcgggaaaag gaacttgatt taacagaaga acatggtaga gcagcatcca gaaagtctgt 3121

tattcctctt ggattttttg aaataatctt cagaggaagg aaggaaaatc ctattttggg 3181

gtatcagtgt ttgactaggg atcatgaaat aataaactga aaaaaacttt agagttcagt 3241

tgatccaaca ctttccttta aaagttgagg gagcagaggc ccatgggatt aaatggctgg 3301

tccaggtcag ccagcaggtg tagggcctga caagaacata ttgtttccct gacccctagg 3361

ccgtcacacc acaccctcca tttcctcatg ttgctgacca ggtgcccata tgatttctac 3421

acttcccaag ccttaccctg gcatctttct tttaaattat atctgtccca ggtgctctcc 3481

acacatagga tggtaatgcc agtcccaggg gagggtgtga tagtaaggaa ggccactgtt 3541

aggtttcctt tagaaataaa gagatctcag cagcttggaa gaaatcccag aagcggaact 3601

ccatcaatcc aagaaagagt tgctttgtgg aaggtgaagg aagacccaca gagtgctcag 3661

gatgatgcta ttgctggaga gcgaaagatg gaacagcctt gtccaggcag aacagtcata 3721

agccaggaaa tgaaacaaag gaaaacaggt gcctgaattt cctggggaaa catggcttgt 3781

ttaaggactt ggagttatgg atggaattta tgggacccac gtgagcagac ctgaggaagg 3841

ctcgatttct tttgtttctt ggtccactct gtcactctgc tctggtcaag ccccatttgg 3901

tctacagccc atgagaagga atgaggctgg ttctgcactc tcagcatgca gtccgaaagc 3961

atgtgggagt ggggagggaa agtgagatga attaagacaa agaacaggtg ccatagaagt 4021

agatttctag gaatgaagtg gggcagatct tatctttgtg gattacaggc actgtactaa 4081

aaacaggttt cctatttaat ataaaaagaa agtgaatctt cttttggata gaatcatcca 4141

ttcccatcgc cgcaccccct accccccaaa cacacacaca cacacacaca cacacacaca 4201

cacacacaca cacacacaca cgccctactc ttcatttgct aggggaaggt cacagcacaa 4261

ctaaatccag gacaggacat tgtgaccatg acccagccac agtcaatacc agaaagatga 4321

ttcagagtct gaagtggtgc cccaggtgcc aacaggataa cctctacccc ccgactttgt 4381

ctctggggtc ctgttccttc ctgcaaagcc caatccaaga ctggcatggc tcagaggttg 4441

tgagaaaggc atggactgga acaatcatgt ccagaggggt ctggagcttt gtttcctgtt 4501

caccagcaaa aaatgtctct cccatttttc tgaaagtggc tgatgtaaga acaggcagaa 4561

ggaaaaccct ttttgtcaat aactctgtcc ttaaggaatg gtcctctggg agggctgtgc 4621

tgctagtggg tacctcagtc acacaccccc aaccccaggc agcctctaga gccttcttgc 4681

tttcattttc cttgaatgta cataggaaca agggggaaag tctcttactg aagtgcctga 4741

aacccaaagc tagagcttct agagacgccg ttcttcctgt ctcagcttgg ccagcctttc 4801

aacaatgttc tctagtttca agctccagct tctcagaaag aattaaagaa cttgctgttc 4861

aaattaagta gaaagtgaga ctcaataata actgaactac agcaaaaggc agagaattac 4921

agggagaaaa aacttgtact taccagccca attctactct cctcaaactg acacacacac 4981

acacacacac acacacacac acacacacac acacacactc ttttagggga ctaagagaga 5041

gaagcatgtt attacatttt actcatccaa acagtaatgc aaaaataaaa cggtagaata 5101

tgaaaagctc aggatctctc ccaaggctac ctactgcagg agggccaaca ggtgagatgg 5161

gaagaatgga aacagggacc gattttgtag ctcatacaat taggacacct taggaatagc 5221

attgtagtaa tggtgatgaa tatgctctgc caaattcatc cagtctgcac catcttatag 5281

ctgcccagca cactcgactg ttcatgtggt ctctttgtag tgtgagtttg gagtgtccta 5341

ttagcctgtt ctggttagga atgagttaac ggctctttcc ctcaacctta gtctagtccc 5401

agggctgagg attcagctgg atccacatgg tcttgagggt tggcatgagg agggggaagc 5461

ttttttgaat cgctttttga tcacataatc tgccatttta agagtaagat ttgctttatg 5521

gaaatcaatt cattaataaa aaatgatatt caagttgcaa taccatttca cagtgaaata 5581

ttttgagtac aattttgttg ctagaatagt catgggcaag agttttatgc aaaatgtttc 5641

aattatgtta ataaataaga caatgcwaaa aaaaaaaaaa aaaaaaaaa 5690

<210> 20
<211> 307
<212> PRT
<213> Homo sapiens

<400> 20
Met Thr Glu Thr Thr Lys Thr His Val Ile Leu Leu Ala Cys Gly Ser
1               5                   10                  15

Phe Asn Pro Ile Thr Lys Gly His Ile Gln Met Phe Glu Arg Ala Arg
            20                  25                  30

Asp Tyr Leu His Lys Thr Gly Arg Phe Ile Val Ile Gly Gly Ile Val
        35                  40                  45

Ser Pro Val His Asp Ser Tyr Gly Lys Gln Gly Leu Val Ser Ser Arg
    50                  55                  60

His Arg Leu Ile Met Cys Gln Leu Ala Val Gln Asn Ser Asp Trp Ile
65                  70                  75                  80

Arg Val Asp Pro Trp Glu Cys Tyr Gln Asp Thr Trp Gln Thr Thr Cys
                85                  90                  95

Ser Val Leu Glu His His Arg Asp Leu Met Lys Arg Val Thr Gly Cys
            100                 105                 110

Ile Leu Ser Asn Val Asn Thr Pro Ser Met Thr Pro Val Ile Gly Gln
        115                 120                 125

Pro Gln Asn Glu Thr Pro Gln Pro Ile Tyr Gln Asn Ser Asn Val Ala
    130                 135                 140

Thr Lys Pro Thr Ala Ala Lys Ile Leu Gly Lys Val Gly Glu Ser Leu
145                 150                 155                 160

Ser Arg Ile Cys Cys Val Arg Pro Pro Val Glu Arg Phe Thr Phe Val
                165                 170                 175

Asp Glu Asn Ala Asn Leu Gly Thr Val Met Arg Tyr Glu Glu Ile Glu
            180                 185                 190

Leu Arg Ile Leu Leu Leu Cys Gly Ser Asp Leu Leu Glu Ser Phe Cys
        195                 200                 205

Ile Pro Gly Leu Trp Asn Glu Ala Asp Met Glu Val Ile Val Gly Asp
    210                 215                 220

Phe Gly Ile Val Val Val Pro Arg Asp Ala Ala Asp Thr Asp Arg Ile
225                 230                 235                 240

Met Asn His Ser Ser Ile Leu Arg Lys Tyr Lys Asn Asn Ile Met Val
                245                 250                 255

Val Lys Asp Asp Ile Asn His Pro Met Ser Val Val Ser Ser Thr Lys
            260                 265                 270

Ser Arg Leu Ala Leu Gln His Gly Asp Gly His Val Val Asp Tyr Leu
        275                 280                 285

Ser Gln Pro Val Ile Asp Tyr Ile Leu Lys Ser Gln Leu Tyr Ile Asn
    290                 295                 300

Ala Ser Gly
305

<210> 21
<211> 20
<212> DNA
<213> Artificial Sequence

<220>
<223> Description of Artificial Sequence: Synthetic
      oligonucleotide

<400> 21
caggcagtcc tttctatttc                                              20

<210> 22
<211> 20
<212> DNA
<213> Artificial Sequence

<220>
<223> Description of Artificial Sequence: Synthetic
      oligonucleotide

<400> 22
gcttgttaac tctccgacag                                              20

<210> 23
<211> 20
<212> DNA
<213> Artificial Sequence

<220>
<223> Description of Artificial Sequence: Synthetic
      oligonucleotide

<400> 23
aatgtcttat caagaccgac                                              20

<210> 24
<211> 20
<212> DNA
<213> Artificial Sequence

<220>
<223> Description of Artificial Sequence: Synthetic
      oligonucleotide

<400> 24
tacagtccag aaatcgctcc                                                   20


<210> 25
<211> 24
<212> DNA
<213> Artificial Sequence

<220>
<223> Description of Artificial Sequence: Synthetic
      oligonucleotide

<400> 25
gaaaggattt gcccggacag tttg                                              24


<210> 26
<211> 24
<212> DNA
<213> Artificial Sequence

<220>
<223> Description of Artificial Sequence: Synthetic
      oligonucleotide

<400> 26
cttcttccca gtagcctgtt cctt                                              24


<210> 27
<211> 25
<212> DNA
<213> Artificial Sequence

<220>
<223> Description of Artificial Sequence: Synthetic
      oligonucleotide

<400> 27
gtggcattac tccacttcaa gtaag                                             25


<210> 28
<211> 19
<212> DNA
<213> Artificial Sequence

<220>
<223> Description of Artificial Sequence: Synthetic
      oligonucleotide

<400> 28
caagagcaag acgatgggg                                                    19

<210> 29
<211> 20
<212> DNA
<213> Artificial Sequence

<220>
<223> Description of Artificial Sequence: Synthetic
      oligonucleotide

<400> 29
ttttccgctg aaccgttcca                                                   20


<210> 30
<211> 20
<212> DNA
<213> Artificial Sequence

<220>
<223> Description of Artificial Sequence: Synthetic
      oligonucleotide

<400> 30
cattggcact catgaccttc                                                   20


<210> 31
<211> 40
<212> PRT
<213> Drosophila sp.

<400> 31
Phe Asp Gly Thr Ser Asn Val Leu Ala Gly Lys Leu Phe Asn Ile Pro
  1               5                  10                  15

Val Lys Gly Thr His Ala His Ala Tyr Ile Thr Ser Phe Ser Ser Ile
             20                  25                  30

Gly Glu Leu Lys Thr Arg Leu Ile
         35                  40


<210> 32
<211> 40
<212> PRT
<213> Caenorhabditis elegans

<400> 32
Phe Asp Ala Thr Ser Asn Val Leu Ala Gly Lys Leu Tyr Gly Ile Pro
  1               5                  10                  15

Val Lys Gly Thr Gln Ala His Ser Phe Ile Cys Ser Phe Ser Ser Pro
             20                  25                  30

Ala Glu Leu Lys Val Arg Leu Leu
         35                  40


<210> 33
<211> 40
<212> PRT
<213> Homo sapiens

<400> 33
Phe Asp Ser Ser Ser Asn Val Leu Ala Gly Gln Leu Arg Gly Val Pro
1               5                   10                  15

Val Ala Gly Thr Leu Ala His Ser Phe Val Thr Ser Phe Ser Gly Ser
            20                  25                  30

Glu Val Pro Pro Asp Pro Met Leu
        35                  40


<210> 34
<211> 37
<212> PRT
<213> Saccharomyces cerevisiae

<400> 34
Leu Leu Leu Gly Thr Ser Asn Ile Leu Phe Ala Lys Lys Tyr Gly Val
1               5                   10                  15

Lys Pro Ile Gly Thr Val Ala His Glu Trp Val Met Gly Val Ala Ser
            20                  25                  30

Ile Ser Glu Asp Tyr
        35


<210> 35
<211> 221
<212> PRT
<213> Homo sapiens

<400> 35
Met Ala Ala Ala Arg Pro Ser Leu Gly Arg Val Leu Pro Gly Ser Ser
1               5                   10                  15

Val Leu Phe Leu Cys Asp Met Gln Glu Lys Phe Arg His Asn Ile Ala
            20                  25                  30

Tyr Phe Pro Gln Ile Val Ser Val Ala Ala Arg Met Leu Lys Val Ala
        35                  40                  45

Arg Leu Leu Glu Val Pro Val Met Leu Thr Glu Gln Tyr Pro Gln Gly
    50                  55                  60

Leu Gly Pro Thr Val Pro Glu Leu Gly Thr Glu Gly Leu Arg Pro Leu
65                  70                  75                  80

Ala Lys Thr Cys Phe Ser Met Val Pro Ala Leu Gln Gln Glu Leu Asp
                85                  90                  95

Ser Arg Pro Gln Leu Arg Ser Val Leu Leu Cys Gly Ile Glu Ala Gln
            100                 105                 110

Ala Cys Ile Leu Asp Pro Arg Ser Tyr Pro Gly Leu Ala Leu Thr Ser
        115                 120                 125

Leu Tyr Pro Gln Asn Thr Thr Leu Asp Leu Leu Asp Arg Gly Leu Gln
    130                 135                 140

Val His Val Val Val Asp Ala Cys Ser Ser Arg Ser Gln Val Asp Arg
145                 150                 155                 160

Leu Val Ala Leu Ala Arg Met Arg Gln Ser Gly Ala Phe Leu Ser Thr
                165                 170                 175

Ser Glu Gly Leu Ile Leu Gln Leu Val Gly Asp Ala Val His Pro Gln
            180                 185                 190

Phe Lys Glu Ile Gln Lys Leu Ile Lys Glu Pro Ala Pro Asp Ser Gly
        195                 200                 205

Leu Leu Gly Leu Phe Gln Gly Gln Asn Ser Leu Leu His
    210                 215                 220


<210> 36
<211> 241
<212> PRT
<213> Homo sapiens

<400> 36
Tyr Gly Asp Gln Ile Asp Met His Arg Lys Phe Val Val Gln Leu Phe
1               5                   10                  15

Ala Glu Glu Trp Gly Gln Tyr Val Asp Leu Pro Lys Gly Phe Ala Val
            20                  25                  30

Ser Glu Arg Cys Lys Val Arg Leu Val Pro Leu Gln Ile Gln Leu Thr
        35                  40                  45

Thr Leu Gly Asn Leu Thr Pro Ser Ser Thr Val Phe Phe Cys Cys Asp
    50                  55                  60

Met Gln Glu Arg Phe Arg Pro Ala Ile Lys Tyr Phe Gly Asp Ile Ile
65                  70                  75                  80

Ser Val Gly Gln Arg Leu Leu Gln Gly Ala Arg Ile Leu Gly Ile Pro
                85                  90                  95

Val Ile Val Thr Glu Gln Tyr Pro Lys Gly Leu Gly Ser Thr Val Gln
            100                 105                 110

Glu Ile Asp Leu Thr Gly Val Lys Leu Val Leu Pro Lys Thr Lys Phe
        115                 120                 125

Ser Met Val Leu Pro Glu Val Glu Ala Ala Leu Ala Glu Ile Pro Gly
    130                 135                 140

Val Arg Ser Val Val Leu Phe Gly Val Glu Thr His Val Cys Ile Gln
145                 150                 155                 160

Gln Thr Ala Leu Glu Leu Val Gly Arg Gly Val Glu Val His Ile Val
                165                 170                 175

Ala Asp Ala Thr Ser Ser Arg Ser Met Met Asp Arg Met Phe Ala Leu
            180                 185                 190

Glu Arg Leu Ala Arg Thr Gly Ile Ile Val Thr Thr Ser Glu Ala Val
        195                 200                 205

Leu Leu Gln Leu Val Ala Asp Lys Asp His Pro Lys Phe Lys Glu Ile
    210                 215                 220

Gln Asn Leu Ile Lys Ala Ser Ala Pro Glu Ser Gly Leu Leu Ser Lys
225                 230                 235                 240

Val

<210> 37
<211> 199
<212> PRT
<213> Homo sapiens

<400> 37
Met His Arg Lys Phe Val Val Gln Leu Phe Ala Glu Glu Trp Gly Gln
1               5                   10                  15

Tyr Val Asp Leu Pro Lys Gly Phe Ala Val Ser Glu Arg Cys Lys Val
            20                  25                  30

Arg Leu Val Pro Leu Gln Ile Gln Leu Thr Thr Leu Gly Asn Leu Thr
        35                  40                  45

Pro Ser Ser Thr Val Phe Phe Cys Cys Asp Met Gln Glu Arg Phe Arg
    50                  55                  60

Pro Ala Ile Lys Tyr Phe Gly Asp Ile Ile Ser Val Gly Gln Arg Leu
65                  70                  75                  80

Leu Gln Gly Ala Arg Ile Leu Gly Ile Pro Val Ile Val Thr Glu Gln
                85                  90                  95

Tyr Pro Lys Gly Leu Gly Ser Thr Val Gln Glu Ile Asp Leu Thr Gly
            100                 105                 110

Val Lys Leu Val Leu Pro Lys Thr Lys Phe Ser Met Val Leu Pro Glu
        115                 120                 125

Val Glu Ala Ala Leu Ala Glu Ile Pro Gly Val Arg Ser Val Val Leu
    130                 135                 140

Phe Gly Val Glu Thr His Val Cys Ile Gln Gln Thr Ala Leu Glu Leu
145                 150                 155                 160

Val Gly Arg Gly Val Glu Val His Ile Val Ala Asp Ala Thr Ser Ser
                165                 170                 175

Arg Ser Met Met Asp Arg Met Phe Ala Arg Leu Thr Ser Arg Ser Asn
            180                 185                 190

Gly Asp His Ser Asp His Glu
        195


<210> 38
<211> 286
<212> PRT
<213> Drosophila sp.

<400> 38
Ser Gln Asp Ser Asn Asp Asn Leu Thr Ser Cys Ser Leu Cys Val Cys
1               5                   10                  15

Gln Ser Leu Arg Ile Val Arg Pro Val Asn Ala Phe Leu Ile Val Asp
            20                  25                  30

Val Gln Asn Asp Phe Ile Ser Gly Ser Leu Asp Ile Ser Asn Cys Ser
        35                  40                  45

Ala Gln Gln Gln Gly His Glu Ile Leu Glu Pro Ile Asn Lys Leu Leu
    50                  55                  60

Asp Thr Val Asp Phe Asp Ala Val Phe Tyr Ser Leu Asp Trp His Pro
65                  70                  75                  80

Ser Asp His Val Ser Phe Ile Asp Asn Val Lys Met Arg Pro Met Asp
                85                  90                  95

Glu Ser Ser Ala Leu Asp Ser Asp Ser Ala Lys Val Phe Asp Thr Val
            100                 105                 110

Ile Phe Ala Gly Pro Pro Pro Met Lys Gln Arg Leu Trp Pro Arg His
        115                 120                 125

Cys Val Gln Asp Ser Trp Gly Ala Glu Leu His Lys Asp Leu Lys Val
    130                 135                 140

Val Asp His Gly Ile Lys Val Tyr Lys Gly Thr Asn Pro Glu Val Asp
145                 150                 155                 160

Ser Tyr Ser Val Phe Trp Asp Asn Lys Lys Leu Ser Asp Thr Thr Leu
                165                 170                 175

Asn Ala Gln Leu Lys Met Lys Gly Ala Thr Asp Ile Tyr Val Cys Gly
            180                 185                 190

Leu Ala Tyr Asp Val Cys Val Gly Ala Thr Ala Val Asp Ala Leu Ser
        195                 200                 205

Ala Gly Tyr Arg Thr Ile Leu Ile Asp Asp Cys Cys Arg Gly Thr Asp
    210                 215                 220

Val His Asp Ile Glu His Thr Lys Glu Lys Val Asn Thr Ser Asp Gly
225                 230                 235                 240

Val Ile Val His Thr Asn Gln Val Lys Ala Met Ala Glu Gly Arg Asp
                245                 250                 255

Arg Arg Pro Glu Leu Gly Tyr Lys Leu Ala Met Glu Leu Lys Ser Pro
            260                 265                 270

Asp Ser Val Leu Ser Gln Arg Asn Gly Phe Arg Pro Ser Tyr
        275                 280                 285

## Description

### Background of the invention

Physiological studies and, more recently, DNA array analysis of gene expression patterns have confirmed that aging is a complex biological process. In contrast, genetic studies in model organisms have demonstrated that relatively minor changes to an organism's environment or genetic makeup can dramatically slow the aging process. For example, the life span of many diverse organisms can be greatly extended simply by limiting calorie intake, in a dietary regime known as caloric restriction (1-3).

How can simple changes have such profound effects on a complex process such as aging? A picture is emerging in which all eukaryotes possess a surprisingly conserved regulatory system that governs the pace of aging (4,5). Such a regulatory system may have arisen in evolution to allow organisms to survive in adverse conditions by redirecting resources from growth and reproduction to pathways that provide stress resistance (4,6).

One model that has proven particularly useful in the identification of regulatory factors of aging is the budding yeast, *S. cerevisiae.* Replicative life span in *S. cerevisiae* is typically defined as the number of buds or "daughter cells" produced by an individual "mother cell" (7). Mother cells undergo age-dependent changes including an increase in size, a slowing of the cell cycle, enlargement of the nucleolus, an increase in steady-state NAD⁺ levels, increased gluconeogenesis and energy storage, and sterility resulting from the loss of silencing at telomeres and mating-type loci (8-13). An alternative measure of yeast life span, known as chronological aging, is the length of time a population of non-dividing cells remains viable when deprived of nutrients (14). Increased chronological life span correlates with increased resistance to heat shock and oxidative stress, suggesting that cumulative damage to cellular components is a major cause of this type of aging (14,15). The extent of overlap between replicative and chronological aging is currently unclear.

One cause of yeast replicative aging has been shown to stem from the instability of the repeated ribosomal DNA (rDNA) locus (16). This instability gives rise to circular forms of rDNA called ERCs that replicate but fail to segregate to daughter cells. Eventually, ERCs accumulate to over 1000 copies, which are thought to kill cells by titrating essential transcription and/or replication factors. (16-18). Regimens that reduce DNA recombination such as caloric restriction or a *fobl* deletion extend replicative life span (17,19,20).

A key regulator of aging in yeast is the Sir2 silencing protein (17), a nicotinamide adenine dinucleotide (NAD⁺)-dependent deacetylase (21-24). Sir2 is a component of the heterotrimeric Sir2/314 complex that catalyzes the formation of silent heterochromatin at telomeres and the two silent mating-type loci (25). Sir2 is also a component of the RENT complex that is required for silencing at the rDNA locus and exit from telophase (26,27). This complex has also recently been shown to directly stimulate transcription of rRNA by Pol I and to be involved in regulation of nucleolar structure (28).

Biochemical studies have shown that Sir2 can readily deacetylate the amino-terminal tails of his tones H3 and H4, resulting in the formation of 1-O-acetyl-ADP-ribose and nicotinamide (21-23,29). Strains with additional copies of *SIR2* display increased rDNA silencing (30) and a 30% longer life span (17). It has recently been shown that additional copies of the *C. elegans SIR2* homolog, *sir-2.1*, greatly extend life span in that organism (31). This implies that the *SIR2*-dependent regulatory pathway for aging arose early in evolution and has been well conserved (4). Yeast life span, like that of metazoans, is also extended by interventions that resemble caloric restriction (19,32). Mutations that reduce the activity of the glucose-responsive cAMP (adenosine 3'5'-monophosphate)-dependent (PKA) pathway extend life span in wild type cells but not in mutant *sir2* strains, demonstrating that *SIR2* is a key downstream component of the caloric restriction pathway (19).

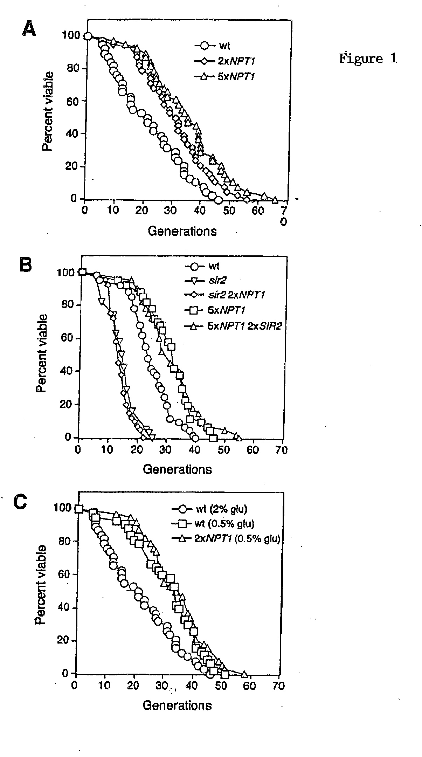

In most organisms, there are two pathways ofNAD+ biosynthesis (see Fig. 1). NAD+ may be synthesized de novo from tryptophan or recycled in four steps from nicotinamide via the NAD+ salvage pathway. The first step in the bacterial NAD⁺ salvage pathway, the hydrolysis of nicotinamide to nicotinic acid and ammonia, is catalyzed by the *pncA* gene product (33). An *S. cerevisiae* gene with homology to *pncA*, *YGL037*, was recently assigned the name *PNCl* (SGD) (34). A nicotinate phosphoribosyltransferase, encoded by the *NPT1* gene in *S. cerevisiae,* converts the nicotinic acid from this reaction to nicotinic acid mononucleotide (NaMN) (35-38). At this point, the NAD⁺ salvage pathway and the *de novo* NAD⁺ pathway converge and NaMN is converted to desamido-NAD⁺ (NaAD) by a nicotinate mononucleotide adenylyltransferase (NaMNAT). In *S. cerevisiae,* there are two putative ORFs with homology to bacterial NaMNAT genes, *YLR328* (39) and an uncharacterized ORF, *YGRO10* (23,39). We refer to these two ORFs as *NMA1* and *NMA2,* respectively. In *Salmonella*, the final step in the regeneration of NAD⁺ is catalyzed by an NAD synthetase (40). An as yet uncharacterized ORF, *QNS1,* is predicted to encode a NAD synthetase (23).

In yeast, null mutations in *NPT1* reduce steady-state NAD⁺ levels by ~2-fold (23) and abolish the longevity provided by limiting calories (19). One current hypothesis explaining how caloric restriction extends replicative life span is that decreased metabolic activity causes an increase in NAD⁺ levels, which then stimulate Sir2 activity (reviewed in Campisi, 2000 and Guarente, 2000).

Transcriptional silencing involves the heritable modification of chromatin at distinct sites in the genome. Silencing is referred to as long-range repression as it is promoter nonspecific and often encompasses an entire genomic locus (1',2'). In yeast these silent regions of DNA, which are similar to the heterochromatin of higher eukaryotes, are subject to a wide variety of modifications (3'). Among the most well studied of these modifications is the reversible acetylation of his tones (reviewed in 4',5'),

There are two classes of enzymes that affect the acetylation state of histones: histone acetyltransferases (HATs) and the opposing histone deacetylases (HDACs). Compared with more transcriptionally active areas of the genome, histones within silent regions of chromatin are known to be hypoacetylated, specifically on the NH₂-terminal tails of core histones H3 and H4 (6'). Three classes of histone deacetylases have been described and classified based on homology to yeast proteins. Proteins in class I (Rpd3-like) and class II (Hda1-like) are characterized by their sensitivity to the inhibitor trichostatin A (TSA) (7',8'). Studies using this inhibitor have provided a wealth of information regarding the cellular function of these proteins, including their involvement in the expression of regulators of cell cycle, differentiation, and apoptosis (reviewed in 9').

Yeast Sir2 is the founding member of Class III HDACs. Sir2-like deacetylases are not inhibited by TSA and have the unique characteristic of being NAD⁺-dependent (10'-13"). Proteins of this class are found in a wide array of organisms, ranging from bacteria to humans. At least two Sir2 homologues, yeast Hst2 and human SIRT2, are localized to the cytoplasm and human SIRS1 has recently been shown to target p53 for deacetylation (11',13'-15'). These results indicate that not all members of this family are specific for histones or other nuclear substrates.

The term, silent information regulator (SIR), was first coined to describe a set of non-essential genes required for repression of the mating type loci (*HML* and *HMR*) in *S. cerevisiae* (16'). Silencing in yeast is also observed at telomeres and the ribosomal DNA (rDNA) locus (2',17'). The formation of heterochromatin at mating type loci and the poly(TG₁₋₃) tracts of yeast telomeres is mediated by a heterotrimeric complex of Sir2, Sir3 and Sir4 (18',19'). At the rDNA locus, Sir2 is part of the RENT (regulator of nuleolar silencing and telophase exit) complex, which includes Net1 and Cdc14 (20',21'). Of these proteins, Sir2 is the only factor that is indispensable for silencing at all three silent regions (22'-24').

The yeast rDNA locus (*RDN1*) consists of 100-200 tandemly-repeated 9 kb units encoding ribosomal RNAs. A major cause of yeast aging has been shown to stem from recombination between these repeats (25'-27') which can lead to the excision of an extrachromosomal rDNA circle (ERC). ERCs are replicated but they fail to segregate to daughter cells, resulting in their exponential amplification as cells divide. ERCs can accumulate to a DNA content greater than that of the entire yeast genome in old cells and are thought to kill cells by titrating essential transcription and/or replication factors (28'). Although Sir2 silences Pol II-transcribed genes integrated at the rDNA, there is evidence that its primary function at this locus is to suppress recombination. Deletion of *SIR2* eliminates rDNA silencing and increases the frequency that a marker gene is recombined out of the rDNA 10-fold (29'). This results in increased ERC formation and a dramatic shortening of life span (29',30').

Sir2 is a limiting component of yeast longevity. A single extra copy of the *SIR2* gene suppresses recombination and extends life span by 40% (26",31',32'). Recently, it has been shown that *SIR2* is essential for the increased longevity provided by calorie restriction (31 "), a regimen that extends the life span of every organism it has been tested on. Moreover, increased dosage of the Sir2 homologue *sir2.1* has been shown to extend the life span of the nematode *C. elegans* (33') and the nearest human homologue SIRT1, has been shown to inhibit apoptosis through deacetylation of p53 (34',35'). These findings suggest that Sir2 and its homologues have a conserved role in the regulation of survival at the cellular and organismal level.

Recently, a great deal of insight has been gained into the biochemistry of Sir2-like deacetylases (reviewed by 36'). *In vitro*, Sir2 has specificity for lysine 16 of histone H4 and lysines 9 and 14 of histone H3 (10',12',13'). Although TSA sensitive HDACs catalyze deacetylation without the need of a cofactor, the Sir2 reaction requires NAD⁺. This allows for regulation of Sir2 activity through changes in availability of this co-substrate (10'-13'). Sir2 deacetylation is coupled to cleavage of the high-energy glycosidic bond that joins the ADP-ribose moiety of NAD⁺ to nicotinamide. Upon cleavage, Sir2 catalyzes the transfer of an acetyl group to ADP-ribose (10',11',15',37'). The product of this transfer reaction is *O-*acetyl-ADP-ribose, a novel metabolite, which has recently been shown to cause a delay/block in the cell cycle and oocyte maturation of embryos (38').

The other product of deacetylation is nicotinamide, a precursor of nicotinic acid and a form of vitamin B3 (39'). High doses of nicotinamide and nicotinic acid are often used interchangeably to self-treat a range of conditions including anxiety, osteoarthritis, psychosis, and nicotinamide is currently in clinical trials as a therapy for cancer and type I diabetes (40'). The long-term safety of the high doses used in these treatments has been questioned (41') and the possible effects of these compounds at the molecular level are not clear.

### Summary of the invention

In one embodidment, the invention provides methods for modulating the life span of a cell or its resistance to stress, comprising modulating the flux through the NAD+ salvage pathway in the cell. The method may comprise increasing or extending the life of a cell or increasing its resistance against stress, comprising increasing the flux through the NAD+ salvage pathway in the cell. Modulating the flux through the NAD+ salvage pathway may occur essentially without changing steady state levels of NAD+ and NADH and essentially by maintaining the NAD+/NADH ratio in the cell.

Increasing the flux throught the NAD+ salvage pathway may comprise increasing the level or activity of a protein selected from the group consisting of NPT1, PNC1, NMA1 and NMA2. The method may comprise introducing into the cell at least one nucleic acid encoding a protein selected from the group consisting of NPT1, PNC1, NMA1 and NMA2, or a nucleic acid comprising at least 5 copies of a gene. Alternatively, the method may comprise introducing into the cell at least one protein selected from the group consisting of NPT1, PNC1, NMA1 and NMA2. The method may comprise contacting the cell with an agent that upregulates the expression of a gene selected from the group consisting of NPT1, PNC 1, NMA1 and NMA2. The cell may live at least about 40% longer, or at least about 60% longer.

The invention also provides methods for increasing the resistance of the cell against stress, e.g., heat shock, osmotic stress, DNA damaging agents (e.g., U.V.), and inadequate nitrogen levels, comprising increasing the flux through the NAD+ salvage pathway in the cell.

In one embodiment, modulating the life span of a cell comprises modulating silencing in the cell. Silencing may include telomeric silencing and rDNA recombination.

The cell whose life span can be extended or who can be protected against stress can be a eukaryotic cell, such as a yeast cell or a prokaryotic cell, such as a bacterial cell. The cell can be *in vitro* or *in vivo.*

In another embodiment, modulating the life span of a cell or its resistance to stress comprises modulating the amount of nicotinamide in the cell. For example, reducing the life span of a cell or rendering a cell more sensitive to stress may comprise increasing the level of nicotinamide in the cell. This may comprise contacting the cell with an amount of nicotinamide of about 1 to 20 mM, preferably of about 2 to 10 mM. The level of nicotinamide in a cell may also be increased by increasing the level or activity of enzymes involved in the biosynthesis of nicotinamide or by decreasing the level or activity of enzymes that degrade or inactivate nicotinamide. Enzymes which inactivate nicotinamide include PNC1; nicotinamide N-methyl transferase (NNMT and NNT1); nicotinamide phosphoribosyltransferase (NAMPRT); NPT1 and human homologs thereof; and optionally nicotinamide mononucleotide adenylyltransferase (NMNAT-1 and 2); NMA1 and 2 and human homologs thereof.

On the contrary, extending the life span of a cell or rendering the cell more resistant (i.e., less sensitive) to stress may comprise decreasing the level of nicotinamide in the cell. This may be achieved by decreasing the level or activity of enzymes involved in the biosynthesis of nicotinamide or by increasing the level or activity of enzymes that degrade or inactivate nicotinamide. Accordingly, increasing lifespan or stress resistance in a cell can be achieved by increasing the activity or level of expression of a protein selected from the group consisting of NPT1, PNC1, NMA1, NMA2, NNMT, NAMPRT, NMNAT-1, and NMNAT-2.

The invention further provides methods for identifying compounds that modulate the life span of a cell or its resistance to stress, comprising (i) contacting a protein selected from the group consisting of NPT1, PNC1, NMA1, NMA2, NNMT, NAMPRT, NMNAT-1, and NMNAT-2 with a test compound for an amount of time that would be sufficient to affect the activity of the protein; and (ii) determining the activity of the enzyme, wherein a difference in the activity of the enzyme in the presence of the test compound relative to the absence of the test compound indicates that the test compound is a compound that modulates the life span of the cell or its resistance to stress. The method may further comprise contacting a cell with the test compound and determining whether the life span of the cell has been modulated. The method may also further comprise contacting a cell with the test compound and determining whether the resistance of the cell to stress has been modulated.

In another embodiment, the invention provides a method for identifying a compound that modulates the life span of a cell or its resistance to certain types of stresses, comprising (i) contacting a cell or a lysate, comprising a transcriptional regulatory nucleic acid of a gene selected from the group consisting of NPT1, PNC1, NMA1, NMA2, NNMT, NAMPRT, NMNAT-1, and NMNAT-2 operably linked to a reporter gene, with a test compound for an amount of time that would be sufficient to affect the transcriptional regulatory nucleic acid; and (ii) determining the level or activity of the reporter gene, wherein a difference in the level or activity of the reporter gene in the presence of the test compound relative to the absence of the test compound indicates that the test compound is a compound that modulates the life span of the cell or its resistance to certain types of stresses. The method may further comprise contacting a cell with the test compound and determining whether the life span of the cell has been modulated. The method may also further comprise contacting a cell with the test compound and determining whether the resistance of the cell to stress has been modulated.

Also provided herein are methods for identifying an agent, e.g., a small molecule that modulates the nicotinamide level in a cell. The method may comprise (i) providing a cell or cell lysate comprising a reporter construct that is sensitive to the level of nicotinamide in a cell; (ii) contacting the cell with a test agent; and (iii) determining the level of nicotinamide in the cell contacted with the test agent, wherein a different level of nicotinamide in the cell treated with the test agent relative to a cell not treated with the test agent indicates that the test agent modulates the level of nicotinamide in the cell. The cell may further comprise a vector encoding a fusion protein that can bind to a DNA binding element operably linked to the reporter gene. The fusion protein may comprise at least an NAD+ binding pocket of a nicotinamide sensitive enzyme, e.g., a Sir2 family member, and a heterologous polypeptide. The heterologous polypeptide may be a transactivation domain of a transcription factor. The method may further comprise contacting a cell with the test compound and determining whether the life span of the cell or its resistance to stress has been modulated.

Also within the scope of the invention are computer-assisted methods for identifying an inhibitor of the activity of a Sir2 family member comprising: (i) supplying a computer modeling application with a set of structure coordinates of a molecule or molecular complex, the molecule or molecular complex including at least a portion of a Sir2 family member comprising a C pocket; (ii) supplying the computer modeling application with a set of structure coordinates of a chemical entity; and (iii) determining whether the chemical entity is an inhibitor expected to bind to or interfere with the molecule or molecular complex, wherein binding to or interfering with the molecule or molecular complex is indicative of potential inhibition of the activity of the Sir2 family member. The chemical entity may be an analog of nicotinamide. Another method for identifying an inhibitor of the activity of a Sir2 family member comprises: (i) contacting a protein of the Sir2 family comprising at least the C pocket with a test compound for a time sufficient for the test compound to potentially bind to the C pocket of the protein of the Sir2 family; and (ii) determining the activity of protein; wherein a lower activity of the protein in the presence of the test compound relative to the absence of the test compound indicates that the test compound is an inhibitor of the activity of a Sir2 family member.

In addition, the invention provides methods for treating or preventing diseases that are associated with cell death (e.g., apoptosis) in a subject, comprising administering to a subject in need thereof an agent that increases the flux through the NAD+ salvage pathway or reduces nicotinamide levels in the cells susceptible or subject to cell death. Diseases can be chronic or acute and include Alzheimer's disease, Parkinson's disease, stroke and myocardial infarction. The methods of the invention for extending life span or increasing resistance to stress can also be used to reduce aging, e.g., for cosmetic purposes. The agent can be administered locally or systemically. Methods for extending life span or increasing resistance to stress can also be used on cells, tissues or organs outside of a subject, e.g., in an organ or tissue prior to transplantation.

The invention also provides methods for treating or preventing diseases in which reducing the life span of cells or rendering cell sensitive to stress is beneficial. Such diseases include those in which cells are undesirable, e.g., cancer and autoimmune diseases.

The methods of the invention can also be used to modulate the lifespan and stress resistance of organisms other than mammals. For example, the method can be used in microorganisms and plants. In particular, the methods of the invention permit to increase the resistance of plants to high salt, drought or disease, e.g., by treating these with a chemical that lowers nicotinamide levels or by genetically modifying genes that modulate the NAD+ salvage pathway or the level of nicotinamide in cells.

### Brief description of the drawings

FIG. 1. Increased dosage of *NPT1* delays aging by mimicking caloric restriction. Life span was determined by scoring the number of daughter cells produced by each mother cell before cessation of cell division (7,10). Cells were pre-grown for a minimum of 48 h on complete glucose medium.
   A, Mortality curves for wild type (PSY316AT, circles), 2x*NPT1* (YDS1544, diamonds) and 5x*NPT1* (YDS1548, triangles) on medium with 2% glucose. Average life spans are 21.9, 30.8 and 35.1 generations respectively.
   B, Mortality curves for wild type (PSY316AT, circles), *sir2::TRP1* (YDS1594, downward triangles), 2x*NPT1* (YDS1544, squares), *sir2::TRP1* 2x*NPT1* (YDS1573, diamonds) and 5x*NPT1 2xSIR2* (YDS1577, upward triangles) on 2% glucose medium. Average life spans were 23.7, 14.4, 13.9, 31.0 and 31.9 generations respectively.
   C, Mortality curves for wild type on 2% glucose (PSY316AT, circles) and 0.5% glucose medium (PSY316AT, squares) and for *2xNPT1* on 0.5% glucose medium (YDS1544, triangles). Average life spans are 21.9, 31.7 and 34.5 generations respectively.

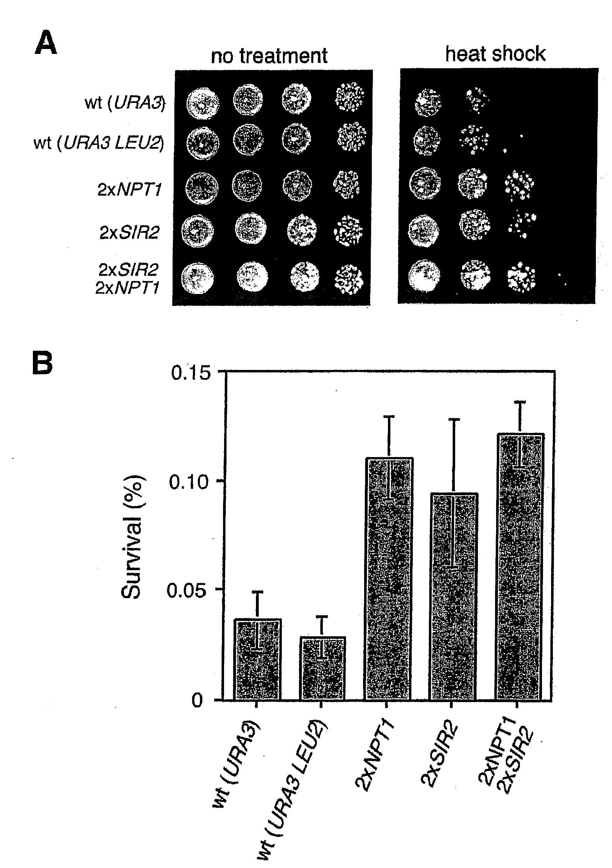

FIG. 2. *NPT1* and *SIR2* provide resistance to heat shock. A, Strains were grown for three days post-diauxic shift in SC medium and incubated for 1 h at 55°C before plating 10-fold dilutions on SC plates. .*B,* Strains were treated as in *A* and plated on SC at low density. Colonies that aroze after 24 hours were scored and expressed as a percentage of colonies arizing from the untreated sample. Values represent the avarage of three independent experiments (+/- s.d.). Strains: W303AR *URA3* (YDS1568), W303AR *URA3 LEU2* (YDS1563) and isogenic derivatives of W303AR, 2x*NPT1-URA3* (YDS1503), *2xSIR2-URA3* (YDS1572) and 2x*NPT1-URA3* 2x*SIR2-LEU2* (YDS1561).

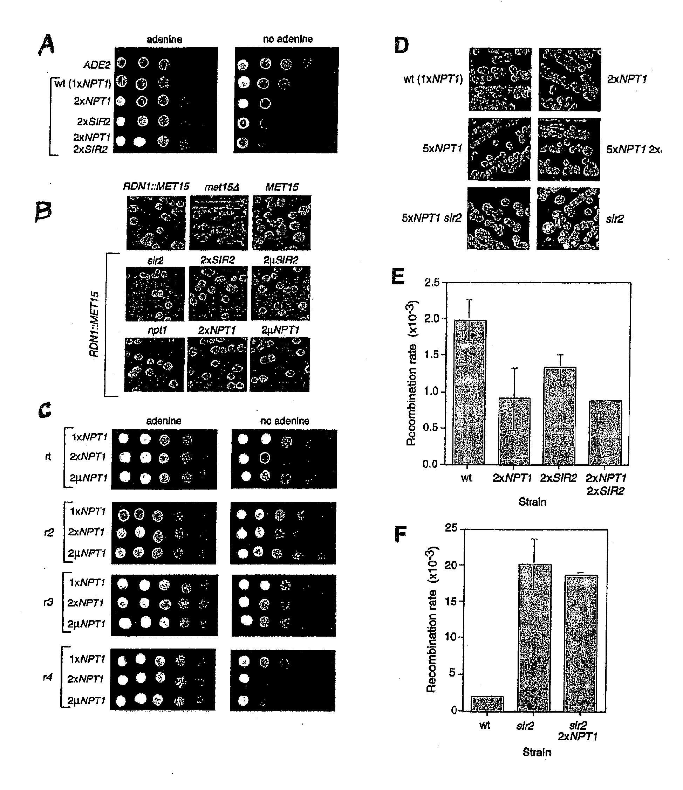

FIG. 3. Additional *NPT1* increases silencing and rDNA stability. *A*, Strains with an *ADE2* marker at the rDNA were pre-grown on SC plates and spotted as 10-fold serial dilutions on SC plates. Increased silencing is indicated by growth retardation on media lacking adenine. Strains: W303-1A *ADE2* (YDS1596), W303-1A *RDN1::ADE2* (W303AR5) and W303AR5 derivatives 2x*NPT1* (YDS1503), 2x*SIR2* (YDS1572) and 2x*NPT1 2xSIR2* (YDS1561). *B,* Silencing of *MET15* art the rDNA locus was assayed by streaking isogenic derivatives of JS237 on rich medium containing 0.07% PbNO₃ and incubating for 5 days at 30°C. Increased silencing is indicated by accumulation of a brown pigment. Relevant genotypes: *met15Δ* (JS209), *MET15* (JS241), *RND1::MET15* (JS237), *sir2::TRP1* (JS218), 2x*SIR2* (YDS1583), 2µ*SIR2* (YDS1522), *npt1Δ::kan*^{r}(YDS1580), 2x*NPT1* (YDS1581) and 2µ*NPT1* (YDS1493). C, Silencing of an *ADE2* marker at the rDNA locus was determined in strains with 1x*NPT1*, 2x*NPT1*, and 2µ*NPT1* in the following backgrounds: wild type (W303AR5, YDS1503, YDS1496), *sir2::TRP1* (YDS878, YDS1504, YDS1494), *sir3::HIS3* (YDS924, YDS1505, YDS1587), and *sir4::HIS3* (YDS882, YDS 1506, YDS1495). *D*, Strains with an *ADE2* marker at the telomere were streaked onto SC medium containing limiting amounts of adenine. Increased silencing is indicated by accumulation of red pigment. Relevant genotypes: (PSY316AT), *2xNPT1* (YDS 1544), 5x*NPT1* (YDS 1548), 5x*NPT1* 2x*SIR2* (YDS1577) and 5x*NPT1 SIR2::TRP1* (YDS1573). *sir2::TRP1* (YDS1594). *E,* Strains in *A* were assayed for rDNA stability by examining the rate of loss of an *ADE2* marker integrated at the rDNA locus. Cells were plated on YPD medium and the frequency of half-sectored colonies, reflecting a marker loss event at the first cell division, was measured. More than 10,000 colonies were examined for each strain and each experiment was performed in triplicate. Average recombination frequencies (+/- s.d.) per cell division are shown. *F,* Ribosomal DNA recombination rates for wild type (W303AR), *sir2::TRP1* (YDS878) and 2x*NPT1 sir2::TRP1* (YDS1504) strains. Assays were performed as in (E).

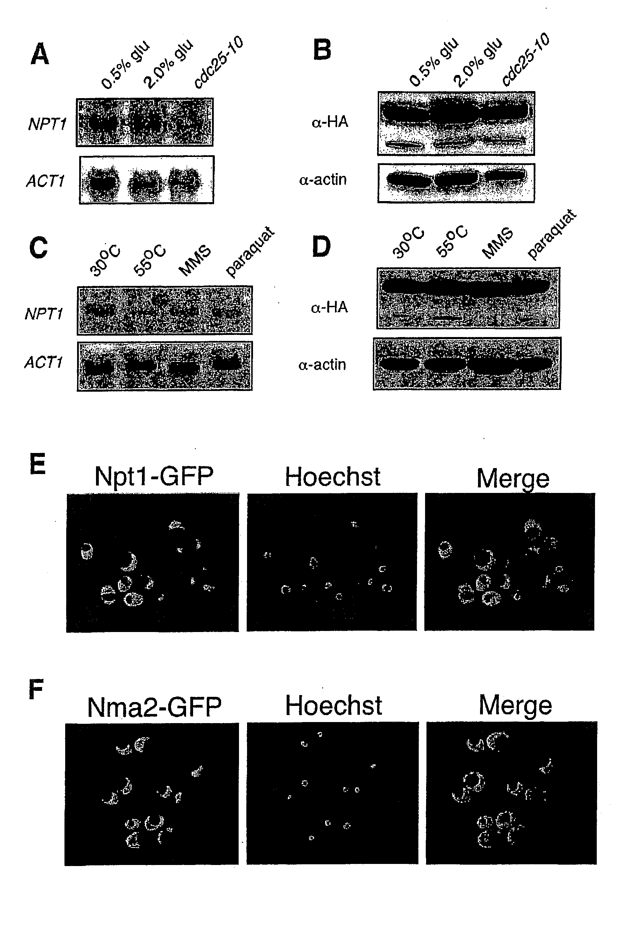

FIG. 4. Expression of *NPT1* in response to caloric restriction and stress. *A*, 3xHA tag sequence was inserted in frame with the 3' end of the native *NPT1* ORF in W303AR5 (YDS1531) and W303cdc10-25 (YDS1537). Cells were grown in YPD medium at 30°C and treated as described. Levels of *NPT1* mRNA were examined for W303AR5 grown in YPD (0.5% and 2.0% glucose) and *W303cdc25-10* grown in YPD (2% glucose). A 1.8 kb *NPT1* transcript was detected and levels were normalized to actin (*ACT1*) control. Similar results were obtained in the PSY316 strain background (not shown). *B*, Protein extracts from cultures in *A* were analyzed by Western blot to detect the HA-tagged Npt1 using α-HA antibody. Two bands of 53 kD and 40 kD were detected in the Npt1-HA strains and no bands were detected in the untagged control strain (not shown). Actin levels served as a loading control. Similar results were obtained in the PSY316 strain background (not shown). *C*, Levels of *NPT1* mRNA were examined in wild type W303AR5 (YDS1531) log phase cultures after 1 h exposure to the following: MMS (0.02% v/v), paraquat (5mM), or heat shock (55°C). *D*, Protein extracts of cultures in *C* were analyzed as in *B. E* and *F*. A green fluorescent protein (GFP) sequence was inserted in-frame at the 3' end of the native *NPT1* and *NMA2* ORFs in W303AR5 (YDS1611 and YDS1624, respectively). Cells were grown in YPD medium at 30°C to mid log phase and photographed live. Regions of overlap between GFP (green) and Hoechst DNA stain (false color red) appear yellow in the merged image.

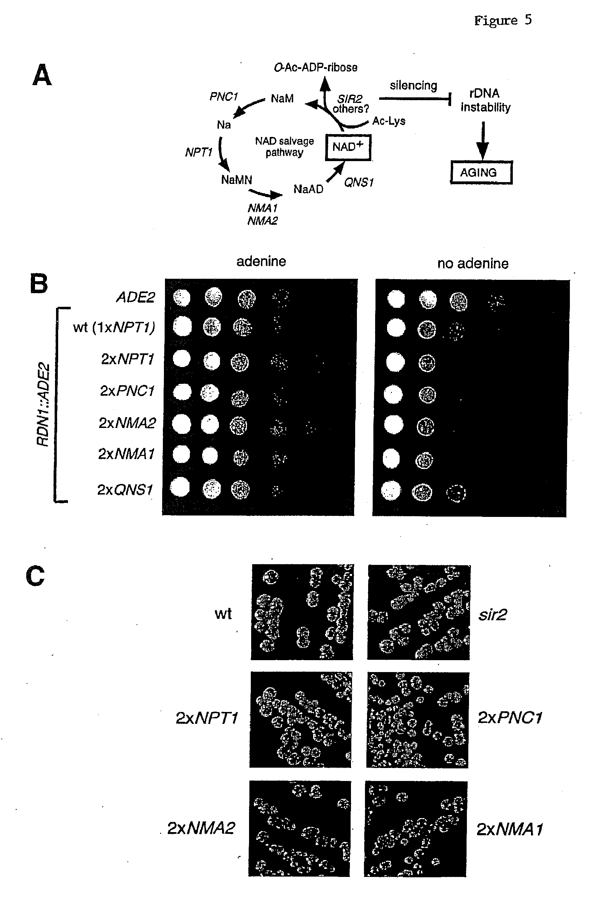

FIG. 5. Multiple limiting components in the NAD⁺ salvage pathway. *A*, The putative steps in NAD⁺ biosynthesis in *S. cerevisiae* based on the known steps in *Salmonella.* The yeast genes that are thought to mediate each step are shown in italics. NaMN, nicotinic acid mononucleotide; NaAD, desamido-NAD⁺; NaM, nicotinamide; Na, nicotinic acid. Adapted from Smith *et al.* (2000). *B,* Silencing of *ADE2* at the rDNA locus in strains *ADE2* (YDS1596), wild type (W303AR5), 2x*NPT1* (YDS1503), 2x*PNC1* (YDS1588), 2x*NMA2* (YDS1589), 2x*NMA1* (YDS1590), and 2x*QNS1* (YDS1614). Increased silencing is indicated by growth retardation on media lacking adenine. *C*, Strains with an *ADE2* marker at the telomere were streaked onto SC medium containing limiting amounts of adenine. Silencing is indicated by the accumulation of a red pigment. Strains tested: wild type (PSY316AT), 2x*NPT1* (YDS1544), 5x*NPT1* (YDS 1548), *sir2::TRP1* (YDS1594), 2x*PNC1* (YDS1591), 2x*NMA2* (YDS1592) and 2x*NMA1* (YDS1593).

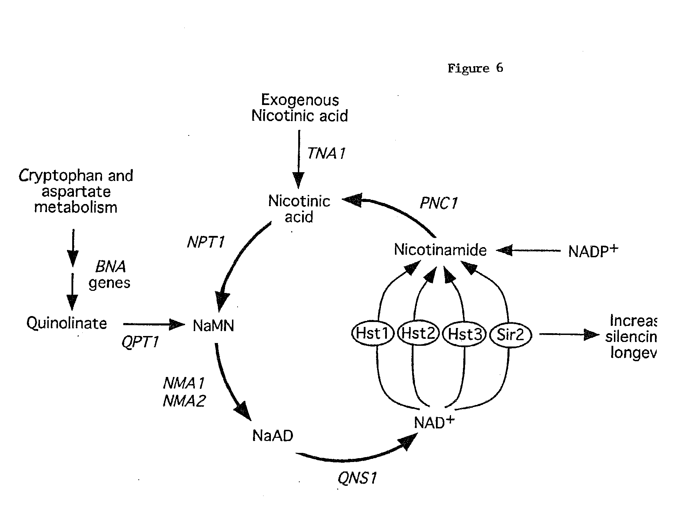

FIG. 6. Model for life span extension via increased flux through the NAD⁺ salvage pathway. Type III histone deacetylases such as Sir2 and Hstl-4 catalyze a key step in the salvage pathway by converting NAD⁺ to nicotinamide. Additional copies of *PNC1*, *NPT1*, *NMA1* and *NMA2* increase flux through the NAD⁺ salvage pathway, which stimulates Sir2 activity and increases life span. Additional copies of *QNS1* fail to increase silencing because, unlike other steps in the pathway, its substrate cannot be supplied from a source outside the salvage pathway and is therefore limiting for the reaction. Abbreviations: NAD⁺, nicotinamide adenine dinucleotide; NaMN, nicotinic acid mononucleotide; NaAD, desamido-NAD⁺.

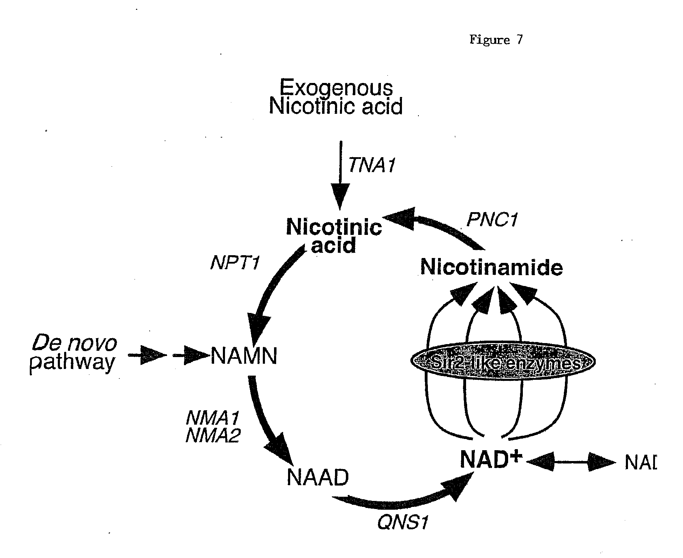

FIG. 7. The NAD⁺ salvage pathway. Nicotinamide generated by Sir2 is converted into nicotinic acid by Pnc 1 and subsequently back to NAD⁺ in three steps. Abbreviations: NAD⁺, nicotinamide adenine dinucleotide; NaMN, nicotinic acid mononucleotide; NaAD, desamido-NAD⁺.

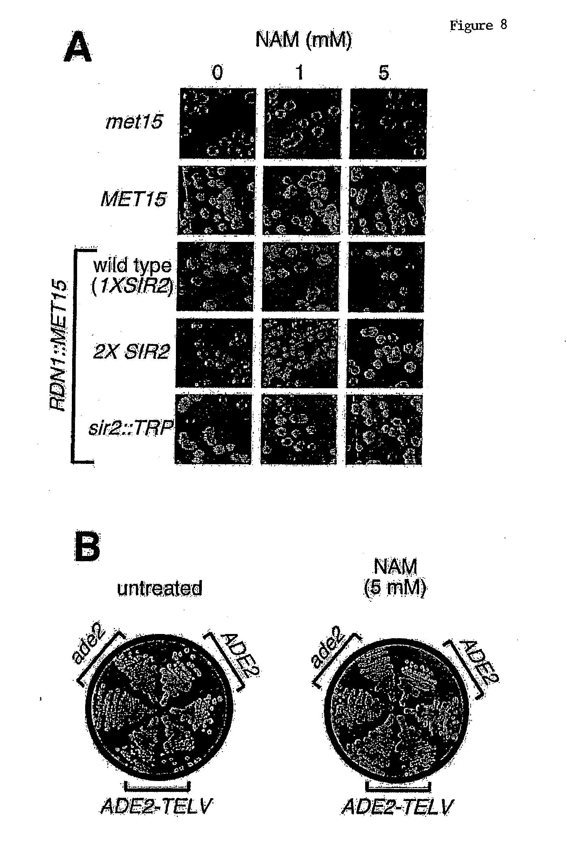

FIG. 8. Nicotinamide inhibits telomeric and rDNA silencing. *A*, Silencing at the rDNA locus was assayed by streaking isogenic derivatives of JS237 (*RDN1::METI5*) on rich medium containing 0.07% PbNO₃ and either 0, 1, or 5 mM nicotinamide. Silencing of the *MET15* marker is indicated by the accumulation of a brown pigment. Single dark brown colonies in *RDN1::MET15* strains represent marker loss events. Relevant genotypes: *met15Δ* (JS209), *MET15* (JS241), *RDN1::MET15* (JS237), *sir2::TRP1* (JS218), *2xSIR2* (YDS 1583). *B*, Strains with *an ADE2* marker at the telomere were streaked onto SC medium containing limiting amounts of adenine and either 0 or 5 mM nicotinamide. Silencing of the *ADE2* marker results in the accumulation of a red pigment. Relevant genotypes: (PSY316AT), W303-1A *ADE2* (YDS1596) and W303-1A *ade2* (YDS1595).

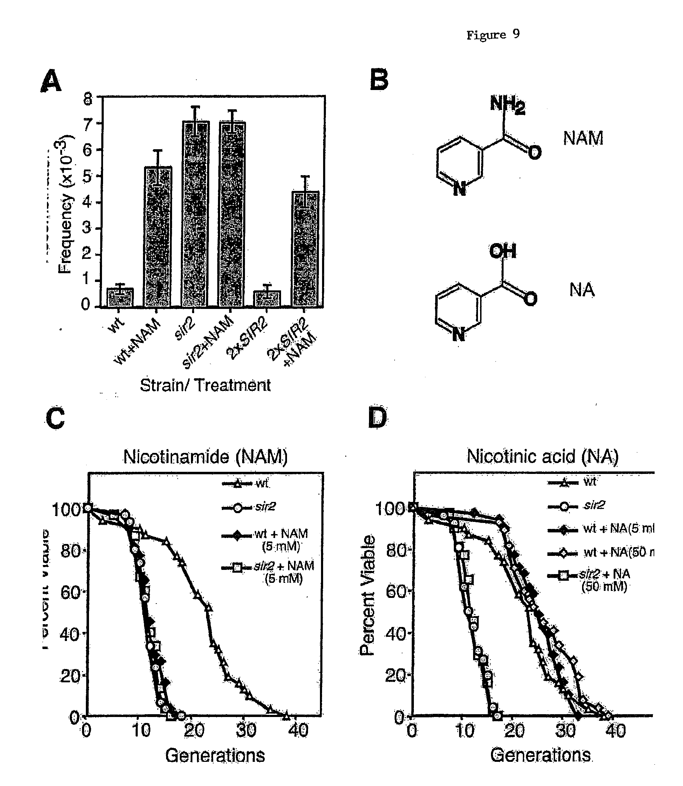

FIG.9. Nicotinamide increases rDNA recombination and shortens yeast life span. *A*, Strains were assayed for rDNA stability by examining the rate of loss of an *ADE2* marker integrated at the rDNA locus. Cells were plated on 2% glucose YPD medium with or without 5 mM nicotinamide (NAM) and the frequency of half-sectored colonies, reflecting a marker loss event at the first cell division, was measured. More than 10,000 colonies were examined for each strain and each experiment was performed in triplicate. Average recombination frequencies (+/- s.d.) per cell division are shown. Relevant strains: *W303-1A RDN1::ADE2* (W303AR5) and W303AR5 derivatives 2x*SIR2* (YDS1572) and *sir2::TRP1* (YDS878). *B*, Comparison of structures for nicotinamide (NAM) and nicotinic acid (NA). *C and D,* Life spans were determined by scoring the number of daughter cells produced by each mother cell before cessation of cell division (68',69'). Cells were pre-grown for a minimum of 48 h on complete glucose medium. *C*, Mortality curves for wild type (PSY316AT) and *sir2::TRP1* (YDS 1594) strains in 0 or 5 mM nicotinamide (NAM). Average life spans were wt: 22.4, 12.1 and *Sir2*: 12.1, 11.7 respectively. *D*, Mortality curves for wild type and *sir2* strains from *C*, in the presence of either 0, 5 mM or 50 mM nicotinic acid (NA). Average life spans were wt: 22.4, 26, 25 and *sir2*: 12.1, 12.2.

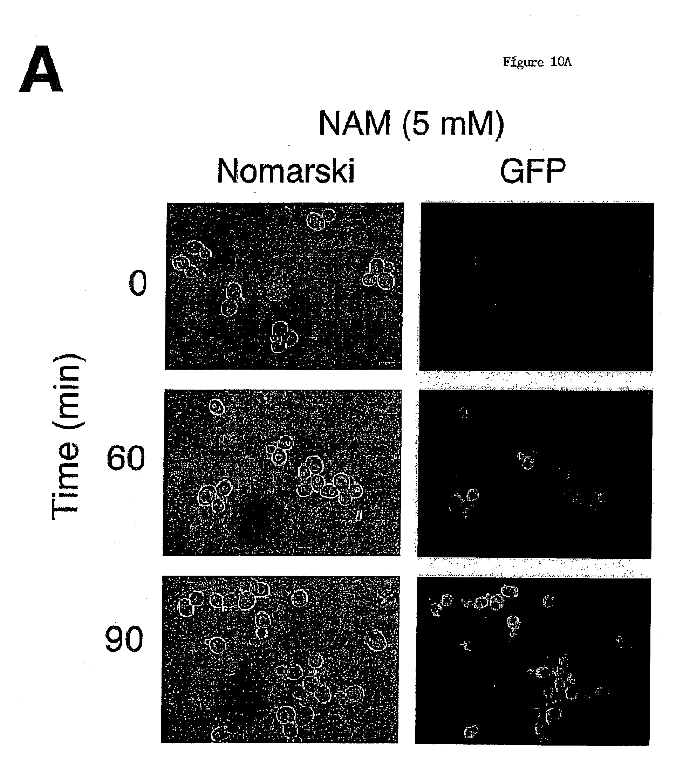

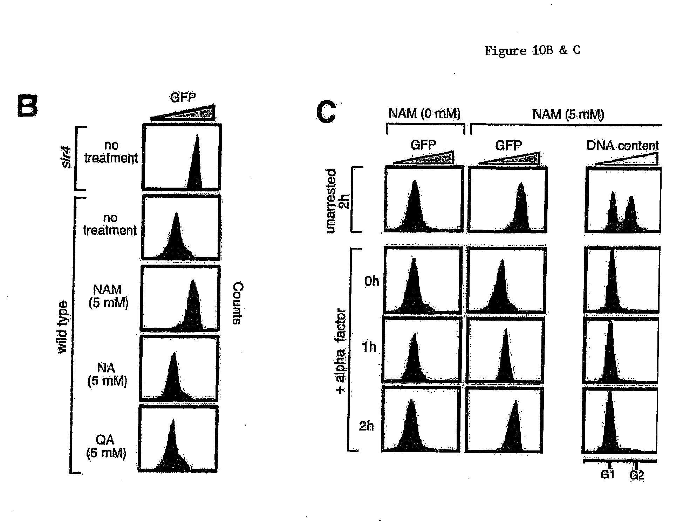

FIG.10. Nicotinamide derepresses the silent mating type locus (*HMR*) in the both cycling and G1 arrested cells. *A,* PSY316 cells containing an *ADH* driven *GFP* transcript inserted at the *HMR* locus (YDS970) were grown in YPD medium at 30°C to mid-log phase and treated with 5 mM nicotinamide (NAM) for the indicated times. Cells were photographed live. *B*, Strain YDS970 or the isogenic *sir4Δ* mutant (YDS1499) were treated with either 5 mM nicotinamide (NAM), 5 mM nicotinic acid (NA) or 5 mM quinolinic acid (QA). Cells were analyzed by fluorescent activated cell sorting (FACS) to determine the extent of *ADH-GFP* expression. *C*, *AMAT***a** derivative of strain YDS970 (YDS1005) was deleted for *HML* and treated with 10 µg/ml alpha-factor for 3 hours. Cells were then grown in the presence of 5 mM nicotinamide for the indicated times and examined by FACS as above. Cell cycle progression was monitored at each time point by FACS analysis of propidium iodide stained cells.

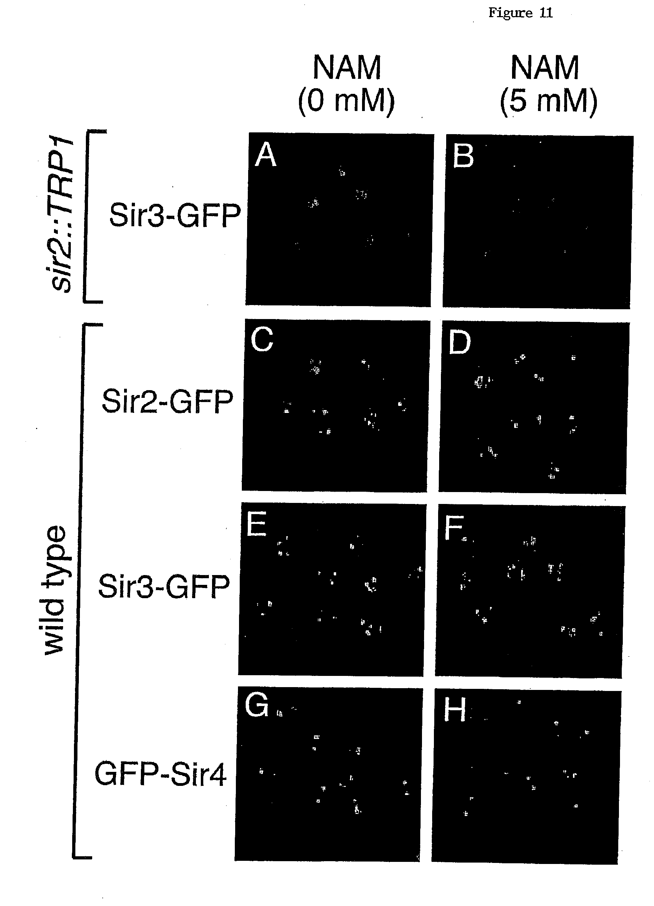

FIG.11. Nicotinamide does not alter the localization of Sir proteins. Wild type strains containing either *SIR2-GFP* (YDS 1078) (*C and D), SIR3-GFP* (YDS1099) (*E and F*), or *GFP-SIR4* (YDS1097) (*G and H*) and an isogenic *sir2* derivative expressing *SIR3-GFP* (YDS 1109) (*A and B*), were grown for 2 hours in the presence of 5 mM nicotinamide. GFP fluorescence was detected in live cells.

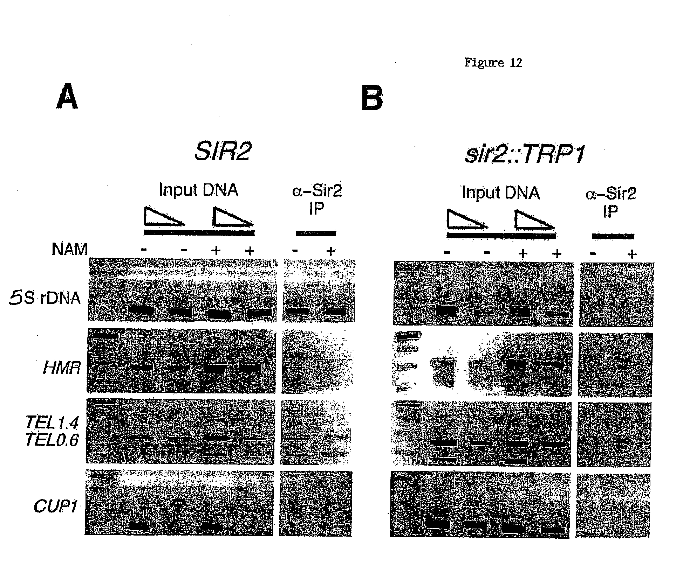

FIG.12. Sir2 does not associate with DNA from telomeres or mating type loci in the presence of nicotinamide. *A and B,* Chromatin immunoprecipitation using a polyclonal α-Sir2 antibody was performed on extracts from either a *sir2* (YDS878) (*A*) or wild type (W303AR5) (*B*) strains in the presence of 5 mM nicotinamide (NAM). PCR amplification of both input DNA from whole cell extracts and immunoprecipitated chromatin are shown. PCR was performed using primer pairs specific for the *CUP1* gene (top panels), 5S rDNA (second panels), the *HMR* locus (third panels), or subtelomeric DNA 1.4 and 0.6 kb from telomeres (bottom panels). Primer sequences are listed in Table 4.

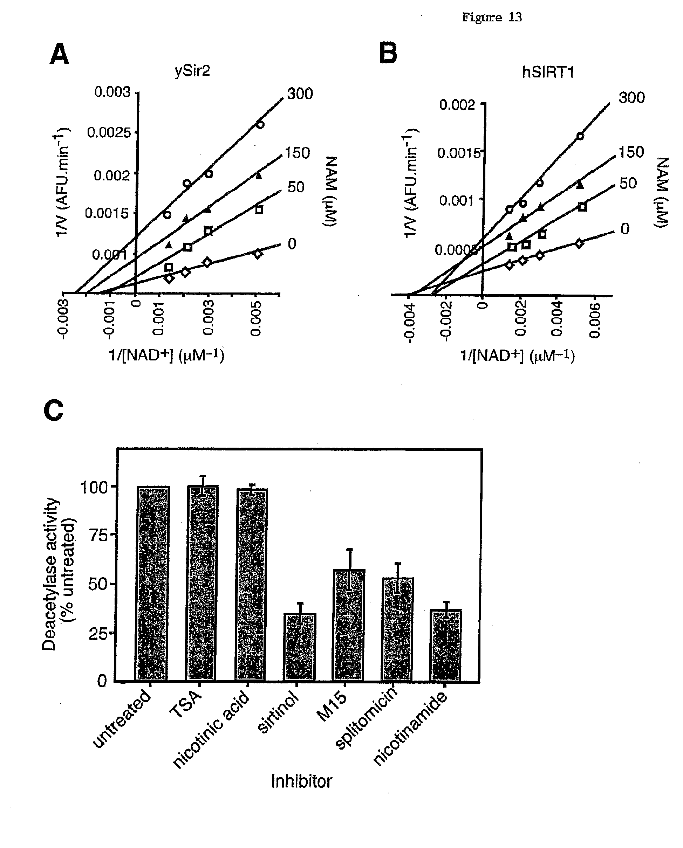

FIG.13. Nicotinamide is a potent non-competitive inhibitor of yeast Sir2 and human SIRT1 *in vitro. A,* Recombinant GST-tagged Sir2 was incubated with acetylated substrate for 30 minutes at 30°C in the presence of 1 mM DTT, 200, 350, 500 or 750 µM NAD⁺ and the indicated concentrations of nicotinamide. Reactions were terminated by the addition of developer and samples were analyzed by fluorometry (excitation set at 360 nm and emission at 460 nm). Experiments were performed in triplicate. Data is shown as a Lineweaver-Burk double reciprocal plot of arbitrary fluorescence units (AFUs) min⁻¹ versus NAD⁺ (µM). *B,* Experiments were performed as in *A*, except that recombinant human SIRT1 was used and reactions were carried out at 37°C. *C*, Deacetylation reactions were performed in triplicate with 2.5 µg of SIRT1, 1 mM DTT, 200 µM NAD⁺ and either 50 µM water blank, DMSO blank, nicotinic acid, sirtinol, M15, splitomicin or nicotinamide. Reactions were carried out at 37°C for 30 minutes and fluorescence was measured as in *A.*

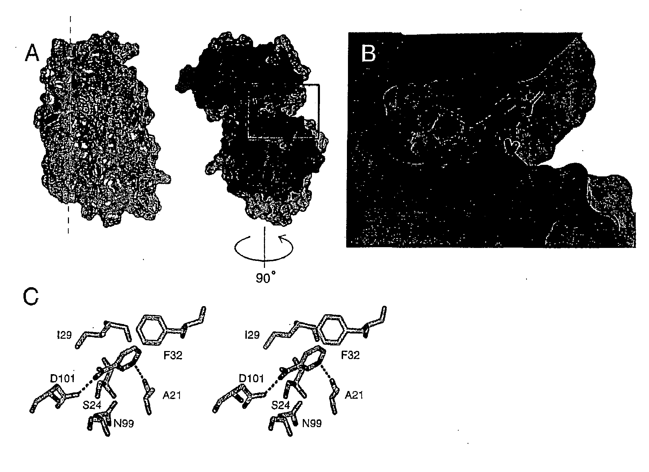

Fig. 14A-C. Nicotinamide docked in the conserved C pocket of Sir2-Afl. (A) The left panel shows a frontal view of the surface representation of Sir2-Afl, with bound NAD⁺ in purple and a red arrow pointing at the acetyl-lysine binding tunnel. The C site is traced with a dashed teal curve. The right panel shows the protein cut through the dashed line and rotated 90 degrees along its vertical axis. The surface of the conserved residues in the C site is colored teal. (B) Close-up view of the black rectangle drawn on the right panel of A, showing the nicotinamide docked deeply inside the C pocket of Sir2-Afl. (C) Stereo view of the docked nicotinamide (green) surrounded by the conserved residues in the C pocket. The putative interactions are shown as dashed lines, including H-bonds (blue), electrostatic (magenta) and Van der Waals (yellow).

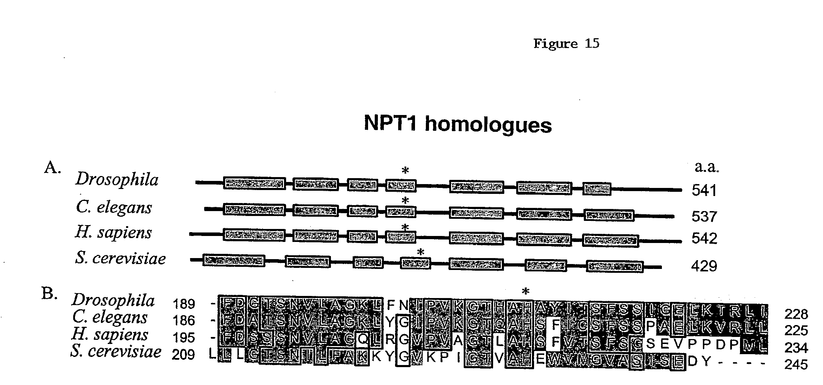

Fig. 15 shows an alignment of NPT1 homologs.

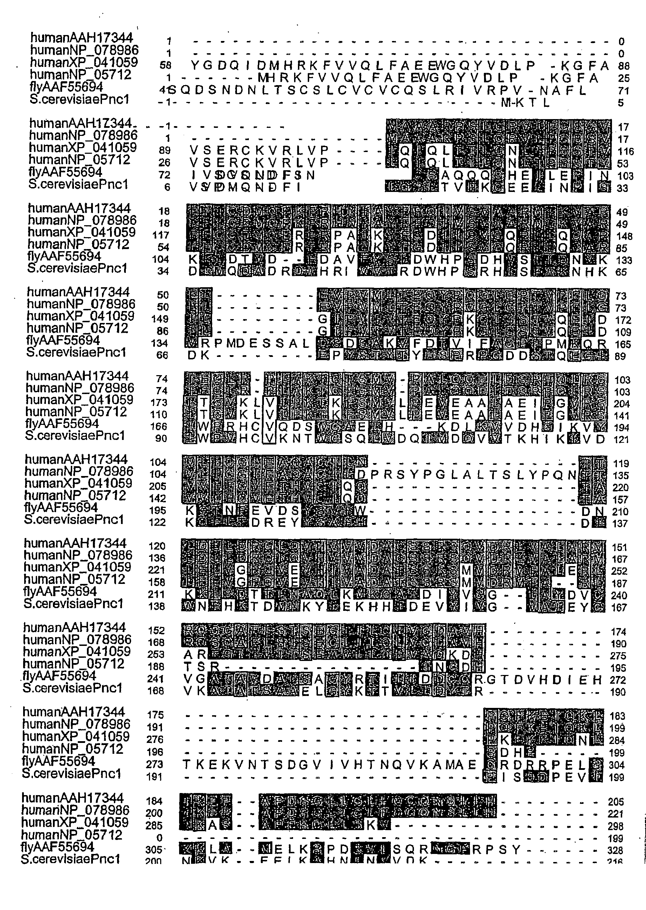

Fig. 16 shows an alignment of PNC1 homologs.

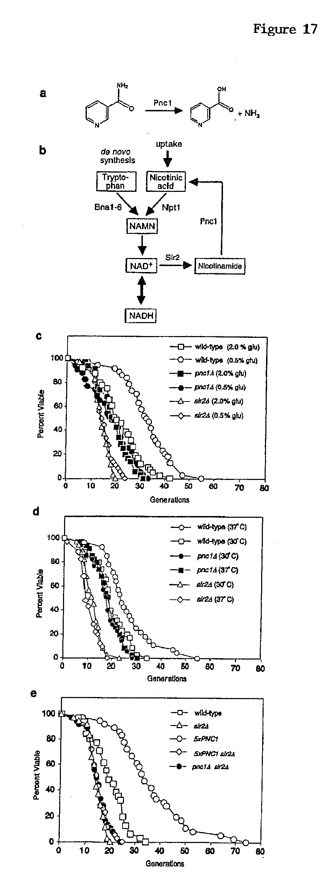

Fig. 17A-E. Calorie restriction and heat stress extend lifespan in a *PNC1*-dependent manner. (A) Pnc1 catalyses the conversion of nicotinamide to nicotinic acid. (B) In yeast NAD⁺ is synthesised *de novo* from tryptophan and recycled from nicotinamide via the NAD⁺ salvage pathway. (C) Lifespan extension by glucose restriction requires *PNC1.* Average lifespan on complete media containing 2.0% (w/v) glucose were: wild-type, *(21.6); pnc1 Δ,* (19.1); *sir2 Δ,* (14.2). Average lifespans on 0.5% glucose were: wild-type, *(32.7);pnc1 Δ,* (18.1); *sir2 Δ,* (14.7). (D) Extension of life span by exposure to mild heat stress. At 30°C, average lifespans were: wild-type, (19.4); *pnc1 Δ,* (18.5); *sir2 Δ,* (12.0). At 37°C, average lifespans were: wild-type, *(23.4); pnc1 Δ,* (17.5); *sir2Δ,* (10.6). (E) Additional *PNCl* extends lifespan in a *SIR2*-dependent manner. Average lifespans on 2.0% glucose/30°C: wild-type, (19.7); *5xPNC1,* (36.1); *sir2Δ,* (14.2); *5xPNC1 sir2Δ,* (15.1); *pnc1 Δsir2Δ, (14.4).*

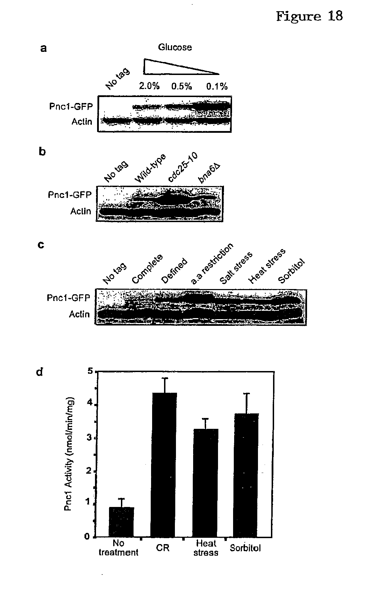

Figure 18A-D. Pnc1 levels and activity are elevated in response to calorie restriction and stress. (A) Detection of Pnc1-GFP in yeast whole cell extracts using an anti-GFP antibody. Actin levels are included as a loading control. Extracts were made from mid-log phase wild-type cultures grown in complete media with 2.0%, 0.5% or 0.1 % glucose (w/v). (B) Pnc1-GFP levels in extracts from mid-log phase wild-type, *cdc25-10* or *bna6Δ* cultures detected as above. (C) Detection of Pnc1-GFP in extracts from mid-log phase wild-type cultures as described above. Cultures were grown under the following conditions: complete medium (no treatment), defined medium (SD), amino acid (a.a.) restriction (SD lacking non-essential amino acids), salt stress (NaCl, 300 mM), heat stress (37°C), sorbitol (1M). (D) Measurement of nicotinamide deamination by Pnc 1 from cell extracts of mid-log phase wild-type cultures grown under the indicated conditions. Values shown are the average of three independent experiments. Activity is expressed as nmol ammonia produced/min/mg of total protein, ± s.d: 2.0% glucose 0.90 ± 0.26, 0.1% glucose 4.38 ± 0.43, 37°C 3.28 ± 0.32, sorbitol (1M) 3.75 ± 0.65.

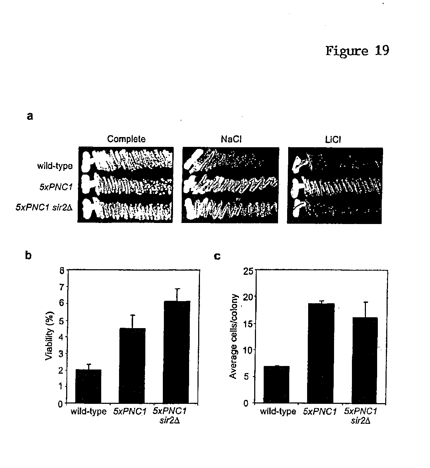

Fig. 19A-C. *PNCl* confers resistance to acute stress. (A) Additional *PNCl* confers resistance to salt stress. Cells from mid-log phase colonies were struck out on complete medium containing 600 mM NaCl or 200 mM LiCl and incubated for 4 d at 25°C. On standard yeast medium (2% glucose, 25°C), there was no detectable difference in growth rate between wild-type, *5xPNC1,* or *5xPNC1 sir2Δ* strains. (B) Additional *PNC1* protects against UV induced damage in a *SIR2* independent manner. Cells from mid-log phase cultures were plated at low density on complete medium and exposed to UV (5 mJ/cm², 254nm). Viability was determined by the ability to form colonies after 3 d growth in the dark at 30°C. Values are expressed as percent viable ± s.e. (C) *PNC1* provides *SIR2-*independent protection against mitochondrial DNA damage. Microcolony analysis of log-phase cells streaked on complete 3% (v/v) glycerol medium and 10 µg/ml ethidium bromide (EtBr). At least 100 microcolonies were scored after 3 d in two independent experiments. Number of cells per colony ± s.e. were: wild-type 6.92 ± 0.06, *5xPNC1* 18.72 ± 0.53, and *5xPNC1 sir2Δ* 16:15 ± 2.82. No difference in growth was detected between these strains on complete 2% (w/v) glucose medium with EtBr

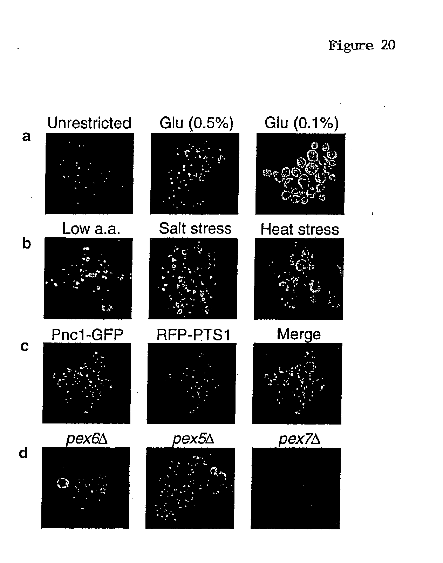

Fig. 20A-D. Pnc1-GFP is localized in the cytoplasm and nucleus and is concentrated in peroxisomes. (A) Pnc1-GFP fluorescence was detected in cells taken from mid-log phase wild-type cultures grown in complete media containing 2.0% glucose (unrestricted), or 0.5% or 0.1 % glucose (Glu). (B) Detection of Pnc1-GFP in cells from wild-type cultures grown under the following conditions: amino acid (a.a) restriction (SD lacking non-essential amino acids), salt stress (300 mM NaCl), heat stress (37°C). (C) Co-localisation of Pnc1-GFP (green) and RFP-PTS 1 (Peroxisomal Targeting Signal 1) (red) in cells from mid-log phase wild-type cultures. Yellow indicates overlap. Cultures were grown in complete media containing 0.5% glucose to facilitate visualization of fluorescence. (D) Localisation of Pnc1-GFP in cells from mid-log phase cultures of peroxisomal mutant strains, *pex6Δ*, *pex5Δ* and *pex7Δ.* Cultures were grown in complete media containing 0.5% glucose to enhance visualization of fluorescence. All images were taken with the same exposure of 1 s.

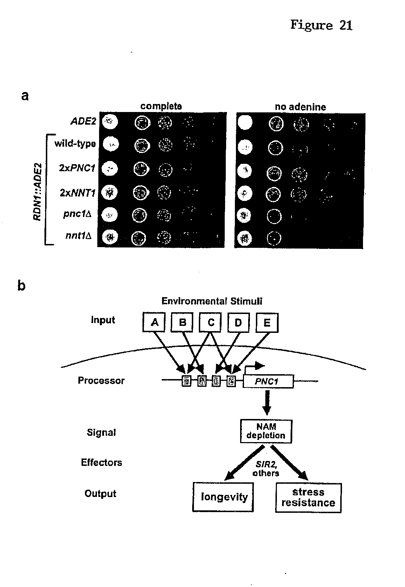

Fig. 21A-B. Manipulation of nicotinamide metabolism affects *SIR2* dependent silencing
(A) To measure silencing, an *ADE2* reporter was integrated at the ribosomal DNA (rDNA) locus. In this system, increased growth on media lacking adenine indicates decreased *ADE2* silencing. Strains were spotted in 10-fold serial dilutions on plates with or without adenine. An Ade⁺ strain served as a control. (B) Model for regulation of lifespan and stress resistance by nicotinamide. Disparate environmental stimuli including calorie restriction, heat and osmotic stress serve as inputs to a common pathway of longevity and stress resistance. Cells coordinate a response to these inputs by inducing transcription of *PNC1,* which encodes an enzyme that converts nicotinamide to nicotinic acid. In addition to alleviating inhibition of Sir2 and promoting longevity, depletion of nicotinamide activates a number of additional target proteins involved in stress resistance and possibly other cellular processes.

### Detailed description of the invention

The invention is based at least on the discovery that the life span of yeast cells can be extended by at least about 60% by increasing the flux through the nicotinamide adenine dinucleotide (NAD)+ salvage pathway (shown in Fig. 1). In addition, it was shown herein that this increase in flux through the NAD+ salvage pathway occurs essentially without increase in NAD+ and NADH levels and essentially by maintaining the ratio of NAD+/NADH constant. As shown in the Examples, increasing the flux through the NAD+ salvage pathway and thereby increasing the life span of cells can be achieved by introducing into the cells additional copies of a gene involved in the NAD+ salvage pathway, e.g., NPT1, PNC1, NMA1 and NMA2. It has also been shown in the Examples, that increasing the flux through the NAD+ salvage pathway protects yeast cells against certain types of stresses, e.g., heatshock. In addition, overexpression of PNC1 increases silencing, lifespan, as well as stress resistance, e.g., protects cells from DNA breakage caused by ultraviolet (U.V.) light and ethidium bromide and osmotic stress. On the other hand, deletion of PNC1 prevents lifespan extension and renders cells sensitive to stress.

The invention is also based at least on the discovery that nicotinamide inhibits silencing in yeast and thereby decreases the life span of cells. Nicotinamide was also shown to render cells more sensitive to stress. In particular, it was shown that overexpression of nicotinamide methyl transferase (NNMT), an enzyme that is involved in the secretion of nicotinamide from cells, stimulated silencing and thus extended life span, and increased tolerance to stress (e.g., radiation exposure), whereas the deletion of this enzyme had the opposite effect.

Based at least on the strong conservation of the NAD+ salvage pathway and silencing events from prokaryotes to eukaryotes, the methods of the invention are expected to be applicable to any eukaryotic cell, in addition to yeast cells, and to prokaryotic cells.

### 1. Definitions

As used herein, the following terms and phrases shall have the meanings set forth below. Unless defined otherwise, all technical and scientific terms used herein have the same meaning as commonly understood to one of ordinary skill in the art to which this invention belongs.

The singular forms "a," "an," and "the" include plural reference unless the context clearly dictates otherwise.

The term "isolated" as used herein with respect to nucleic acids, such as DNA or RNA, refers to molecules separated from other DNAs, or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

"Modulating the flux through the NAD+ salvage pathway of a cell" refers to an action resulting in increasing or decreasing the number of NAD+ molecules that are generated by the NAD+ salvage pathway, e.g. shown in Fig. 1.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids.

The phrase "nucleic acid corresponding to a gene" refers to a nucleic acid that can be used for detecting the gene, e.g., a nucleic acid which is capable of hybridizing specifically to the gene.

The term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. *See* Meth. Mol. Biol. 70: 173-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases. Databases with individual sequences are described in Methods in Enzymology, ed. Doolittle, *supra.* Databases include Genbank, EMBL, and DNA Database of Japan (DDBJ).

"Replicative life span" which is used interchangeably herein with "life span" or "lifespan" of a cell refers to the number of daughter cells produced by an individual "mother cell." "Chronological aging," on the other hand, refers to the length of time a population of non-dividing cells remains viable when deprived of nutrients. The life span of cells can be increased by at least about 20%, 30%, 40%, 50%, 60% or between 20% and 70%, 30% and 60%, 40 and 60% or more using the methods of the invention.

"Sir2 family members" or "Sir2 protein family members" refers to *S. cerevisiae* Sir2 protein as well as any histone deacetylases having substantial structural similarities to Sir2, e.g., the human homologs hSIRT1, gSIRT2, hSIRT3, hSIRT4, hSIRT5, hSIRT6 and hSIRT7; and Sir-2.1.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays described herein.

The term "specific hybridization" of a probe to a target site of a template nucleic acid refers to hybridization of the probe predominantly to the target, such that the hybridization signal can be clearly interpreted. As further described herein, such conditions resulting in specific hybridization vary depending on the length of the region of homology, the GC content of the region, the melting temperature "Tm" of the hybrid. Hybridization conditions will thus vary in the salt content, acidity, and temperature of the hybridization solution and the washes.

"Stress" refers to any non-optimal condition for growth, development or reproduction.

A "variant" of a polypeptide refers to a polypeptide having the amino acid sequence of the polypeptide in which is altered in one or more amino acid residues. The variant may have "conservative" changes, wherein a substituted amino acid has similar structural or chemical properties (e.g., replacement of leucine with isoleucine). A variant may have "nonconservative" changes (e.g., replacement of glycine with tryptophan). Analogous minor variations may also include amino acid deletions or insertions, or both. Guidance in determining which amino acid residues may be substituted, inserted, or deleted without abolishing biological or immunological activity may be found using computer programs well known in the art, for example, LASERGENE software (DNASTAR).

The term "variant," when used in the context of a polynucleotide sequence, may encompass a polynucleotide sequence related to that of a particular gene or the coding sequence thereof. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. The resulting polypeptides generally will have significant amino acid identity relative to each other. A polymorphic variantion is a variation in the polynucleotide sequence of a particular gene between individuals of a given species. Polymorphic variants also may encompass "single nucleotide polymorphisms" (SNPs) in which the polynucleotide sequence varies by one base. The presence of SNPs may be indicative of, for example, a certain population, a disease state, or a propensity for a disease state.

The term "aliphatic" is art-recognized and refers to a linear, branched, cyclic alkane, alkene, or alkyne. In certain embodiments, aliphatic groups in the present invention are linear or branched and have from 1 to about 20 carbon atoms.

The term "alkyl" is art-recognized, and includes saturated aliphatic groups, including straight-chain alkyl groups, branched-chain alkyl groups, cycloalkyl (alicyclic) groups, alkyl substituted cycloalkyl groups, and cycloalkyl substituted alkyl groups. In certain embodiments, a straight chain or branched chain alkyl has about 30 or fewer carbon atoms in its backbone (e.g., C₁-C₃₀ for straight chain, C₃-C₃₀ for branched chain), and alternatively, about 20 or fewer. Likewise, cycloalkyls have from about 3 to about 10 carbon atoms in their ring structure, and alternatively about 5, 6 or 7 carbons in the ring structure. The term "alkyl" is also defined to include halosubstituted alkyls.

The term "aralkyl" is art-recognized and refers to an alkyl group substituted with an aryl group (e.g., an aromatic or heteroaromatic group).

The terms "alkenyl" and "alkynyl" are art-recognized and refer to unsaturated aliphatic groups analogous in length and possible substitution to the alkyls described above, but that contain at least one double or triple bond respectively.

Unless the number of carbons is otherwise specified, "lower alkyl" refers to an alkyl group, as defined above, but having from one to about ten carbons, alternatively from one to about six carbon atoms in its backbone structure. Likewise, "lower alkenyl" and "lower alkynyl" have similar chain lengths.

The term "heteroatom" is art-recognized and refers to an atom of any element other than carbon or hydrogen. Illustrative heteroatoms include boron, nitrogen, oxygen, phosphorus, sulfur and selenium.

The term "aryl" is art-recognized and refers to 5-, 6- and 7-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics." The aromatic ring may be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings may be cycloalkyls, cycloalkenyls, cycloalkynyls, aryls and/or heterocyclyls.

The terms ortho, meta and para are art-recognized and refer to 1,2-, 1,3- and 1,4-disubstituted benzenes, respectively. For example, the names 1,2-dimethylbenzene and ortho-dimethylbenzene are synonymous.

The terms "heterocyclyl" or "heterocyclic group" are art-recognized and refer to 3-to about 10-membered ring structures, alternatively 3- to about 7-membered rings, whose ring structures include one to four heteroatoms. Heterocycles may also be polycycles. Heterocyclyl groups include, for example, thiophene, thianthrene, furan, pyran, isobenzofuran, chromene, xanthene, phenoxanthene, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring may be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The terms "polycyclyl" or "polycyclic group" are art-recognized and refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle may be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The term "carbocycle" is art-recognized and refers to an aromatic or non-aromatic ring in which each atom of the ring is carbon.

The term "nitro" is art-recognized and refers to -NO₂; the term "halogen" is art-recognized and refers to -F, -Cl, -Br or -I; the term "sulfhydryl" is art-recognized and refers to -SH; the term "hydroxyl" means -OH; and the term "sulfonyl" is art-recognized and refers to -SO₂⁻. "Halide" designates the corresponding anion of the halogens, and "pseudohalide" has the definition set forth on 560 of "Advanced Inorganic Chemistry" by Cotton and Wilkinson.

The terms "amine" and "amino" are art-recognized and refer to both unsubstituted and substituted amines, e.g., a moiety that may be represented by the general formulas: 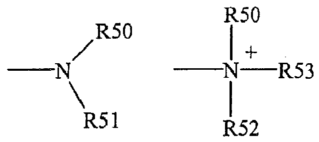 wherein R50, R51 and R52 each independently represent a hydrogen, an alkyl, an alkenyl, - (CH₂)ₘ-R61, or R50 and R51, taken together with the N atom to which they are attached complete a heterocycle having from 4 to 8 atoms in the ring structure; R61 represents an aryl, a cycloalkyl, a cycloalkenyl, a heterocycle or a polycycle; and m is zero or an integer in the range of 1 to 8. In certain embodiments, only one of R50 or R51 may be a carbonyl, e.g., R50, R51 and the nitrogen together do not form an imide. In other embodiments, R50 and R51 (and optionally R52) each independently represent a hydrogen, an alkyl, an alkenyl, or -(CH₂)ₘ-R61. Thus, the term "alkylamine" includes an amine group, as defined above, having a substituted or unsubstituted alkyl attached thereto, i.e., at least one of R50 and R51 is an alkyl group.

The term "acylamino" is art-recognized and refers to a moiety that may be represented by the general formula: 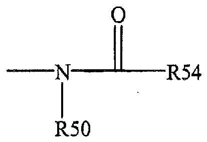 wherein R50 is as defined above, and R54 represents a hydrogen, an alkyl, an alkenyl or - (CH₂)ₘ-R61, where m and R61 are as defined above.

The term "amido" is art recognized as an amino-substituted carbonyl and includes a moiety that may be represented by the general formula: 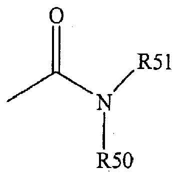 wherein R50 and R51 are as defined above. Certain embodiments of the amide in the present invention will not include imides which may be unstable,

The term "alkylthio" refers to an alkyl group, as defined above, having a sulfur radical attached thereto. In certain embodiments, the "alkylthio" moiety is represented by one of -S-alkyl, -S-alkenyl, -S-alkynyl, and -S-(CH₂)ₘ-R61, wherein m and R61 are defined above. Representative alkylthio groups include methylthio, ethyl thio, and the like.

The term "carbonyl" is art recognized and includes such moieties as may be represented by the general formulas: 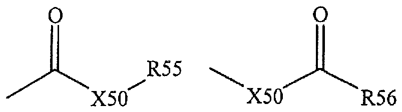 wherein X50 is a bond or represents an oxygen or a sulfur, and R55 and R56 represents a hydrogen, an alkyl, an alkenyl, -(CH₂)ₘ-R61or a pharmaceutically acceptable salt, R56 represents a hydrogen, an alkyl, an alkenyl or -(CH₂)ₘ-R61, where m and R61 are defined above. Where X50 is an oxygen and R55 or R56 is not hydrogen, the formula represents an "ester". Where X50 is an oxygen, and R55 is as defined above, the moiety is referred to herein as a carboxyl group, and particularly when R55 is a hydrogen, the formula represents a "carboxylic acid". Where X50 is an oxygen, and R56 is hydrogen, the formula represents a "formate". In general, where the oxygen atom of the above formula is replaced by sulfur, the formula represents a "thiolcarbonyl" group. Where X50 is a sulfur and R55 or R56 is not hydrogen, the formula represents a "thiolester." Where X50 is a sulfur and R55 is hydrogen, the formula represents a "thiolcarboxylic acid." Where X50 is a sulfur and R56 is hydrogen, the formula represents a "thiolformate." On the other hand, where X50 is a bond, and R55 is not hydrogen, the above formula represents a "ketone" group. Where X50 is a bond, and R55 is hydrogen, the above formula represents an "aldehyde" group.

The terms "alkoxyl" or "alkoxy" are art-recognized and refer to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as may be represented by one of -O-alkyl, -O-alkenyl, -O-alkynyl, -O--(CH₂)ₘ-R61, where m and R61 are described above.

The definition of each expression, e.g. alkyl, m, n, and the like, when it occurs more than once in any structure, is intended to be independent of its definition elsewhere in the same structure.

The terms triflyl, tosyl, mesyl, and nonaflyl are art-recognized and refer to trifluoromethanesulfonyl, *p*-toluenesulfonyl, methanesulfonyl, and nonafluorobutanesulfonyl groups, respectively. The terms triflate, tosylate, mesylate, and nonaflate are art-recognized and refer to trifluoromethanesulfonate ester, *p*-toluenesulfonate ester, methanesulfonate ester, and nonafluorobutanesulfonate ester functional groups and molecules that contain said groups, respectively.

The abbreviations Me, Et, Ph, Tf, Nf, Ts, and Ms represent methyl, ethyl, phenyl, trifluoromethanesulfonyl, nonafluorobutanesulfonyl, *p*-toluenesuIfonyl and methanesulfonyl, respectively. A more comprehensive list of the abbreviations utilized by organic chemists of ordinary skill in the art appears in the first issue of each volume of the Journal of Organic Chemistry; this list is typically presented in a table entitled Standard List of Abbreviations.

Certain compounds contained in compositions of the present invention may exist in particular geometric or stereoisomeric forms. In addition, polymers of the present invention may also be optically active. The present invention contemplates all such compounds, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

For purposes of this invention, the chemical elements are identified in accordance with the Periodic Table of the Elements, CAS version, Handbook of Chemistry and Physics, 67th Ed., 1986-87, inside cover. Also for purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds that may be substituted or unsubstituted.

The definition of each expression, e.g. lower alkyl, m, n, p and the like, when it occurs more than once in any structure, is intended to be independent of its definition elsewhere in the same structure.

The term "pharmaceutically-acceptable salts" is art-recognized and refers to the relatively non-toxic, inorganic and organic acid addition salts of compounds, including, for example, those contained in compositions of the present invention.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be "acceptable" in the sense of being compatible with the subject composition and its components and not injurious to the patient. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The terms "systemic administration," "administered systemically," "peripheral administration" and "administered peripherally" are art-recognized and refer to the administration of a subject composition, therapeutic or other material other than directly into the central nervous system, such that it enters the patient's system and, thus, is subject to metabolism and other like processes, for example, subcutaneous administration.

The terms "parenteral administration" and "administered parenterally" are art-recognized and refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articulare, subcapsular, subarachnoid, intraspinal, and intrastemal injection and infusion.

### 2. Methods for increasing the life span of a cell or protecting it against certain stresses

In one embodiment, the life span of a cell is increased and/or the cell is protected against certain stresses by increasing the flux through the NAD+ salvage pathway. This can be achieved, e.g., increasing the level or activity of at least one protein involved in the NAD+ salvage pathway, such as a protein selected from the group consisting of NPT1, PNC1, NMA1 and NMA2.

The level of protein can be increased in a cell, e.g., by introducing into the cell a nucleic acid encoding the protein operably linked to a transcriptional regulatory sequence directing the expression of the protein in the cell. Methods for expressing nucleic acids in cells and appropriate transcriptional regulatory elements for doing so are well known in the art. Alternatively, a protein can be introduced into a cell, usually in the presence of a vector facilitating the entry of the protein into the cells, e.g., liposomes. Proteins can also be linked to transcytosis peptides for that purpose. Yet in other methods, an agent that stimulates expression of the endogenous gene is contacted with a cell. Such agents can be identified as further described herein.

A nucleotide sequence encoding *S. cerevisiae* nicotinate phosphoribosyltransferase (NPT1) and the protein encoded thereby are set forth as SEQ ID Nos: 1 and 2, respectively. NPT1 is also known as "LSR2." The *S. cerevisiae* NPT1 complete cDNA and encoded protein are provided by GenBank Accession numbers NC_001147 and AAB59317, respectively, which are set forth as SEQ ID NOs: 1 and 2, respectively. Accession numbers L11274 and AAB59317 also appear to refer to *S. cerevisiae* nucleotide and amino acid sequences, respectively. The NPT1 homolog in bacteria is PncB (35, 37 and 38). The *E. col*i NPT1 is provided as GenBank accession number J05568. The human nucleotide and amino acid sequences are provided by GenBank Accession numbers BC006284 and AAH06284, respectively, and X71355 and CAA50490, respectively, AAH32466 and BC032466 and are described in Chong et al. (1993) Genomics 18:355. The human nucleotide and amino acid sequences are also set forth as SEQ ID NOs: 13 and 14, respectively (and correspond to GenBank Accession No. BC032466). The human protein is also referred to as a "renal sodium phosphate transport protein." A mouse NPT1 nucleotide and amino acid sequences are provided by GenBank Accession numbers X77241 and CAA54459 and are described in Chong et al. (1995) Am. J. Physiol. 268:1038. The promoter region of mouse NPT1 is provided as GenBank Accession number AF361762 and is described in Soumounou et al. (2001) Am J. Physiol. 281: F1082. NPT1 is also set forth as an IMAGE Clone, under number 3957135. An alignment of NPT1 homologs is set forth in Fig. 15.

A nucleotide sequence encoding *S. cerevisiae* PNC1 and the protein encoded thereby are set forth as SEQ ID Nos: 3 and 4, respectively, which correspond to GenBank Accession numbers NC_001139 and NP_011478, respectively. PNC1 is the yeast homologue of the bacterial protein pncA, which catalyzes the hydrolysis of nicotinamide to nicotinic acid and ammonia. *S. cerevisiae* PNC1, also referred to as open reading frame (ORF) YGL037 is described in Ghislain et al. (2002) Yeast 19:215. The nucleotide and amino acid sequences of an Arachis hypogaea PNC1 is provided by GenBank Accession numbers M37636 and AAB06183 and are described in Buffard et al. (1990) PNAS 87:8874. Nucleotide and amino acid sequences of a human homolog are provided by GenBank Accession numbers BC017344 and AAH17344, respectively; AK027122 and NP_078986, respectively; XM_041059 and XP_041059, respectively; and NM_016048 and NP_057132, respectively. The nucleotide and amino acid sequences of human PNC1 are set forth as SEQ ID NOs: 15 and 16, respectively and correspond to GenBank Accession No. BC017344. An alignment of human, fly and *S. cerevisiae* PNC1 is set forth in Fig. 16.

A nucleotide sequence encoding *S. cerevisae* NMA1 and the protein encoded thereby are set forth as SEQ ID Nos: 5 and 6, respectively, which correspond to GenBank Accession Numbers NC_001144.2 and NP_013432, respectively. The *S. cerevisiae* NMA1 corresponds to ORF YLR328, described in Smith et al. (2000) PNAS 97:6658. NMA1 is the *S. cerevisae* homolog of the bacterial NaMNAT gene. Nucleotide and amino acid sequences of human homologs are provided by GenBank Accession numbers NM_022787 and NP_073624, respectively; AK026065 and BAB15345, respectively; AF459819 and AAL76934, respectively; XM_087387 and XP_087387, respectively; and AF345564 and AAK52726, respectively, and NP_073624; AAL76934; NP_073624; and AF314163. The nucleotide and amino acid sequence of human NMA1 is set forth as SEQ ID NOs: 17 and 18, respectively, and correspond to GenBank Accession number NM_022787. An IMAGE Clone is provided under number 4874147 and HRC clone hrc08458. Bacterial homologs are described, e.g., in Zhang et al. (2002) Structure 10:69.

A nucleotide sequence encoding *S. cerevisiae* NMA2 and the protein encoded thereby are set forth as SEQ ID Nos: 7 and 8, respectively, which correspond to GenBank Accession numbers NC_001139 and NP_011524, respectively. The *S. cerevisiiae* NMA2 corresponds to ORF YGR010, described in Emanuelli et al. (1999) FEBS Lett. 455;13. NMA2 is the *S. cerevisiae* homolog of the bacterial NaMNAT gene. Nucleotide and amino acid sequences of human homologs are provided by GenBank Accession numbers NM_015039 and NP_055854, respectively. The nucleotide and amino acid sequences of human NMA2 are set forth as SEQ ID NOs: 19 and 20, respectively, and correspond to GenBank Accession number NM_015039.

It will be apparent to a person of skill in the art that a full length protein or nucleic acid encoding such or a portion thereof can be used according to the methods described herein. A portion of a protein is preferably a biologically active portion thereof. Portions that are biologically active can be identified according to methods known in the art and using an assay that can monitor the activity of the particular protein. Assays for determining the activity of an NPT1 protein are described, e.g., in Pescanglini et al. (1994) Clin. Chim. Acta 229: 15-25 and Sestini et al. (2000) Archives of Biochem. Biophys. 379:277. Assays for determining the activity of a PNC1 protein are described, e.g., in Ghislain et al. Yeast 19:215. Assays for determining the activity of an NMA1 and NMA2 protein are described, e.g., in Sestini et al., *supra.* Alternatively, the activity of such a protein can be tested in an assay in which the life span of a cell is determined. For example, a cell is transfected with a nucleic acid comprising one or more copies of a sequence encoding a portion of an NPT1, PNC1, NMA1 or NMA2 protein or a control nucleic acid, and the life span of the cells is compared. A longer life span of a cell transfected with a portion of one of the proteins indicates that the portion of the protein is a biologically active portion. Assays for determining the life span of a cell are known in the art and are also further described herein. In particular, assays for determining the life span of a mammalian cell can be conducted as described, e.g., in Cell Growth, Differentiation and Senescence: A Practical Approach. George P. Studzinski (ed.). Instead of measuring the life span, one can also measure the resistance of a transfected cell to certain stresses, e.g., heatshock, for determining whether a portion of a protein is a biologically active portion. Methods for measuring resistance to certain stresses are known in the art and are also further described herein. In particular, assays for determining the resistance of a mammalian cell to heatshock can be conducted as described, e.g., in Bunelli et al. (1999) Exp. Cell Res. 262: 20.

In addition to portions of NPT1, PNC1, NMA1 or NMA2 proteins, other variants, such as proteins containing a deletion, insertion or addition of one or more amino acids can be used, provided that the protein is biologically active. Exemplary amino acid changes include conservative amino acid substitutions. Other changes include substitutions for non-naturally occurring amino acids. Proteins encoded by nucleic acids that hybridize to a nucleic acid encoding NPT1, PNC1, NMA1 or NMA2 under high or medium stringency conditions and which are biologically active can also be used. For example, nucleic acids that hybridize under high stringency conditions of 0.2 to 1 x SSC at 65 °C followed by a wash at 0.2 x SSC at 65 °C to a gene encoding NPT1, PNC1, NMA1 or NMA2 can be used. Nucleic acids that hybridize under low stringency conditions of 6 x SSC at room temperature followed by a wash at 2 x SSC at room temperature to a gene encoding NPT1, PNC1, NMA1 or NMA2 can be used. Other Other hybridization conditions include 3 x SSC at 40 or 50 °C, followed by a wash in 1 or 2 x SSC at 20, 30, 40, 50, 60, or 65°C. Hybridizations can be conducted in the presence of formaldehyde, e.g., 10%, 20%, 30% 40% or 50%, which further increases the stringency of hybridization. Theory and practice of nucleic acid hybridization is described, e.g., in S. Agrawal (ed.) Methods in Molecular Biology, volume 20; and Tijssen (1993) Laboratory Techniques in biochemistry and molecular biology-hybridization with nucleic acid probes, e.g., part I chapter 2 "Overview of principles of hybridization and the strategy of nucleic acid probe assays," Elsevier, New York provide a basic guide to nucleic acid hybridization.

Exemplary proteins may have at least about 50%, 70%, 80%, 90%, preferably at least about 95%, even more preferably at least about 98% and most preferably at least 99% homology or identity with a wild-type NPT1, PNC1, NMA1 or NMA2 protein or a domain thereof, e.g., the catalytic domain. Other exemplary proteins may be encoded by a nucleic acid that is at least about 90%, preferably at least about 95%, even more preferably at least about 98% and most preferably at least 99% homology or identity with a wild-type NPT1, PNC1, NMA1 or NMA2 nucleic acid, e.g., those described herein.

In other embodiments proteins are fusion proteins, e.g., proteins fused to a transcytosis peptide. Fusion proteins may also comprise a heterologous peptide that can be used to purify the protein and/or to detect it.

In other embodiments, non-naturally occurring protein variants are used. Such variants can be peptidomimetics.

In yet other embodiments, the activity of one or more proteins selected from the group consisting of NPT1, PNC1, NMA1 and NMA2 is enhanced or increased. This can be achieved, e.g., by contacting a cell with a compound that increases the activity, e.g., enzymatic activity, of one of these proteins. Assays for identifying such compounds are further described herein.

In preferred embodiments, the flux through the NAD+ salvage pathway is increased without substantially changing the level of NAD+, NADH and the ratio of NAD+/NADH in a cell. Levels of NAD+ and NADH and ratios of these two molecules can be determined, e.g., as described in the Examples.

Any means for the introduction of polynucleotides into mammals, human or non-human, or cells thereof may be adapted to the practice of this invention for the delivery of the various constructs of the invention into the intended recipient. In one embodiment of the invention, the DNA constructs are delivered to cells by transfection, i.e., by delivery of "naked" DNA or in a complex with a colloidal dispersion system. A colloidal system includes macromolecule complexes, nanocapsules, microspheres, beads, and lipid-based systems including oil-in-water emulsions, micelles, mixed micelles, and liposomes. The preferred colloidal system of this invention is a lipid-complexed or liposome-formulated DNA. In the former approach, prior to formulation of DNA, e.g., with lipid, a plasmid containing a transgene bearing the desired DNA constructs may first be experimentally optimized for expression (e.g., inclusion of an intron in the 5' untranslated region and elimination of unnecessary sequences (Felgner, et al., Ann NY Acad Sci 126-139, 1995). Formulation of DNA, e.g. with various lipid or liposome materials, may then be effected using known methods and materials and delivered to the recipient mammal. See, e.g., Canonico et al, Am J Respir Cell Mol Biol 10:24-29, 1994; Tsan et al, Am J Physiol 268; Alton et al., Nat Genet. 5:135-142, 1993 and U.S. patent No. 5,679,647 by Carson et al.

The targeting of liposomes can be classified based on anatomical and mechanistic factors. Anatomical classification is based on the level of selectivity, for example, organ-specific, cell-specific, and organelle-specific. Mechanistic targeting can be distinguished based upon whether it is passive or active. Passive targeting utilizes the natural tendency of liposomes to distribute to cells of the reticulo-endothelial system (RES) in organs, which contain sinusoidal capillaries. Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types other than the naturally occurring sites of localization.

The surface of the targeted delivery system may be modified in a variety of ways. In the case of a liposomal targeted delivery system, lipid groups can be incorporated into the lipid bilayer of the liposome in order to maintain the targeting ligand in stable association with the liposomal bilayer. Various linking groups can be used for joining the lipid chains to the targeting ligand. Naked DNA or DNA associated with a delivery vehicle, e.g., liposomes, can be administered to several sites in a subject (see below).

In a preferred method of the invention, the DNA constructs are delivered using viral vectors. The transgene may be incorporated into any of a variety of viral vectors useful in gene therapy, such as recombinant retroviruses, adenovirus, adeno-associated virus (AAV), and herpes simplex virus-1, or recombinant bacterial or eukaryotic plasmids. While various viral vectors may be used in the practice of this invention, AAV- and adenovirus-based approaches are of particular interest. Such vectors are generally understood to be the recombinant gene delivery system of choice for the transfer of exogenous genes *in vivo*, particularly into humans. The following additional guidance on the choice and use of viral vectors may be helpful to the practitioner. As described in greater detail below, such embodiments of the subject expression constructs are specifically contemplated for use in various *in vivo* and *ex vivo* gene therapy protocols.

A viral gene delivery system useful in the present invention utilizes adenovirus-derived vectors. Knowledge of the genetic organization of adenovirus, a 36 kB, linear and double-stranded DNA virus, allows substitution of a large piece of adenoviral DNA with foreign sequences up to 8 kB. In contrast to retrovirus, the infection of adenoviral DNA into host cells does not result in chromosomal integration because adenoviral DNA can replicate in an episomal manner without potential genotoxicity. Also, adenoviruses are structurally stable, and no genome rearrangement has been detected after extensive amplification. Adenovirus can infect virtually all epithelial cells regardless of their cell cycle stage. So far, adenoviral infection appears to be linked only to mild disease such as acute respiratory disease in the human.

Adenovirus is particularly suitable for use as a gene transfer vector because of its mid-sized genome, ease of manipulation, high titer, wide target-cell range, and high infectivity. The virus particle is relatively stable and amenable to purification and concentration, and as above, can be modified so as to affect the spectrum of infectivity. Additionally, adenovirus is easy to grow and manipulate and exhibits broad host range *in vitro* and *in vivo.* This group of viruses can be obtained in high titers, e.g., 10⁹ -10¹¹ plaque-forming unit (PFU)/ml, and they are highly infective. The life cycle of adenovirus does not require integration into the host cell genome. The foreign genes delivered by adenovirus vectors are episomal, and therefore, have low genotoxicity to host cells. No side effects have been reported in studies of vaccination with wild-type adenovirus (Couch et al., 1963; Top et al., 1971), demonstrating their safety and therapeutic potential as *in vivo* gene transfer vectors. Moreover, the carrying capacity of the adenoviral genome for foreign DNA is large (up to 8 kilobases) relative to other gene delivery vectors (Berkner et al., supra; Haj-Ahmand and Graham (1986) J. Virol. 57:267). Most replication-defective adenoviral vectors currently in use and therefore favored by the present invention are deleted for all or parts of the viral E1 and E3 genes but retain as much as 80% of the adenoviral genetic material (see, e.g., Jones et al., (1979) Cell 16:683; Berkner et al., supra; and Graham et al., in Methods in Molecular Biology, E.J. Murray, Ed. (Humana, Clifton, NJ, 1991) vol. 7. pp. 109-127). Expression of the inserted polynucleotide of the invention can be under control of, for example, the E1A promoter, the major late promoter (MLP) and associated leader sequences, the viral E3 promoter, or exogenously added promoter sequences.

The genome of an adenovirus can be manipulated such that it encodes a gene product of interest, but is inactivated in terms of its ability to replicate in a normal lytic viral life cycle (see, for example, Berkner et al., (1988) BioTechniques 6:616; Rosenfeld et al., (1991) Science 252:431-434; and Rosenfeld et al., (1992) Cell 68:143-155). Suitable adenoviral vectors derived from the adenovirus strain Ad type 5 d1324 or other strains of adenovirus (e.g., Ad2, Ad3, Ad7 etc.) are well known to those skilled in the art. Recombinant adenoviruses can be advantageous in certain circumstances in that they are not capable of infecting nondividing cells and can be used to infect a wide variety of cell types, including airway epithelium (Rosenfeld et al., (1992) cited supra), endothelial cells (Lemarchand et al., (1992) PNAS USA 89:6482-6486), hepatocytes (Herz and Gerard, (1993) PNAS USA 90:2812-2816) and muscle cells (Quantin et al., (1992) PNAS USA 89:2581-2584).

Adenoviruses can also be cell type specific, i.e., infect only restricted types of cells and/or express a transgene only in restricted types of cells. For example, the viruses comprise a gene under the transcriptional control of a transcription initiation region specifically regulated by target host cells, as described e.g., in U.S. Patent No. 5,698,443, by Henderson and Schuur, issued December 16, 1997. Thus, replication competent adenoviruses can be restricted to certain cells by, e.g., inserting a cell specific response element to regulate a synthesis of a protein necessary for replication, e.g., E1A or E1B.

DNA sequences of a number of adenovirus types are available from Genbank. For example, human adenovirus type 5 has GenBank Accession No.M73260. The adenovirus DNA sequences may be obtained from any of the 42 human adenovirus types currently identified. Various adenovirus strains are available from the American Type Culture Collection, Rockville, Maryland, or by request from a number of commercial and academic sources. A transgene as described herein may be incorporated into any adenoviral vector and delivery protocol, by restriction digest, linker ligation or filling in of ends, and ligation.

Adenovirus producer cell lines can include one or more of the adenoviral genes E1, E2a, and E4 DNA sequence, for packaging adenovirus vectors in which one or more of these genes have been mutated or deleted are described, e.g., in PCT/US95/15947 (WO 96/18418) by Kadan et al.; PCT/US95/07341 (WO 95/346671) by Kovesdi et al.; PCT/FR94/00624 (WO94/28152) by Imler et al.;PCT/FR94/00851 (WO 95/02697) by Perrocaudet et al., PCT/US95/14793 (WO96/14061) by Wang et al.

Yet another viral vector system useful for delivery of the subject polynucleotides is the adeno-associated virus (AAV). Adeno-associated virus is a naturally occurring defective virus that requires another virus, such as an adenovirus or a herpes virus, as a helper virus for efficient replication and a productive life cycle. (For a review, see Muzyczka et al., Curr. Topics in Micro. and Immunol. (1992) 158:97-129).

AAV has not been associated with the cause of any disease. AAV is not a transforming or oncogenic virus. AAV integration into chromosomes of human cell lines does not cause any significant alteration in the growth properties or morphological characteristics of the cells. These properties of AAV also recommend it as a potentially useful human gene therapy vector.

AAV is also one of the few viruses that may integrate its DNA into non-dividing cells, e.g., pulmonary epithelial cells, and exhibits a high frequency of stable integration (see for example Flotte et al., (1992) Am. J. Respir. Cell. Mol. Biol. 7:349-356; Samulski et al., (1989) J. Virol. 63:3822-3828; and McLaughlin et al., (1989) J. Virol. 62:1963-1973). Vectors containing as little as 300 base pairs of AAV can be packaged and can integrate. Space for exogenous DNA is limited to about 4.5 kb. An AAV vector such as that described in Tratschin et al., (1985) Mol. Cell. Biol. 5:3251-3260 can be used to introduce DNA into cells. A variety of nucleic acids have been introduced into different cell types using AAV vectors (see for example Hermonat et al., (1984) PNAS USA 81:6466-6470; Tratschin et al., (1985) Mol. Cell. Biol. 4:2072-2081; Wondisford et al., (1988) Mol. Endocrinol. 2:32-39; Tratschin et al., (1984) J. Virol. 51:611-619; and Flotte et al., (1993) J. Biol. Chem. 268:3781-3790).

The AAV-based expression vector to be used typically includes the 145 nucleotide AAV inverted terminal repeats (ITRs) flanking a restriction site that can be used for subcloning of the transgene, either directly using the restriction site available, or by excision of the transgene with restriction enzymes followed by blunting of the ends, ligation of appropriate DNA linkers, restriction digestion, and ligation into the site between the ITRs. The capacity of AAV vectors is about 4.4 kb (Kotin, R.M., Human Gene Therapy 5:793-801, 1994 and Flotte, et al. J. Biol.Chem. 268:3781-3790, 1993).

AAV stocks can be produced as described in Hermonat and Muzyczka (1984) PNAS 81:6466, modified by using the pAAV/Ad described by Samulski et al. (1989) J. Virol. 63:3822. Concentration and purification of the virus can be achieved by reported methods such as banding in cesium chloride gradients, as was used for the initial report of AAV vector expression *in vivo* (Flotte, et al. J.Biol. Chem. 268:3781-3790, 1993) or chromatographic purification, as described in O'Riordan et al., WO97/08298. Methods for *in vitro* packaging AAV vectors are also available and have the advantage that there is no size limitation of the DNA packaged into the particles (see, U.S. Patent No. 5,688,676, by Zhou et al., issued Nov. 18, 1997). This procedure involves the preparation of cell free packaging extracts.

Hybrid Adenovirus-AAV vectors represented by an adenovirus capsid containing a nucleic acid comprising a portion of an adenovirus, and 5' and 3' ITR sequences from an AAV which flank a selected transgene under the control of a promoter. See e.g. Wilson et al, International Patent Application Publication No. WO 96/13598. This hybrid vector is characterized by high titer transgene delivery to a host cell and the ability to stably integrate the transgene into the host cell chromosome in the presence of the rep gene. This virus is capable of infecting virtually all cell types (conferred by its adenovirus sequences) and stable long term transgene integration into the host cell genome (conferred by its AAV sequences).

The adenovirus nucleic acid sequences employed in this vector can range from a minimum sequence amount, which requires the use of a helper virus to produce the hybrid virus particle, to only selected deletions of adenovirus genes, which deleted gene products can be supplied in the hybrid viral process by a packaging cell. For example, a hybrid virus can comprise the 5' and 3' inverted terminal repeat (ITR) sequences of an adenovirus (which function as origins of replication). The left terminal sequence (5') sequence of the Ad5 genome that can be used spans bp 1 to about 360 of the conventional adenovirus genome (also referred to as map units 0-1) and includes the 5' ITR and the packaging/enhancer domain. The 3' adenovirus sequences of the hybrid virus include the right terminal 3' ITR sequence which is about 580 nucleotides (about bp 35,353- end of the adenovirus, referred to as about map units 98.4-100).

The preparation of the hybrid vector is further described in detail in published PCT application entitled "Hybrid Adenovirus-AAV Virus and Method of Use Thereof", WO 96/13598 by Wilson et al. For additional detailed guidance on adenovirus and hybrid adenovirus-AAV technology which may be useful in the practice of the subject invention, including methods and materials for the incorporation of a transgene, the propagation and purification of recombinant virus containing the transgene, and its use in transfecting cells and mammals, see also Wilson et al, WO 94/28938, WO 96/13597 and WO 96/26285, and references cited therein.

In order to construct a retroviral vector, a nucleic acid of interest is inserted into the viral genome in the place of certain viral sequences to produce a virus that is replication-defective. In order to produce virions, a packaging cell line containing the gag, pol, and env genes but without the LTR and psi components is constructed (Mann et al. (1983) Cell 33:153). When a recombinant plasmid containing a human cDNA, together with the retroviral LTR and psi sequences is introduced into this cell line (by calcium phosphate precipitation for example), the psi sequence allows the RNA transcript of the recombinant plasmid to be packaged into viral particles, which are then secreted into the culture media (Nicolas and Rubenstein (1988) "Retroviral Vectors", In: Rodriguez and Denhardt ed. Vectors: A Survey of Molecular Cloning Vectors and their Uses. Stoneham:Butterworth; Temin, (1986) "Retrovirus Vectors for Gene Transfer: Efficient Integration into and Expression of Exogenous DNA in Vertebrate Cell Genome", In: Kucherlapati ed. Gene Transfer. New York: Plenum Press; Mann et al., 1983, supra). The media containing the recombinant retroviruses is then collected, optionally concentrated, and used for gene transfer. Retroviral vectors are able to infect a broad variety of cell types. Integration and stable expression require the division of host cells (Paskind et al. (1975) Virology 67:242). This aspect is particularly relevant for the treatment of PVR, since these vectors allow selective targeting of cells which proliferate, i.e., selective targeting of the cells in the epiretinal membrane, since these are the only ones proliferating in eyes of PVR subjects.

A major prerequisite for the use of retroviruses is to ensure the safety of their use, particularly with regard to the possibility of the spread of wild-type virus in the cell population. The development of specialized cell lines (termed "packaging cells") which produce only replication-defective retroviruses has increased the utility of retroviruses for gene therapy, and defective retroviruses are well characterized for use in gene transfer for gene therapy purposes (for a review see Miller, A.D. (1990) Blood 76:271). Thus, recombinant retrovirus can be constructed in which part of the retroviral coding sequence (gag, pol, env) has been replaced by nucleic acid encoding a protein of the present invention, e.g., a transcriptional activator, rendering the retrovirus replication defective. The replication defective retrovirus is then packaged into virions which can be used to infect a target cell through the use of a helper virus by standard techniques. Protocols for producing recombinant retroviruses and for infecting cells *in vitro* or *in vivo* with such viruses can be found in Current Protocols in Molecular Biology, Ausubel, F.M. et al., (eds.) Greene Publishing Associates, (1989), Sections 9.10-9.14 and other standard laboratory manuals. Examples of suitable retroviruses include pLJ, pZIP, pWE and pEM which are well known to those skilled in the art. A preferred retroviral vector is a pSR MSVtkNeo (Muller et al. (1991) Mol. Cell Biol. 11:1785 and pSR MSV(XbaI) (Sawyers et al. (1995) J. Exp. Med. 181:307) and derivatives thereof. For example, the unique BamHI sites in both of these vectors can be removed by digesting the vectors with BamHI, filling in with Klenow and religating to produce pSMTN2 and pSMTX2, respectively, as described in PCT/US96/09948 by Clackson et al. Examples of suitable packaging virus lines for preparing both ecotropic and amphotropic retroviral systems include Crip, Cre, 2 and Am.

Retroviruses, including lentiviruses, have been used to introduce a variety of genes into many different cell types, including neural cells, epithelial cells, retinal cells, endothelial cells, lymphocytes, myoblasts, hepatocytes, bone marrow cells, *in vitro* and/or *in vivo* (see for example, review by Federico (1999) Curr. Opin. Biotechnol. 10:448; Eglitis et al., (1985) Science 230:1395-1398; Danos and Mulligan, (1988) PNAS USA 85:6460-6464; Wilson et al., (1988) PNAS USA 85:3014-3018; Armentano et al., (1990) PNAS USA 87:6141-6145; Huber et al., (1991) PNAS USA 88:8039-8043; Ferry et al., (1991) PNAS USA 88:8377-8381; Chowdhury et al., (1991) Science 254:1802-1805; van Beusechem et al., (1992) PNAS USA 89:7640-7644; Kay et al., (1992) Human Gene Therapy 3:641-647; Dai et al., (1992) PNAS USA 89:10892-10895; Hwu et al., (1993) J. Immunol. 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573).

Furthermore, it has been shown that it is possible to limit the infection spectrum of retroviruses and consequently of retroviral-based vectors, by modifying the viral packaging proteins on the surface of the viral particle (see, for example PCT publications WO93/25234, WO94/06920, and WO94/11524) For instance, strategies for the modification of the infection spectrum ofretroviral vectors include: coupling antibodies specific for cell surface antigens to the viral env protein (Roux et al., (1989) PNAS USA 86:9079-9083; Julan et al., (1992) J. Gen Virol 73:3251-3255; and Goud et al., (1983) Virology 163:251-254); or coupling cell surface ligands to the viral env proteins (Neda et al., (1991) J. Biol. Chem. 266:14143-14146). Coupling can be in the form of the chemical cross-linking with a protein or other variety (e.g. lactose to convert the env protein to an asialoglycoprotein), as well as by generating fusion proteins (e.g. single-chain anfibody/env fusion proteins). This technique, while useful to limit or otherwise direct the infection to certain tissue types, and can also be used to convert an ecotropic vector in to an amphotropic vector.

Other viral vector systems that can be used to deliver a polynucleotide of the invention have been derived from herpes virus, e.g., Herpes Simplex Virus (U.S. Patent No. 5,631,236 by Woo et al., issued May 20, 1997 and WO 00/08191 by Neurovex), vaccinia virus (Ridgeway (1988) Ridgeway, "Mammalian expression vectors," In: Rodriguez R L, Denhardt D T, ed. Vectors: A survey of molecular cloning vectors and their uses. Stoneham: Butterworth,; Baichwal and Sugden (1986) "Vectors for gene transfer derived from animal DNA viruses: Transient and stable expression of transferred genes," In: Kucherlapati R, ed. Gene transfer. New York: Plenum Press; Coupar et al. (1988) Gene, 68:1-10), and several RNA viruses. Preferred viruses include an alphavirus, a poxivirus, an arena virus, a vaccinia virus, a polio virus, and the like. They offer several attractive features for various mammalian cells (Friedmann (1989) Science, 244:1275-1281 ; Ridgeway, 1988, supra; Baichwal and Sugden, 1986, supra; Coupar et al., 1988; Horwich et al.(1990) J.Virol., 64:642-650).

The expression of a protein, e.g., a protein selected from the group consisting of NPT1, PNC1, NMA1 and NMA2 or a biologically active variant thereof in cells of a subject to whom, e.g., a nucleic acid encoding the protein was administered, can be determined, e.g., by obtaining a sample of the cells of the patient and determining the level of the protein in the sample, relative to a control sample.

In another embodiment, a protein or biologically active variant thereof, is administered to the subject such that it reaches the target cells, and traverses the cellular membrane. Polypeptides can be synthesized in prokaryotes or eukaryotes or cells thereof and purified according to methods known in the art. For example, recombinant polypeptides can be synthesized in human cells, mouse cells, rat cells, insect cells, yeast cells, and plant cells. Polypeptides can also be synthesized in cell free extracts, e.g., reticulocyte lysates or wheat germ extracts. Purification of proteins can be done by various methods, e.g., chromatographic methods *(see, e.g.,* Robert K Scopes "Protein Purification: Principles and Practice" Third Ed. Springer-Verlag, N.Y. 1994). In one embodiment, the polypeptide is produced as a fusion polypeptide comprising an epitope tag consisting of about six consecutive histidine residues. The fusion polypeptide can then be purified on a Ni⁺⁺ column. By inserting a protease site between the tag and the polypeptide, the tag can be removed after purification of the peptide on the Ni⁺⁺ column. These methods are well known in the art and commercial vectors and affinity matrices are commercially available.

Administration of polypeptides can be done by mixing them with liposomes, as described above. The surface of the liposomes can be modified by adding molecules that will target the liposome to the desired physiological location.

In one embodiment, a protein is modified so that its rate of traversing the cellular membrane is increased. For example, the polypeptide can be fused to a second peptide which promotes "transcytosis," e.g., uptake of the peptide by cells. In one embodiment, the peptide is a portion of the HIV transactivator (TAT) protein, such as the fragment corresponding to residues 37 -62 or 48-60 of TAT, portions which are rapidly taken up by cell *in vitro* (Green and Loewenstein, (1989) Cell 55:1179-1188). In another embodiment, the internalizing peptide is derived from the Drosophila antennapedia protein, or homologs thereof. The 60 amino acid long homeodomain of the homeo-protein antennapedia has been demonstrated to translocate through biological membranes and can facilitate the translocation of heterologous polypeptides to which it is couples. Thus, polypeptides can be fused to a peptide consisting of about amino acids 42-58 of Drosophila antennapedia or shorter fragments for transcytosis. See for example Derossi et al. (1996) J Biol Chem 271:18188-18193; Derossi et al. (1994) J Biol Chem 269:10444-10450; and Perez et al. (1992) J Cell Sci 102:717-722.

In another embodiment, the amount of nicotinamide is decreased in a cell. This can be achieved, e.g., by inhibiting the expression of genes of the NAD+ salvage pathway or other pathway that produce nicotinamide. Inhibition of the genes can be conducted, e.g., as further described herein, such as by performing RNAi on the NAD+ salvage pathway genes that produce nicotinamide. One can also inhibit genes that are involved in the *de novo* synthesis of nicotinamide. For example, nicotinamide levels in cells can be regulated by regulating the level or activity of poly(adenosine diphosphate-ribose) polymerase-1 (PARP). In particular, nicotinamide levels can be reduced by reducing the level or activity of PARP, since this enzyme generates nicotinamide. Nicotinamide levels may also be decreased in cells by reducing the level or activity of glycohydrolases (e.g., human CD38, an ectoenzyme that is expressed on the surface of immune cells, such as neutrophils; gi:4502665 and GenBank Accession No. NP_001766), which cleave NAD to nicotinamide.

Nicotinamide levels may also be decreased by inhibiting the de novo nicotinamide synthesis pathway. Genes involved in this pathway include the BNA genes in S. cerevisiae (BNA1-6). Alternatively, poly(adenosine diphosphate-ribose) polymerase (PARP) family members, e.g., PARP-1 and PARPv and tankyrase can also be inhibited to decrease nicotinamide levels.

It is also possible to reduce the level or activity of nicotinamide transporters to reduce the level of nicotinamide that is imported into cells. For example, in yeast, nicotinic acid is transported by the Tna1 (nicotinate/nicotinamide mononucleotide transport) protein. Human homologues of yeast TNA1 have the following GenBank Accession numbers: gi:9719374 and AAF97769; gi:6912666 and NP_036566; gi:18676562 and AB84933; gi: 12718201 and CAC28600; gi:19263934 and AAH25312; gi:9966811 and NP_065079; and gi:22761334 and BAC11546. Other nucleoside transporters that can be modulated include bacterial and fly nucleoside transporter and the following human genes that are homologous thereto: gi:8923160 and NP_060164; gi:14336678 and AAK61212; gi: 22749231 and NP_689812; and gi: 18603939 and XP_091525.

Alternatively, nicotinamide levels can be decreased or nicotinamide inactivated, e.g., by stimulating the activity or increase the level of enzymes that metabolize, degrade or inhibit nicotinamide, e.g., nicotinamide N-methyl transferase, also referred to as nicotinamide methyltransferase (NNMT; EC 2.1.1.1; CAS registry number 9029-74-7). This enzyme catalyzes the reaction S-adenosyl-L-methionine + nicotinamide = S-adenosyl-L-homocysteine + 1-methylnicotinamide and promotes excretion of nicotinamide from the cell (see also, Cantoni (1951) J. Biol. Chem. 203-216). The human enzyme is referred to as NNMT and its complete sequence can be found at GenBank Accession number U08021 and as SEQ ID NO: 9 for the nucleotide sequence and SEQ ID NO: 10 for the protein (Aksoy et al. (1994) J. Biol. Chem. 269:14835). The yeast version of this enzyme is referred to as NNT1 (also referred to as YLR258w).

Yet another enzyme that metabolizes nicotinamide and thereby reduces the level of nicotinamide is nicotinamide phosphribosyltransferase (NAMPRT; E.C.2.4.2.12). The human gene is also referred to as pre-B-cell colony enhancing factor (PBEF), and its sequence is available under GenBank Accession numbers NP_005737; NM_005746; AAH20691; and BC020691. The nucleotide and amino acid sequences of human NAMPRT (BC020691) are set forth as SEQ ID NOs: 11 and 12, respectively. In yeast and human cells, the level of NPT1 or human homolog thereof, respectively, can be increased to reduce nicotinamide levels.

Another enzyme that metabolizes nicotinamide and may thereby modulate, e.g., reduce, the level of nicotinamide is nicotinamide mononucleotide (NMN) adenylyltransferase in human cells. The human enzyme is referred to as NMNAT-1 (E.C.2.7.7.18). The following GenBank Accession numbers are provided for the human enzyme: NP_073624; NM_022787; AAL76934; AF459819; and NP_073624; AF314163. A variant of this gene is NMNAT-2 (KIAA0479), the human version of which can be found under GenBank Accession numbers NP_055854 and NM_015039 (Raffaelli et al. (2002) Biochem Biophys Res Commun 297:835). In yeast cells, the equivalent enzymes in the NAD+ salvage pathway are nicotinate mononucleotide adenyltransferase 1 and 2 (NMA1 and NMA2, respectively) (E.C. 2.7.7.1).

Yet another enzyme that may be increased to decrease nicotinamide levels is phosphoribosyl pyrophosphate (PRPP) synthase (PRPS), which converts ribose 5-phosphate to PRPP, the substrate of NPT1. There are several related enzymes, having the following GenBank Accession numbers: gi:4506127 and NP_002755 (Prps1); gi:4506129 and NP_002756 (Prps2); gi:20539448; gi:4506133 and NP_002758 (Prps associated protein 2); gi:24418495 and Q14558 (Prps associated protein 1); gi:17644236 and CAD18892; gi:2160401 and BAA05675 (Prps isoform 1); and gi:2160402 and BAA05676 (Prps isoform 2).

Reducing nicotinamide levels in cells may also provide other advantages, such as stimulating DNA break repair. Indeed, PARP is regulated by nicotinamide (nicotinamide negatively regulates PARP). Thus, regulating the level of nicotinamide in cells, e.g., as further described herein, will regulate the activity of PARP. Accordingly, since PARP is involved in numerous cellular functions, such as DNA break repair, telomere-length regulation, and histone modification, modulating nicotinamide levels will modulate these activities. For example, reducing nicotinamide levels in cells will increase the activity of PARP and thereby further enhance the DNA break repair mechanism of cells.

In addition to applying the methods of the invention in eukaryotic cells, such as mammalian cells and yeast cells, the methods can also be applied to plant cells, based at least on the fact that Sir2 family members are present in plants. Accordingly, the invention also provides methods for extending the life span of plants and plant cells and for rendering the plant and plant cells more resistant to stress, e.g., excessive salt conditions. This can be achieved, e.g., by modulating the level or activity of proteins in the plant cells that are essentially homologous to the proteins described herein in the yeast and mammalian systems as increasing the life span and/or the stress resistance of cells. Alternatively, the level of nicotinamide in plant cells can be reduced, in particular, as described herein for modulating their level in other eukaryotic cells. Nucleic acids can be introduced into plant cells according to methods known in the art.

For example, the following are genes form *Arabidopsis thalainia* that are homologous to the genes described above that can be modulated to modulate the flux throught the NAD+ salvage pathway or nicotinamide levels in cells. Homologues of yeast PNC1: gi 18401044 NP_566539.1 (a putative hydrolase); gi 15237256 NP_1977131; and gi 15237258 NP_197714.1. Homologues of yeast NPT1: gi 2026021 AAM13003.1; gi 15234571 NP_195412.1; gi 25054896 AAN71931.1; and gi 15227832 NP_179923.1. Homologues of yeast NMA1/2: gi 22327861 NP_200392.2 and gi 9758615 BAB09248.1. Homologues of yeast NNT1 (YL285W): gi 20197178 AAC14529; gi 22325900 NP_565619.2; gi 15219438 NP_177475.1 (a Tumor related Protein); gi 12324311 AA652120.1; gi:22330409 NP_683465; gi:15240506 NP_199767; gi 8778835 AAF79834.1; and gi 15231011 NP_188637. Homologue of human NNMT: gi 15238203 NP_196623. Homologue of yeast QNS1 (gene downstream of NMA1/2 in the NAD+ salvage pathway): gi:15221990 NP_175906. Homologues of yeast BNA6: gi:18379203 NP_565259 and gi:21555686 AAM63914.

The methods of the invention can also be used to increase the lifespan and stress resistance in microorganisms, such as prokaryotes, based on the fact that Sir2 family members are also present in these organisms.

### 3. Methods for reducing the life span of a cell or rendering it more susceptible to certain stresses

In one embodiment, the level of expression or activity of a protein selected from the group consisting of NPT1, PNC1, NMA1 and NMA2 is decreased in a cell. This can be achieved by introducing into the cell an agent that inhibits the expression of the corresponding gene. An agent can be a small molecule that acts directly or indirectly on the promoter of the corresponding gene to reduce or inhibit its transcription. An agent can also be a compound that inhibits the biological activity of the protein. An agent can also be an antisense molecule, a triplex molecule or a si RNA. Yet other agents are nucleic acids encoding a protein, such as a dominant negative mutant or an intracellular antibody or other protein that interferes with the biological activity of the protein. Such methods are well known in the art. Exemplary methods are set forth below.

One method for decreasing the level of expression of a gene in a cell is to introduce into the cell antisense molecules which are complementary to at least a portion of the target gene or RNA. An "antisense" nucleic acid as used herein refers to a nucleic acid capable of hybridizing to a sequence-specific (e.g., non-poly A) portion of the target RNA, for example its translation initiation region, by virtue of some sequence complementarity to a coding and/or non-coding region. The antisense nucleic acids of the invention can be oligonucleotides that are double-stranded or single-stranded, RNA or DNA or a modification or derivative thereof, which can be directly administered in a controllable manner to a cell or which can be produced intracellularly by transcription of exogenous, introduced sequences in controllable quantities sufficient to perturb translation of the target RNA.

Preferably, antisense nucleic acids are of at least six nucleotides and are preferably oligonucleotides (ranging from 6 to about 200 oligonucleotides). In specific aspects, the oligonucleotide is at least 10 nucleotides, at least 15 nucleotides, at least 100 nucleotides, or at least 200 nucleotides. The oligonucleotides can be DNA or RNA or chimeric mixtures or derivatives or modified versions thereof, single-stranded or double-stranded. The oligonucleotide can be modified at the base moiety, sugar moiety, or phosphate backbone. The oligonucleotide may include other appending groups such as peptides, or agents facilitating transport across the cell membrane *(see, e.g.,* Letsinger et al., 1989, Proc. Natl. Acad. Sci. U.S.A. 86: 6553-6556; Lemaitre et al., 1987, Proc. Natl. Acad. Sci. 84: 648-652: PCT Publication No. WO 88/09810, published Dec. 15, 1988), hybridization-triggered cleavage agents (*see*, *e.g.*, Krol et al., 1988, BioTechniques 6: 958-976) or intercalating agents (*see*, *e.g.*, Zon, 1988, Pharm. Res. 5: 539-549).

In a preferred aspect of the invention, an antisense oligonucleotide is provided, preferably as single-stranded DNA. The oligonucleotide may be modified at any position on its structure with constituents generally known in the art. For example, the antisense oligonucleotides may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl)uracil, (acp3)w, and 2,6-diaminopurine.

In another embodiment, the oligonucleotide comprises at least one modified sugar moiety selected from the group including, but not limited to, arabinose, 2-fluoroarabinose, xylulose, and hexose.

In yet another embodiment, the oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the oligonucleotide is a 2-α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641).

The oligonucleotide may be conjugated to another molecule, e.g., a peptide, hybridization triggered cross-linking agent transport agent, hybridization-triggered cleavage agent, etc. An antisense molecule can be a "peptide nucleic acid" (PNA). PNA refers to an antisense molecule or anti-gene agent which comprises an oligonucleotide of at least about 5 nucleotides in length linked to a peptide backbone of amino acid residues ending in lysine. The terminal lysine confers solubility to the composition. PNAs preferentially bind complementary single stranded DNA or RNA and stop transcript elongation, and may be pegylated to extend their lifespan in the cell.

The antisense nucleic acids of the invention comprise a sequence complementary to at least a portion of a target RNA species. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a target RNA it may contain and still form a stable duplex (or triplex, as the case may be). One skilled in the art can ascertain a tolerable degree of mismatch by use of standard procedures to determine the melting point of the hybridized complex. The amount of antisense nucleic acid that will be effective in the inhibiting translation of the target RNA can be determined by standard assay techniques.

The synthesized antisense oligonucleotides can then be administered to a cell in a controlled manner. For example, the antisense oligonucleotides can be placed in the growth environment of the cell at controlled levels where they may be taken up by the cell. The uptake of the antisense oligonucleotides can be assisted by use of methods well known in the art.

In an alternative embodiment, the antisense nucleic acids of the invention are controllably expressed intracellularly by transcription from an exogenous sequence. For example, a vector can be introduced *in vivo* such that it is taken up by a cell, within which cell the vector or a portion thereof is transcribed, producing an antisense nucleic acid (RNA) of the invention. Such a vector would contain a sequence encoding the antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequences encoding the antisense RNAs can be by any promoter known in the art to act in a cell of interest. Such promoters can be inducible or constitutive. Most preferably, promoters are controllable or inducible by the administration of an exogenous moiety in order to achieve controlled expression of the antisense oligonucleotide. Such controllable promoters include the Tet promoter. Other usable promoters for mammalian cells include, but are not limited to: the SV40 early promoter region (Bernoist and Chambon, 1981, Nature 290: 304-310), the promoter contained in the 3' long terminal repeat of Rous sarcoma virus (Yamamoto et al., 1980, Cell 22: 787-797), the herpes thymidine kinase promoter (Wagner et al., 1981, Proc. Natl. Acad. Sci. U.S.A. 78: 1441-1445), the regulatory sequences of the metallothionein gene (Brinster et al., 1982, Nature 296: 39-42), etc.

Antisense therapy for a variety of cancers is in clinical phase and has been discussed extensively in the literature. Reed reviewed antisense therapy directed at the Bcl-2 gene in tumors; gene transfer-mediated overexpression of Bcl-2 in tumor cell lines conferred resistance to many types of cancer drugs. (Reed, J.C., N.C.I. (1997) 89:988-990). The potential for clinical development of antisense inhibitors of *ras* is discussed by Cowsert, L.M., Anti-CancerDrugDesign (1997) 12:359-371. Additional important antisense targets include leukemia (Geurtz, A.M., Anti-Cancer Drug Design (1997) 12:341-358); human C-ref kinase (Monia, B.P., Anti-Cancer Drug Design (1997) 12:327-339); and protein kinase C (McGraw et al., Anti-Cancer Drug Design (1997) 12:315-326.

In another embodiment, the level of a particular mRNA or polypeptide in a cell is reduced by introduction of a ribozyme into the cell or nucleic acid encoding such. Ribozyme molecules designed to catalytically cleave mRNA transcripts can also be introduced into, or expressed, in cells to inhibit expression of a gene (*see, e.g.*, Sarver et al., 1990, Science 247:1222-1225 and U.S. Patent No. 5,093,246). One commonly used ribozyme motif is the hammerhead, for which the substrate sequence requirements are minimal. Design of the hammerhead ribozyme is disclosed in Usman et al., Current Opin. Struct. Biol. (1996) 6:527-533. Usman also discusses the therapeutic uses of ribozymes. Ribozymes can also be prepared and used as described in Long et al., FASEB J. (1993) 7:25; Symons, Ann. Rev. Biochem. (1992) 61:641; Perrotta et al., Biochem. (1992) 31:16-17; Ojwang et al., Proc. Natl. Acad. Sci. (USA) (1992) 89:10802-10806; and U.S. Patent No. 5,254,678. Ribozyme cleavage of HIV-I RNA is described in U.S. Patent No. 5,144,019; methods of cleaving RNA using ribozymes is described in U.S. Patent No. 5,116,742; and methods for increasing the specificity of ribozymes are described in U.S. Patent No. 5,225,337 and Koizumi et al., Nucleic Acid Res. (1989) 17:7059-7071. Preparation and use of ribozyme fragments in a hammerhead structure are also described by Koizumi et al., Nucleic Acids Res. (1989) 17:7059-7071. Preparation and use of ribozyme fragments in a hairpin structure are described by Chowrira and Burke, Nucleic Acids Res. (1992) 20:2835. Ribozymes can also be made by rolling transcription as described in Daubendiek and Kool, Nat. Biotechnol. (1997) 15(3):273-277.

Another method for decreasing or blocking gene expression is by introducing double stranded small interfering RNAs (siRNAs), which mediate sequence specific mRNA degradation. RNA interference (RNAi) is the process of sequence-specific, post-transcriptional gene silencing in animals and plants, initiated by double-stranded RNA (dsRNA) that is homologous in sequence to the silenced gene. *In vivo*, long dsRNA are cleaved by ribonuclease III to generate 21- and 22-nucleotide siRNAs. It has been shown that 21-nucleotide siRNA duplexes specifically suppress expression of endogenous and heterologous genes in different mammalian cell lines, including human embryonic kidney (293) and HeLa cells (Elbashir et al. Nature 2001 ;411(6836):494-8). Accordingly, translation of a gene in a cell can be inhibited by contacting the cell with short doublestranded RNAs having a length of about 15 to 30 nucleotides, preferably of about 18 to 21 nucleotides and most preferably 19 to 21 nucleotides. Alternatively, a vector encoding for such siRNAs or hairpin RNAs that are metabolized into siRNAs can be introduced into a target cell (see, e.g., McManus et al. (2002) RNA 8:842; Xia et al. (2002) Nature Biotechnology 20:1006; and Brummelkamp et al. (2002) Science 296:550. Vectors that can be used are commercially available, e.g., from OligoEngine under the name pSuper RNAi System^{™}.

Gene expression can also be reduced by targeting deoxyribonucleotide sequences complementary to the regulatory region of the target gene (i.e., the gene promoter and/or enhancers) to form triple helical structures that prevent transcription of the gene in target cells in the body. (See generally, Helene, C. 1991, Anticancer Drug Des., 6(6):569-84; Helene, C., et al., 1992, Ann, N.Y. Accad. Sci., 660:27-36; and Maher, L.J., 1992, Bioassays 14(12):807-15).

In a further embodiment, RNA aptamers can be introduced into or expressed in a cell. RNA aptamers are specific RNA ligands for proteins, such as for Tat and Rev RNA (Good et al., 1997, Gene Therapy 4: 45-54) that can specifically inhibit their translation.

Yet another method of decreasing the biological activity of a polypeptide is by introducing into the cell a dominant negative mutant. A dominant negative mutant polypeptide will interact with a molecule with which the polypeptide normally interacts, thereby competing for the molecule, but since it is biologically inactive, it will inhibit the biological activity of the polypeptide. A dominant negative mutant of a protein can be created, e.g., by mutating the substrate-binding domain, the catalytic domain, or a cellular localization domain of the polypeptide. Preferably, the mutant polypeptide will be overproduced. Point mutations are made that have such an effect. In addition, fusion of different polypeptides of various lengths to the terminus of a protein can yield dominant negative mutants. General strategies are available for making dominant negative mutants. *See* Herskowitz, Nature (1987) 329:219-222.

In another embodiment, the activity of one or more proteins selected from the group consisting of NPT1, PNC1, NMA1 and NMA2 is decreased. This can be accomplished, e.g., by contacting a cell with a compound that inhibits the activity, e.g., enzymatic activity, of one of these proteins. Assays for identifying such compounds are further described herein.

In another embodiment, the flux through the NAD+ salvage pathway in a cell is decreased by contacting the cell with nicotinamide or a variant thereof having substantially the same biological activity. In a preferred embodiment, a cell is contacted with an amount of nicotinamide of about 0.1 mM to about 100 mM, preferably about 1 mM to about 20mM, even more preferably 2 mM to about 10 mM, and most preferably about 5 mM. Nicotinamide is commercially available (see, e.g., the source provided in the Examples). A cell is contacted with nicotinamide for a time sufficient to exert the desired effect. For example, a cell can be contacted for at least about 60 minutes or at least about 24 hours with nicotinamide. A cell may also be contacted continously with nicotinamide.

In addition to nicotinamide, cells can be contacted with analogs thereof. Exemplary analogs include Pyrazinamide, which is sold as an antituberculous agent. ) Analogs can be identified, e.g., by screening of combinatorial libraries of analogs for those having the desired activity. For example, an assay for measuring life span can be used. Alternatively, analogs of nicotinamide or agents that interact with the C pocket of Sir2 family members can be identified by rational drug design, as further described herein.

Exemplary analogs or derivatives of nicotinamide include compounds of formula I: 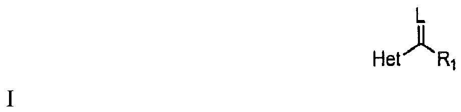 wherein,
L is O, NR, or S;
R is alkyl or phenyl;
R₁ is -NH₂, -O-alkyl, -N(R)₂, or -NH(R); and
Het is heteroaryl or heterocycloalkyl,

Particular analogs that may be used include compounds of formula I and the attendant definitions, wherein L is O; compounds of formula I and the attendant definitions, wherein R₁ is -NH₂; compounds of formula I and the attendant definitions, wherein Het is selected from the group consisting of pyridine, furan, oxazole, imidazole, thiazole, isoxazole, pyrazole, isothiazole, pyridazine, pyrimidine, pyrazine, pyrrole, tetrahydrofuran, 1:4 dioxane, 1,3,5-trioxane, pyrrolidine, piperidine, and piperazine; compounds of formula I and the attendant definitions, wherein Het is pyridine; compounds of formula I and the attendant definitions, wherein L is O and R₁ is -NH₂; compounds of formula I and the attendant definitions, wherein L is O and Het is pyridine; compounds of formula I and the attendant definitions, wherein R₁ is -NH₂ and Het is pyridine; and compounds of formula I and the attendant definitions, wherein L is O, R₁ is -NH₂, and Het is pyridine.

Other exemplary analogs or derivatives of nicotinamide that can be used include compounds of formula II: 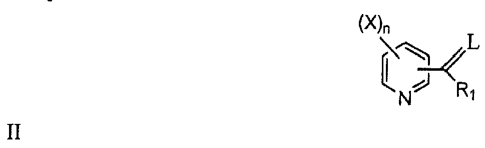 wherein,
L is O, NR, or S;
R is alkyl or phenyl;
R₁ is -NH₂, -O-alkyl, -N(R)₂, or -NH(R);
X is H, alkyl, -O-alkyl, OH, halide, or NH₂; and
n is an integer from 1 to 4 inclusive.

Particular analogs that may be used include compounds of formula II and the attendant definitions, wherein L is O; compounds of formula II and the attendant definitions, wherein R₁ is -NH₂; compounds of formula II and the attendant definitions, wherein X is H and n is 4; compounds of formula II and the attendant definitions, wherein L is O and R₁ is -NH₂; compounds of formula II and the attendant definitions, wherein L is O, X is H, and n is 4; compounds of formula II and the attendant definitions, wherein R₁ is - NH₂, X is H, and n is 4; and compounds of formula II and the attendant definitions, wherein L is O, R₁ is -NH₂, X is H, and n is 4.

Pharmaceutically acceptable salts and prodrugs of the compounds described herein may also be used.

Generally, any inhibitor of a Sir2 family member can be used to reduce the life span of cells. Preferred inhibitors are molecules that bind to the C pocket of a Sir2 family member, e.g., nicotinamide or analogs thereof.

Alternatively, the level or activity of enzymes that produce nicotinamide can be increased in a cell in which it is desired to reduce its lifespan or render it more susceptible to stress. For example, the level or activity of enzymes involved in the biosynthesis of nicotinamide in the NAD+ salvage pathway or in *de novo* synthesis pathways can be increased. Exemplary enzymes are set forth above in the previous section. Yet another method for increasing the level of nicotinamide in cells includes inhibiting enzymes that inactivate or degrade nicotinamide, e.g., nicotinamide methyl transferase in yeast and human cells; nicotinamide phosphoribosyltransferase in human cells (discussed above) and yeast NPT1 or human homologs thereof (also described above). Methods for modulating gene expression levels or protein activity are further described herein and also known in the art.

In yet other embodiments, nicotinamide levels can be increased in cells by increasing the level or activity of glycohydrolases, which cleave NAD to nicotinamide. It is also possible to increase the level or activity of nicotinamide transporters to increase the level of nicotinamide in cells.

Decreasing the lifespan of cells or their resistance to stress can also be achieved in plant cells and microorganisms, by modulating plant genes that correspond to the genes described above. These genes have been described in the previous section.

### 4. Methods for identifying agents that modulate the flux through the NAD+ salvage pathway or the level of nicotinamide in cells

Agents include small molecules, e.g., small organic molecules, or any biological macromolecule, e.g., a nucleic acid, such as DNA or RNA, single stranded or double stranded; a protein or peptide; a polysaccharide; a lipid; or molecular combinations thereof.

In one embodiment, a method for identifying a compound that modulates the life span of a cell or its resistance to certain types of stresses, comprises (i) contacting a protein selected from the group consisting of NPT1, PNC1, NMA1 and NMA2 with a test compound for an amount of time that would be sufficient to affect the activity of the protein; and (ii) determining the activity of the enzyme, wherein a difference in the activity of the enzyme in the presence of the test compound relative to the absence of the test compound indicates that the test compound is a compound that modulates the life span of the cell. The method may further comprise contacting a cell with the test compound and determining whether the life span of the cell has been modulated. Alternatively, the method may further comprise contacting a cell with the test compound and determining whether the resistance of the cell to certain stresses, e.g., heatshock, osmotic stress, high temperature, calorie restriction, DNA damaging agents (e.g., U.V. and the mitochondrial mutagen ethidium bromide), inappropriate nitrogen conditions, has been modulated. Determining the activity of the enzyme can be conducted as further described herein. It can also consist of measuring the effect of the test compounds on the life span of a cell or on its resitance to stress, e.g., heatshock, osmotic stress, etc.

As will be understood by a person of skill in the art, the above-assay can also be conducted with a biologically active portion or variant of one of the above-described proteins, such as those described above. For example, a portion of a protein can consist of its catalytic site. The catalytic site of *S. cerevisae* and human NPT1 is located between about amino acids 209 and 240. The catalytic site of *S. cerevisiae* PNC1 is located at about amino acids 150-186. The catalytic site of human NMNAT (homolog of NMA1 and NMA2) is located at about amino acids 100-110 and 280-310 (both sequences contribute to the active site).

In another embodiment, the invention provides a method for identifying a compound that modulates the life span of a cell or its resistance to certain types of stresses, comprising (i) contacting a cell or a lysate, comprising a transcriptional regulatory nucleic acid of a gene selected from the group consisting of NPT1, PNC1, NMA1 and NMA2 operably linked to a reporter gene, with a test compound for an amount of time that would be sufficient to affect the transcriptional regulatory nucleic acid; and (ii) determining the level or activity of the reporter gene, wherein a difference in the level or activity of the reporter gene in the presence of the test compound relative to the absence of the test compound indicates that the test compound is a compound that modulates the life span of the cell or its resistance to certain types of stresses. The method may further comprise contacting a cell with the test compound and determining whether the life span of the cell has been modulated. The method may also further comprise contacting a cell with the test compound and determining whether the resistance of the cell to certain stresses, e.g., heatshock, has been modulated. Transcriptional regulatory nucleic acids are either known in the art or can easily be isolated according to methods well known in the art. The reporter gene can be any gene encoding a protein whose expression can be detected, e.g., by fluorescence activated cell sorting. The cell can be a prokaryotic or eukaryotic cell. The lysate can be a complete lysate of a cell, prepared according to methods known in the art, or it can be a fraction of a cell lysate or a combination of several cell lysates or fractions of cell lysates. A lysate may also comprise one or more recombinant proteins.

The invention also provides methods for regulating the level of nicotinamide in cells. Such methods may comprising identifying agents that modulate an enzyme that increases or decreases nicotinamide levels in a cell. Exemplary enzymes are described herein. Assays can be conducted essentially as described above for identifying agents that modulate the NAD+ salvage pathway.

### 5. Methods for identifying inhibitors of Sir2 and Sir2 family members

As shown herein, nicotinamide inhibits Sir2 and human SRT1. It has also been shown that nicotinamide inhibits Sir2 non-competitively by binding to the C pocket of Sir2. Accordingly, the invention provides assays, e.g., based on rational drug design, for identifying analogs of nicotinamide that are also inhibitors of Sir2 and other members of the Sir2 family of proteins which comprise a C pocket.

Accordingly, the present invention provides methods of identifying agents that can be used for reducing the life span of cells, such as to treat conditions that may benefit from reducing the life span of certain cells. One such embodiment comprises a method of identifying an agent for use as an inhibitor of a Sir2 family member using a dataset comprising the three-dimensional coordinates of at least a portion a Sir2 family member comprising the C pocket. The crystal structure of a Sir2 homolog is described in Min et al. (2001) Cell 105 269 and the structure is provided in Protein Data Bank ID code 1ICI. The C pocket is located at about amino acids 70-90 and 127-167 of human SIRT1. The C pocket of Sir2 is located at about amino acids 250-270 and 310-350. The coordinates may further comprise the coordinates of nicotinamide or an analog thereof. In a particular embodiment the three-dimensional coordinates are those of a Sir2 homolog. In other embodiments, assays comprise co-crystallizing at least a portion of a Sir2 family member comprising the C pocket with a compound, e.g., a nicotinamide analog. Co-crystallization may be in the presence or absence of NAD+,

In one embodiment a potential agent is selected by performing rational drug design with the three-dimensional coordinates of a portion of a Sir2 family member comprising at least the C pocket. As noted above, preferably the selection is performed in conjunction with computer modeling. The potential agent is then contacted with the Sir2 family member and the activity of the Sir2 family member is determined (*e.g.*, measured). A potential agent is identified as an agent that inhibits a Sir2 family member when there is a decrease in the activity determined for the Sir2 family member.

In a preferred embodiment the method further comprises preparing a supplemental crystal containing at least a portion of a Sir2 family member comprising the C pocket bound to the potential agent. Preferably the supplemental crystal effectively diffracts X-rays for the determination of the atomic coordinates to a resolution of better than 5.0 Angstroms, more preferably to a resolution equal to or better than 3.5 Angstroms, and even more preferably to a resolution equal to or better than 3.3 Angstroms. The three-dimensional coordinates of the supplemental crystal are then determined with molecular replacement analysis and a second generation agent is selected by performing rational drug design with the three-dimensional coordinates determined for the supplemental crystal. Preferably the selection is performed in conjunction with computer modeling. The second generation agent can be an analog of nicotinamide.

As should be readily apparent the three-dimensional structure of a supplemental crystal can be determined by molecular replacement analysis or multiwavelength anomalous dispersion or multiple isomorphous replacement. A candidate drug can then selected by performing rational drug design with the three-dimensional structure determined for the supplemental crystal, preferably in conjunction with computer modeling. The candidate drug can then be tested in a large number of drug screening assays using standard biochemical methodology exemplified herein.

The method can further comprise contacting the second generation agent with a Sir2 family member or portion thereof of a different species and determining (*e.g.*, measuring) the activity of the Sir2 family member or portion thereof of the other species. A potential agent is then identified as an agent for use as an essentially specific inhibitor of a Sir2 family member of a first species when there is significantly less change (a factor of two or more) in the activity of the Sir2 family member of other species relative to that observed for the Sir2 family member of the first species. Preferably no, or alternatively minimal change *(i.e.,* less than 15%) in the activity of the other species is observed.

In one aspect, the present invention provides a computer-assisted method for identifying an inhibitor of the activity of a Sir2 family member including: supplying a computer modeling application with a set of structure coordinates of a molecule or molecular complex, the molecule or molecular complex including at least a portion of a Sir2 family member comprising a C pocket; supplying the computer modeling application with a set of structure coordinates of a chemical entity, e.g., an analog of nicotinamide; and determining whether the chemical entity is an inhibitor expected to bind to or interfere with the molecule or molecular complex, wherein binding to or interfering with the molecule or molecular complex is indicative of potential inhibition of the activity of the Sir2 family member. Preferably determining whether the chemical entity is an inhibitor expected to bind to or interfere with the molecule or molecular complex includes performing a fitting operation between the chemical entity and a binding pocket of the molecule or molecular complex, followed by computationally analyzing the results of the fitting operation to quantify the association between the chemical entity and the binding pocket. The method may further includes screening a library of chemical entities. The method may also further include supplying or synthesizing the potential inhibitor, then assaying the potential inhibitor to determine whether it inhibits the activity of a Sir2 family member.

In another aspect, the present invention provides a method for making an inhibitor of a Sir2 family member, the method including chemically or enzymatically synthesizing a chemical entity to yield an inhibitor of the activity of a Sir2 family member, the chemical entity having been designed during a computer-assisted process, e.g., as described above.

The present invention further provides an apparatus that comprises a representation of a complex between Sir2 family member and nicotinamide or analog thereof. One such apparatus is a computer that comprises the representation of the complex in computer memory. In one embodiment, the computer comprises a machine-readable data storage medium which contains data storage material that is encoded with machine-readable data which comprises the atomic coordinates of the complex. The computer may further comprise a working memory for storing instructions for processing the machine-readable data, a central processing unit coupled to both the working memory and to the machine-readable data storage medium for processing the machine readable data into a three-dimensional representation of the complex. In a preferred embodiment, the computer also comprises a display that is coupled to the central-processing unit for displaying the three-dimensional representation.

### 6. Uses of the invention

In one embodiment, increasing the flux through the NAD+ salvage pathway or decreasing nicotinamide levels is used to increase the life span of cells and protect cells against at least certain stresses *in vitro.* For example, cells in culture can be treated as described herein, such as to keep them proliferating longer. This is particularly useful for primary cell cultures (i.e., cells obtained from an organism, e.g., a human), which are known to have only a limited life span in culture. Treating such cells according to methods of the invention, e.g., by integrating one or more additional copies of one or more genes selected from the group consisting of NPT1, PNC1, NMA1, NMA2, nicotinamide N-methyl transferase (NNMT and NNT1), nicotinamide phosphoribosyltransferase (NAMPRT), and optionally human nicotinamide mononucleotide adenylyltransferase (NMNAT, NMAT-1 and 2), will result in increasing the amount of time that the cells are kept alive in culture. Embryonic stem (ES) cells and pluripotent cells, and cells differentiated therefrom, can also be modified according to the methods of the invention such as to keep the cells or progeny thereof in culture for longer periods of time. Primary cultures of cells, ES cells, pluripotent cells and progeny thereof can be used, e.g., to identify compounds having particular biological effects on the cells or for testing the toxicity of compounds on the cells (i.e., cytotoxicity assays).

In other embodiments, cells that are intended to be preserved for long periods of time are treated as described herein. The cells can be cells in suspension, e.g., blood cells, or tissues or organs. For example, blood collected from an individual for administering to an individual can be treated according to the invention, such as to preserve the blood cells for longer periods of time. Other cells that one may treat for extending their lifespan and/or protect them against certain types of stresses include cells for consumption, e.g., cells from non-human mammals (such as meat), or plant cells (such as vegetables).

In another embodiment, cells obtained from a subject, e.g., a human or other mammal, are treated according to the methods of the invention and then administered to the same or a different subject. Accordingly, cells or tissues obtained from a donor for use as a graft can be treated as described herein prior to administering to the recipient of the graft. For example, bone marrow cells can be obtained from a subject, treated *ex vivo* to extend their life span and protect the cells against certain types of stresses and then administered to a recipient. In certain embodiments, the cells of the graft, e.g., bone marrow, are transfected with one or more copies of one or more genes selected from the group consisting of NPT1, PNC1, NMA1, NMA2, NMNAT, NNT1, NAMPRT, and optionally NMAT-1 or 2. The graft can be an organ, a tissue or loose cells.

In yet other embodiments, cells are treated *in vivo* to increase their life span and/or protect them against certain types of stresses. For example, skin can be protected from aging, e.g., developing wrinkles, by treating skin, e.g., epithelial cells, as described herein. In an exemplary embodiment, skin is contacted with a pharmaceutical or cosmetic composition comprising a compound that is capable of increasing the transcription of one or more genes selected from the group consisting of NPT1, PNC1, NMA1, NMA2, NMNAT, NNT1, NAMPRT, and optionally NMAT-1 or 2. In another embodiment, skin cells are contacted with a composition comprising a protein selected from the group consisting of NPT1, PNC1, NMA1, NMA2, NMNAT, NNT1, NAMPRT, and optionally NMAT-1 or 2, or a nucleic acid encoding such, and a vehicle for delivering the nucleic acid or protein to the cells.

Compounds, nucleic acids and proteins can also be delivered to a tissue or organ within a subject, such as by injection, to extend the life span of the cells or protect the cells against certain stresses.

In yet another embodiment, an agent of the invention is administered to subjects, such as to generally increase the life span of its cells and protect its cells against certain types of stresses. For example, an agent can be taken by subjects as food supplements. In one embodiment, such an agent is a component of a multi-vitamin complex.

Agents that extend the life span of cells and protect them from stress can also be administered to subjects for treatement of diseases, e.g., chronic diseases, associated with cell death, such as to protect the cells from cell death, e.g., diseases associated with neural cell death or muscular cell death. Exemplary diseases include Parkinson's disease, Alzheimer's disease, multiple sclerosis, amniotropic lateral sclerosis, and muscular dystrophy. In such cases, the agent may be administered in the tissue or organ likely to encounter cell death.

Such agents can also be administered to a subject suffering from an acute damage to an organ or tissue, e.g., a subject suffering from stroke or myocardial infarction or a subject suffering from a spinal cord injury. Agents can also be used to repair an alcoholic's liver.

Since DNA repair is also inhibited by nicotinamide, agents that reduce nicotinamide levels in cells can be used to promote DNA repair in cells. Accordingly, cells exposed to conditions that may trigger DNA damage, e.g., U.S. radiation and ethidium bromide, may be protected by contacting them before, during and/or after exposure to the DNA damaging agent, with an agent that reduces nicotinamide levels in the cell.

In other embodiments, the methods of the invention are applied to yeast cells. Situations in which it may be desirable to extend the life span of yeast cells and to protect them against certain types of stress include any process in which yeast is used, e.g., the making of beer, yogurt, and bakery, e.g., making of bread. Use of yeast having an extended life span can result in using less yeast or in having the yeast be active for longer periods of time.

The invention also provides methods for reducing the life span of a cell or rendering it more susceptible to certain stresses, e.g., heatshock, radioactivity, osmotic stress, DNA damage, e.g., from U.V. Such methods can be used whenever it is desired to reduce the life span of a cell. Exemplary methods include decreasing the level or activity of a protein selected from the group consisting of NPT1, PNC1, NMA1, NMA2, NMNAT, NNT1, NAMPRT, and optionally NMAT-1 or 2.

Another method includes increasing the level of nicotinamide in the cell, e.g., by contacting the cell with nicotinamide, or by increasing the level or activity of an enzyme stimulating nicotinamide biosynthesis or decreasing the level or activity of an enzyme inhibiting or degrading nicotinamide, e.g., by decreasing the level or activity of NPT1, PNC1, NMA1, NMA2, NMNAT, NNT1, NAMPRT, and optionally NMAT-1 or 2. Exemplary situations in which one may wish to reduce the life span of a cell or render it more susceptible to certain stresses include treatment of cancer, autoimmune diseases or any other situation in which it is desirable to eliminate cells in a subject. Nicotinamide or other compounds or agents of the invention can be administered directly to the area containing the underirable cells, e.g., in a tumor. These methods can also be used to eliminate cells or prevent further proliferation of undesirable cells of non-malignant tumors, e.g., warts, beauty spots and fibromas. For example, nicotinamide can be injected into a wart, or alternatively be included in a pharmaceutical composition for applying onto the wart.

Methods for decreasing the life span of cells or increasing their susceptibility to certain stresses can be applied to yeast, e.g., yeast infecting subjects. Accordingly, a composition comprising an agent, e.g., nicotinamide, can be applied to the location of the yeast infection.

Subjects that may be treated as described herein include eukaryotes, such as mammals, e.g., humans, ovines, bovines, equines, porcines, canines, felines, non-human primate, mice, and rats. Cells that may be treated include eukaryotic cells, e.g., from a subject described above, or plant cells, yeast cells and prokaryotic cells, e.g., bacterial cells.

### 7. Pharmaceutical compositions and methods

Compounds, nucleic acids, proteins, cells and other compositions can be administered to a subject according to methods known in the art. For example, nucleic acids encoding a protein or an antisense molecule can be administered to a subject as described above, e.g., using a viral vector. Cells can be administered according to methods for administering a graft to a subject, which may be accompanied, e.g., by administration of an immunosuppressant drug, e.g., cyclosporin A. For general principles in medicinal formulation, the reader is referred to Cell Therapy: Stem Cell Transplantation, Gene Therapy, and Cellular Immunotherapy, by G. Morstyn & W. Sheridan eds, Cambridge University Press, 1996; and Hematopoietic Stem Cell Therapy, E. D. Ball, J. Lister & P. Law, Churchill Livingstone, 2000.

The present invention is further illustrated by the following examples which should not be construed as limiting in any way. The contents of all cited references (including literature references, issued patents, published patent applications as cited throughout this application) are hereby expressly incorporated by reference.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization(B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### Examples

### Example 1: Manipulation of a nuclear NAD⁺ salvage pathway delays aging

Yeast deprived of nutrients exhibit a marked life span extension that requires the activity of the NAD⁺-dependent histone deacetylase, Sir2p. Here we show that increased dosage of *NPT1,* encoding a nicotinate phosphoribosyltranfserase critical for the NAD⁺ salvage pathway, increases Sir2-dependent silencing, stabilizes the rDNA locus and extends yeast replicative life span by up to 60%. Both *NPT1* and *SIR2* provide resistance against heat shock, demonstrating that these genes act in a more general manner to promote cell survival. We show that Npt1 and a previously uncharacterized salvage pathway enzyme, Nma2, are both concentrated in the nucleus, indicating that a significant amount of NAD⁺ is regenerated in this organelle. Additional copies of the salvage pathway genes, *PNC1, NMA1* and *NMA2* increase telomeric and rDNA silencing, implying that multiple steps affect the rate of the pathway. Although SIR2-dependent processes are enhanced by additional *NPT1,* steady-state NAD⁺ levels and NAD⁺/NADH ratios remain unaltered. This finding suggests that yeast life span extension may be facilitated by an increase in the availability of NAD⁺ to Sir2, though not through a simple increase in steady-state levels. We propose a model in which increased flux through the NAD⁺ salvage pathway is responsible for the Sir2-dependent extension of life span.

### EXPERIMENTAL PROCEDURES

*Plasmids and strains-Strains* used in this study are listed in Table 2. W303AR5 *sir3::URA3* (16), W303AR5 *sir4::HIS3,* W303AR5 *sir2::TRP1* and PSY316AT are described (41). Deletion of *SIR2* in PSY316AT was performed using *ScaI*/*PvuII* linearized pC369 (41). JS209, JS241, JS237 and JS218 were gifts from J. Smith (42). The coding region and 1.1 kb of upstream sequence of *NPT1* were amplified by PCR (43) and the 2.4 kb product fragment was subcloned into the pRS306 based vector pSP400 between *NotI* and *Sac*I (gift from L. Guarente, M.I.T.) and the 2µ-based vector pDB20 (44) to generate pSPNPT1 and pDBNPT1 respectively.

**Table 2. Yeast strains used in this study.**

| Strain | Genotype |
|---|---|
| W303AR5 | W303 *MAT***a**, *ade2-1, leu2-3,112, can1-100, trpl-1, ura3-52, his3-11,15, RDN1::ADE2, RAD5* |
| YDS878 | W303 *MAT***a**,, *ade2-1, leu2-3,112, can1-100, trpl-1, ura3-52, his3-11,15, RDN1::ADE2, RAD5, sir2:TRP1* |
| YDS924 | W303AR5 *MAT***a**, *ade2-1, leu2-3,112, can1-100, trp1-1, ura3-52, his3-11,15, RDN1:ADE2, RAD5, sir3:HIS3* |
| YDS882 | W303 *MAT***a**, *ade2-1, leu2-3,112, can1-100, trpl-1, ura3-52, his3-11,15, RDN1::ADE2, RAD5, sir4:HIS3* |
| YDS1503 | W303 *MAT***a**, *ade2-1,* /*eu2-3, 112, can1-100, trpl-1, ura3-52, his3-11,15, RDN1::ADE2, RAD5, URA3*/*NPT1* |
| YDS1504 | W303 *MAT***a,** *ade2-1, leu2-3,112, can1-100, irpl-1, ura3-52, his3-11,15, RDN1.:ADE2, RAD5, sir2:TRP1, URA3*/*NPT1* |
| YDS1505 | W303 *MAT***a**, *ade2-1, leu2-3, I12, can1-100, trpl-1, ura3-52, his3-11,15, RDN1::ADE2, RAD5, sir3:HIS3, URA3*/*NPT1* |
| YDS1506 | W303 *MAT***a**, *ade2-1, leu2-3,112, can1-100, trpl-1, ura3-52, his3-11,15, RDN1::ADE2, RAD5, sir4:H1S3, URA3*/*NPT1* |
| YDS1496 | W303 *MAT***a**, *ade2-1, leu2-3,112, can1-100, trpl-1, ura3-52, his3-11,15, RDN1::ADE2, RAD5,* pDBNPT1 |
| YDS1494 | W303 *MAT***a**, *ade2-1, leu2-3,112, car:1-100, trpl-1, ura3-52, his3-11,15, RDN0::ADE2, RAD5, sir2:TRP1,* pDBNPT1 |
| YDS1587 | W303 *MAT***a**, *ade2-1,* /*eu2-3,112, can1-100, Irpl-1, ura3-52, his3-11,15, RDN1::ADE2, RAD5, sir3:HIS3,* pDBNPT1 |
| YDS1495 | W303 *MAT***a**, *ade2-1, leu2-3,112, can1-100, trpl-1, ura3-52, his3-11,15, RDN1::ADE2, RAD5, sir4:HIS3,* pDBNPT1 |
| YDS1572 | W303 *MAT***a**, *ade2-1, leu2-3,112, can1-100, trp1-1, ura3-52, his3-11,15, RDN1::ADE2, RAD5, LEU2*/*SIR2* |
| YDS1561 | W303 *MAT***a**, *ade2-1, feu2-3,112, can1-100, trp1-1, ura3-52, his3-11,15, RDN1::ADE2, RAD5, URA3*/*NPT1, LEU2*/*SIR2* |
| YDS1595 | W303 *MAT***a**, *ade2-1, leu2-3, 112, can1-100, trp1-1, ura3-52, his3-11,15, RAD5* |
| YDS 1596 | W303 *MAT*a, *ADE2, leu2-3,112, can1-100, trp1-1, ura3-52, his3-11,15, RAD5* |
| YDS1568 | W303 *MAT*a, *ade2-1, leu2-3,112, can1-100, trp1-1, URA3, his3-11,15, RDN1::ADE2, RAD5* |
| YDS1563 | W303 *MAT*a, *ade2-1, LEU2, can1-100, trp1-1, URA3, his3-11,15, RDN1::ADE2, RAD5* |
| YDS1588 | W303 *MAT*a, *ade2-1, leu2-3,112, can1-100, Irp1-1, ura3-52, his3-11,15, RDN1::ADE2, RAD5, pSPYGL037* |
| YDS1589 | W303 *MAT*a, *ade2-1, leu2-3,112, can1-100, trp1-1, ura3-52, his3-11,15, RDN1::ADE2, RAD5, pSPYGR010* |
| YDS1590 | W303 *MAT*a, *ade2-1, leu2-3,112, can1-100, trp1-1, ura3-52, his3-11,15, RDN1::ADE2, RAD5, p306YLR328* |
| YDS1614 | W303 *MAT*a, *ade2-1, leu2-3,112, can1-100, trp1-1, ura3-52, his3-11,15, RDN1::ADE2, RAD5, p306YHR074* |
| YDS 1531 | W303 *MAT*a, *ade2-1, leu2-3,112, can1-100, trp1-1, ura3-52, his3-11,15, RDN1::ADE2, RAD5, NPT1-HA* |
| W303cdc25-10 | W303 *MAT*a, *ade2-1, leu2-3, 112, can1-100, trp1-1, ura3-52, his3-11,15, RDN1::ADE2, RAD5, cdc25-10* |
| YDS1537 | W303 *MAT*a, *ade2-1, leu2-3,112, can1-100, trp1-1, ura3-52, his3-11,15, RDN1::ADE2, RAD5, cdc25-10, NPT1-HA* |
| YDS1611 | W303 *MAT*a, *ade2-1, leu2-3,112, can1-100, trp1-1, ura3-52, his3-11,15, RDN1::ADE2, RAD5, NPT1-GFP* |
| YDS1625 | W303 *MAT*a, *ade2-1, leu2-3,112, can1-100, trp1-1, ura3-52, his3-11,15, RDN1::ADE2, RAD5, NMA1-GFP* |
| YDS1624 | W303 *MAT*a, *ade2-1, leu2-3,112, can1-100, trp1-1, ura3-52, his3-11,15, RDN1::ADE2, RAD5, NMA2-GFP* |
| PSY316AT | *MAT*α, *urα3-53 leu2-3,112 his3-Δ200 ade2-1,01 can1-100 ADE2-TEL V-R* |
| YDS1594 | *PSY316 MATα, ura3-53 leu2-3,112 his3-Δ200 ade2-1,01 canI-100ADE2-TEL V-R, sir2:TRP1* |
| YDS1544 | PSY316 *MATα, ura3-53 leu2-3, 112 his3-Δ200 ade2-1,01 can1-100 ADE2-TEL V-R, URA3*/*NPT1* |
| YDS1548 | *PSY316 MATα, ura3-53 leu2-3,112 his3-Δ200 ade2-1.01 can1-100 ADE2-TEL V-R, (4x)URA3*/*NPT1* |
| YDS1527 | PSY316 *6 MATα, ura3-53 leu2-3, 112 his3-Δ200 ade2-1,01 canl-100ADE2-TEL V-R.* pDBNPT1 |
| YDS 1577 | PSY316 *MATα, ura3-53 leu2-3,112 his3-Δ200 ade2-1,01 can1-100 ADE2-TEL V-R, (4x)URA3*/*NPT1, LEU2*/*SIR2* |
| YDS 1573 | PSY316 *6 MATα, ura3-53 leu2-3, 112 his3-Δ200 ade2-1,01 can1-100 ADE2-TEL V-R, sir2::HIS3, URA3*/*NPT1* |
| YDS 1591 | PSY316 *MATα, ura3-53 leu2-3,112 his3-Δ200 ade2-1,01 can1-100 ADE2-TEL V-R, pSPYGL037* |
| YDS1592 | PSY316 *MATα, ura3-53 leu2-3,112 his3-Δ200 ade2-1,01 can1-100 ADE2-TEL V-R, pSPYGR010* |
| YDS1593 | PSY316 *MATα, ura3-53 leu2-3,112 his3-Δ200 ade2-1,01 can1-100 ADE2-TEL V-R, p306YLR328* |
| JS209 | *MATα, his3Δ200, leu2Δ1, met15Δ200, trp1Δ63, ura3-167* |
| JS241 | JS209 *MATα his3Δ200, leu2Δ1, met15Δ200, trp1Δ63, ura3-167, MET15* |
| JS237 | JS209 *MATα, his3Δ200, leu2Δ1, met15Δ200, trp1Δ63, ura3-167, RDN1::Ty-MET15* |
| JS218 | JS237 *MATα his3Δ200, leu2Δ1, met15Δ200, trp1Δ63, ura3-167, RDN1::Ty-MET15, sir2::HIS3* |
| YDS1583 | JS237 *MATα, his3Δ200, leu2Δ1, met15Δ200, trp1Δ63, ura3-167, RDN1::Ty-MET15. LEU2*/*SIR2* |
| YDS1522 | JS237 *MATα his3Δ200, leu2Δ1, met15Δ200, trp1Δ63, ura3-167, RDN1::Ty-MET15,* p2µSIR2 |
| YDS1580 | JS237 *MATα, his3Δ200, leu2Δ1, met15Δ200, trp1Δ63, ura3-167, RDN1::Ty-MET15. npt1Δ::kan* |
| YDS1581 | JS237 *MATα, his3Δ200, leu2Δ1, met15Δ200, trp1Δ63, ura3-167, PDN1::Ty-MET1, URA3*/*NPT1* |
| YDS1493 | JS237 *MATα, his3Δ200, leu2Δ1, met15Δ200, trp1Δ63, ura3-167, RDN1::Ty-MET15,* pDBNPT1 |

Additional copies of *NPT1* were integrated at the *URA3* locus using plasmid pSPNPT1 linearized with *StuI,* Integrants were first identified by PCR, *NPT1* copy-number was then determined by probing for *NPT1* and *ACT1* DNA on Southern blots. The density of the *NPT1* band was compared to an *ACT1* band using ImageQuant software (Molecular Dynamics, Sunnyvale, CA). Strains carrying an additional copy of *SIR2* were generated by integrating plasmids p306SIR2 or p305SIR2 (17) linearized with *XcmI.* High copy *SIR2* was introduced on the 2µ-based plasmid p2µSIR2 (gift of L. Pillus, UCSD). W303AR5 was transformed to Ura⁺ and Leu⁺ prototrophy by integrating pRS306 or pRS305 (45) linearized with *Stu*I *and Xcm*I respectively. YDS1595 was generated from W303AR5 by selecting a colony that had experienced an *ADE2* loss event. YDS 1595 was transformed with StuI-cut pRS402 (carrying the *ADE2* gene) to create YDS 1596. W303cdc25-10 was a gift from S. Lin (M.I.T) (19). The *NPT1* deletion strain, YDS1580, was generated by replacing the wildtype gene with the *kan*^{r} marker as described (46). The coding region and 650 bp upstream of *PNC1*/*YGL037* was amplified by PCR from genomic DNA. The 1350 bp *Sac*I/*Not*I fragment was cloned into the vector pSR400 to generate pSPYGL037. The coding region and 500 bp upstream of *NMA2*/*YGR010* were amplified by PCR from genomic template and the 1730 bp *Sac*I/*Not*I fragment was cloned into pSP400 to generate pSPYGR010. The coding region of *NMA1*/*YLR328* and 450 bp upstream were amplified from genomic template by PCR and the 2150 bp fragment was cloned into pRS306 to generate p306YLR328. The coding region and 600 bp upstream of *QNS1*/*YHR074* was amplified by PCR and the 2.8 kb *Sac*I*lNot*I fragment was cloned into pSP400 to make pSPYHR074. Additional copies of *PNC1*/*YGL037*, *NMA1*/*YLR328*, *NMA2*/*YGR010*, and *QNS1*/*YHR074* were integrated at the *URA3* locus of W303AR5 and PSY316AT by transformation. All amplified DNA was confirmed to to be free of mutations by sequencing.

HA-tagged *NPT1* was generated using a tag-*kan*^{r} integration method (47) in strains W303AR5 and W303cdc25-10 (19). A green fluorescent protein (GFP) cassette was introduced at the carboxy-terminus of Npt1, Nma1 and Nma2 as described (48). The functionality of tagged proteins was confirmed by assaying rDNA silencing.

*Life span determination*-Replicative life span determination was performed as described (16). Cells were grown on YPD medium (1% yeast extract, 2% bactopeptone, 2% glucose w/v) unless otherwise stated with a minimum of 40 cells per experiment. Each experiment was performed at least twice independently. Statistical significance of life span differences was determined using the Wilcoxon rank sum test. Differences are stated to be different when the confidence is higher than 95%.

*mRNA and protein determination-Northern* and Western blots were performed using standard techniques. *NPT1* transcripts were detected using a probe derived from the complete open reading frame of the *NPT1* gene. *ACT1* mRNA was detected using a full-length ACT1 probe (gift of G. Fink, M.I.T). The HA epitope tag was detected using monoclonal antibody HA.11 (CRP, Richmond, CA). Actin was detected with monoclonal antibody MAB1501R (Chemicon, Temecula, CA).

*Yeast assays and GFP localization-Yeast* strains were grown at 30°C unless otherwise stated. The extent of silencing at the ribosomal DNA locus was determined using two assays. For the *ADE2* silencing assay, cells were pre-grown on synthetic complete (SC) medium (1.67% yeast nitrogen base, 2% glucose, 40 mg/l of histidine, uridine, tryptophan, adenine and leucine) for 3 days. Cells were resuspended in SD medium and serially diluted 10-fold in phosphate-buffered saline and spotted onto SC medium lacking adenine. *MET15* silencing assays were performed on Pb²⁺-containing plates as previously described (42). Telomeric silencing was assayed on SC medium containing 0.7 mg/l adenine. Cells were grown for 3 days and placed at 4°C for 3 days to enhance color. Heat shock assays were performed essentially as described (14). Strains were pre-grown overnight in SC-complete medium with limiting histidine (20 mg/ml), diluted to 1x10⁵ cells/ml in 3 ml of the same medium and grown for 5 days. Cultures were diluted 10-fold in expired medium, incubated for 1 h at 55°C and spotted on SC plates. Ribosomal DNA recombination rates were determined as previously described (49). At least 10,000 colonies were examined for each strain and each experiment was performed in triplicate.

NAD⁺ and NADH determinations were measured as described elsewhere (50). Cells expressing a GFP fusions were grown to mid log phase in YPD medium or YPD low glucose (0.5% w/v) then incubated in PBS containing 20 µM Hoechst 33342 DNA stain (Sigma) for 5 min. Images were captured under a 100X magnification on a Nikon E600 fluorescence microscope and analyzed using Photoshop 6.0 software.

### RESULTS

*Increased dosage of NPT1 increases longevity but not steady-state NAD⁺ levels-SIR2* is a limiting component of longevity in yeast and requires NAD⁺ for catalysis. Studies in *E. coli* have shown that PncB catalyzes a rate-limiting step in the salvage pathway that recycles NAD⁺(35,37,38). We asked whether additional copies of the yeast *pncB* homolog, *NPT1,* could increase NAD⁺ production to Sir2 and hence extend yeast life span. *NPT1* was integrated at the *URA3* locus under the control of its native promoter. Strains that carried one or four tandem copies of *NPT1* were then identified by Southern blotting. We refer to the resulting genotypes as *2xNPT1* and 5x*NPT1* respectively.

For the replicative life span assay, cells were grown for at least two days on fresh yeast extract/peptone/glucose (YPD) medium to ensure that they had fully recovered from conditions of caloric restriction prior to the assay. Daughter cells that emerged from previously unbudded mother cells were then micro-manipulated away and scored. As shown in Fig. 1A, the 2x*NPT1* strain lived an average of ∼40% longer than the wild type strain and the 5x*NPT1* strain lived a striking ∼60% longer. The *NPT1*-induced life span extension was completely abrogated by a *sir2* deletion and not significantly enhanced by an additional copy of *SIR2* (Fig. 1B) indicating that the life span extension provided by *NPT1* is mediated by Sir2.

It has recently been shown that wild type cells grown in low glucose medium (0.5% w/v) have an average life span significantly greater than those grown on standard (2%) glucose medium (19,32). As shown in Fig. 1C, on low glucose medium the life span of the 5x*NPT1* strain was not significantly greater than the wild type strain. The fact that the effect of *NPT1* and low glucose were not additive suggests that these two regimens act via the same pathway.

Biochemical studies have shown that Sir2 requires NAD⁺ as a cofactor. This has led to the hypothesis that replicative life span may be extended by increased NAD⁺ levels. Consistent with this idea, NAD⁺ levels have been shown to increase significantly in old cells, perhaps as a defense against aging or as the result of decreased metabolic activity (50). To date the intracellular levels of NAD⁺ in any long-lived strain have not been reported. We found that steady-state NAD⁺ levels and NAD⁺/NADH ratios in the 2x*NPT1* strain were not significantly different from the wild type (Table 1). We also examined *Δsir2* and 2x*NPT1 Δsir2* strains and again found no difference from wild type, indicating that the failure to detect increased NAD⁺ levels was not due to the activity of Sir2.

**Table 1. Steady-state NAD⁺ and NADH levels in various long-and short-lived strains.**

| | NAD⁺ | NADH | NAD⁺/NADH | ATP |
|---|---|---|---|---|
| Genotype | (amol/pg protein)¹ | (amol/pg protein)¹ | ratio | (amol/pg protein)¹ |
| *1xNPT1* (wild type) | 23.7 (3.2) | 9.3 (0.8) | 2.8 (0.5) | 15.5 (3) |
| *2xNPT1* | 21.9 (2.0) | 6.0 (0.6) | 3.3 (0.3) | 7.6 (1.6) |
| *2xNPT1 sir2::TRP1* | 22.5 (1.6) | 7.0 (0.3) | 2.4 (0.9) | 5.3 (1.1) |
| *sir2::TRP1* | 23.6 (1.2) | 7.0 (0.6) | 2.8 (1.2) | 7.9 (1.9) |

| | | | | |
|---|---|---|---|---|
| ¹ average of five independent experiments (s.e.) | | | | |

*NPT1 and SIR2 increase resistance to heat shock but not to other stresses*-Mutations in components of the *C. elegans* and *Drosophila* insulin/IGF-1 pathway allow animals to live up to twice as long as controls (5). In *C. elegans* this longevity is coupled to stress resistance (4). In contrast, the *chico* mutation in *Drosophila*, which extends life span by ∼50% in homozygotes, does not protect against heat shock or oxidative stress (51). The link between *sir2.1* life span extension and stress resistance in *C. elegans* has not been examined, though there is evidence from yeast that the Sir2/3/4 complex may be involved in such a response. The yeast *sir4-42* mutation increases replicative life span as well as resistance to starvation and heat shock (52). This raises the possibility that the *SIR2* longevity pathway may also influence stress resistance.

To explore this, we examined the ability of extra copies of *NPT1* and *SIR2* to confer resistance to a variety of stresses including heat shock, starvation, and exposure to methylmethane sulfonate (MMS) or paraquat. MMS is a DNA damaging agent that causes a variety of DNA lesions, whereas paraquat induces oxidative stress by generating reactive oxygen species. Additional copies of either *NPT1*, *SIR2*, or both did not provide resistance against paraquat or MMS, nor did they enhance the ability to survive in stationary phase .

To assay heat shock resitance, strains with an additional copy of *NPT1* or *SIR2* were grown to stationary phase in SC medium, heat shocked for 1 hour at 55°C, then spotted in 10-fold serial dilutions onto SC plates. As shown in Fig. 2A, stains with a single additional copy of *NPT1* or *SIR2* were significantly more resistant to heat shock than the otherwise isogenic wild type control strain. No additive effect of *NPT1* and *SIR2* was apparent, consistent with these two genes acting in the same pathway. To provide a more quantitative measure of this phenotype, strains were subjected to 1 hour heat shock, plated for single colonies and the number of colonies after 24 hours was scored as a percentage of the untreated sample. As shown in Fig. 2B, additional copies of *NPT1* and *SIR2,* or both provided ∼8-fold greater survival than wild type, consistent with our earlier finding.

*Additional NPT1 increase silencing and rDNA stability-We* wished to determine the molecular basis of the *SIR2*-dependent life span extension provided by additional *NPT1.* A simple model predicts that increased dosage of *NPT1* would stimulate the NAD⁺ salvage pathway, which would in turn increase Sir2 activity. We thus examined the effect of additional copies of *NPT1* on the *SIR2*-dependent processes of silencing and stability at the rDNA locus.

To determine the effect of *NPT1* on rDNA silencing, we utilized strains with either an *ADE2* or *MET15* marker integrated at the rDNA locus (*RDN1*). We used two marker genes to ensure that the effects we observed were not simply due to changes in adenine or methionine biosynthesis. Silencing of *ADE2* results in slower growth of cells on media lacking adenine and the accumulation of a red pigment on plates with limiting adenine. Silencing of *MET15* leads to production of a brown pigment on Pb²+ -containing medium. Strains with additional copies of *SIR2* were included for comparison. The 2x*NPT1* strains showed higher levels of rDNA silencing than wild type in the *ADE2* assay (Fig. 3A, compare growth on adenine with growth on no adenine) and the *MET15* assay (Fig. 3B). Introduction of an additional copy of *NPT1* into the 2x*SIR2* strain did not further increase silencing, again consistent with the placement of these two genes in the same pathway. Strains carrying *SIR2* and *NPT1* on high-copy 2µ-based plasmids also showed increased levels of rDNA silencing (Fig. 3B and C). An additional copy of *NPT1* also increased silencing in *sir3* and *sir4* null strains (Fig. 3C). High-copy *NPT1* had a disruptive effect on rDNA silencing in the *sir3* strain, whereas this effect was not observed in the *sir4* strain. This can be explained by the fact that *sir4* mutants relocalize Sir2 to the rDNA, which may counteract the high levels of Npt1. Additional copies of *NPT1* in a *sir2* mutant caused a slight increase in rDNA silencing that was considerably weaker than *SIR2-*dependent silencing. The basis of this apparent increase is unclear. To determine whether this was a global effect on silencing, we examined silencing at a telomeric locus. An additional copy of *NPT1* was introduced into PSY316AT, which has an *ADE2* marker inserted in the subtelomeric region of chromosome V (53). As shown in Fig. 3D, additional copies of *NPT1* increased telomeric silencing in a *SIR2*-dependent manner.

Instability of the rDNA has been shown to be a major cause of yeast replicative aging. To test whether *NPT1* extends life span by increasing stability at this locus, we determined the rate of rDNA recombination in *2xNPT1* and 2x*SIR2* strains. This was achieved by measuring the rate of loss of an *ADE2* marker inserted at the rDNA. As shown in Fig. 3E, an additional copy of *NPT1* decreased rDNA recombination by 2-fold, similar to the 2x*SIR2* and 2x*NPT1* 2x*SIR2* strains. When *sir2* was deleted from the *2xNPT1* strain, rDNA recombination increased dramatically to the levels of a *sir2* null strain (Fig. 3F). These results are consistent with a model in which *NPT1* extends replicative life span by increasing the ability of Sir2 to inhibit rDNA recombination.

One plausible explanation for the increase in rDNA silencing associated with additional copies of *NPT1* is that the telomeric Sir2 in these strains is relocalized to the rDNA, which would result in the loss of telomeric silencing. We have shown that additional copies of *NPT1* increase telomeric silencing in a *SIR2*-dependent manner, arguing against relocalization of Sir2 from telomeres as the mechanism of life span extension. Another possible explanation is that additional *NPT1* upregulates Sir2 expression. By Western blotting we found that the steady-state levels of Sir2 did not change in response to additional *NPT1.* A third possibility for the increase in rDNA silencing is that additional *NPT1* stimulates overall Sir2 activity. Although it is not currently possible to measure this activity *in vivo,* this idea is consistent with our findings that additional *NPT1* enhances each of the *SIR2*-dependent processes thus far examined.

*Caloric restriction does not alter NPT1 expression or localization-Given* that additional *NPT1* and caloric restriction appear to extend life span via the same pathway, we tested whether caloric restriction acts by increasing *NPT1* expression. A triple hemagglutinin epitope (3xHA) tag was added to the carboxy-terminus of Npt1 by integrating an 3xHA-kanamycin resistance cassette into the native *NPT1* locus. We confirmed that the fusion protein was functional by assaying its ability to maintain wild type levels of rDNA silencing. *NPT1* levels were then determined in strains grown on (0.5%) glucose medium and in the long-lived *cdc25-10* strain, which is considered a genetic mimic of caloric restriction (19). As shown in Fig. 4A and B, no increase in *NPT1* expression was detected at the mRNA or protein level. In fact under low glucose conditions a consistent ∼2-fold decrease in *NPT1* expression was observed. We did not detect significant changes in *NPT1* expression after heat shock or exposure to MMS or paraquat (Fig. 4C and D). We conclude that caloric restriction does not increase longevity by upregulating *NPT1* expression.

Given that *NPT1* expression was not enhanced in response to caloric restriction, we examined the possibility that the activity of this protein may be modulated by other means. Specifically, we examined the subcellular localization of GFP-tagged Npt1 in live cells grown in complete or low glucose medium. To our surprise, Npt1 was observed throughout the cell with an apparent concentration of the protein in the nucleus of most cells (Fig. 4E). The large regions of exclusion correspond to vacuoles. These findings raise the intriguing possibility that a significant fraction of NAD⁺ is regenerated in the nucleus. In low glucose medium the localization pattern of Nptl-GFP was unaltered, indicating that there is no gross relocalization of Npt1 in response to caloric restriction.

If our hypothesis that the entire NAD⁺ salvage pathway exists in the nuclear compartment, then we should expect that the other enzymes in the pathway will show a similar localization pattern to Npt1. Based on the bacterial salvage pathway, the step immediately downstream of *NPT1* is predicted to be catalyzed by a nicotinate mononucleotide adenylyltransferase (NaMAT). There are two yeast ORFs with similar homology to NaMATs from other species, *YLR0328* and *YGR010,* which we have designated *NMA1* and *NMA2,* respectively. To localize these two proteins, a GFP cassette was integrated in frame prior to the stop codon of each ORF to generate C-terminal fusions. As shown in Fig. 4F, Nma2-GFP was concentrated in the nucleus in the majority of cells, in a pattern identical to that of Nptl-GFP. This finding further supports our hypothesis that NAD⁺ is recycled from nicotinamide entirely within the nucleus. The localization pattern of Nma1 was unable to be determined due to low expression levels.

*Identification of other putative longevity genes in the NAD⁺ salvage pathway*-The discovery that Nma2 shows a similar localization to Npt1 prompted us to test whether other genes in the NAD⁺ salvage pathway could have similar effects to Npt1 when overexpressed. While the bacterial genes in NAD⁺ salvage pathway have been studied in detail, in *S. cerevisiae* some of the key genes in the pathway remain to be characterized. *PNC1*, a recently identified gene, encodes a nicotinamidase which catalyses the conversion of nicoinamide to nicotinic acid, the step immediately upstream of *NPT1.* As discussed above, the two genes *NMA1* and *NMA2* encode NaMNATs which catalyze the step immediately downstream of *NPT1.* In bacteria, the next step in the pathway, the generation of NAD⁺, is catalyzed by an NAD synthetase. An uncharacterized ORF, *QNS1*/*YHR074*, shows high homolgy to NAD synthetases. Each of these salvage pathway genes was integrated as a single copy into the *URA3* locus of W303AR5 and PSY316AT and assayed for silencing as previously described. Additional copies of either *PNC1*, *NMA1* or *NMA2* increased rDNA and telomeric silencing to levels similar to those in a 2x*NPT1* strain (Fig. 5B and C). In contrast, additional copies of *QNS1* had no effect on either rDNA silencing (Fig. 5B) or telomeric silencing. As discussed below, these results indicate there are multiple steps that can affect the rate of the pathway and that the two homologs *NMA1* and *NMA2* may have overlapping functions.

### DISCUSSION

*NPT1* encodes a key component of the yeast salvage pathway that recycles NAD⁺, a cofactor of Sir2. We have shown that additional copies of *NPT1* increase life span by up to 60% in a *SIR2*-dependent manner. It has been proposed that longevity in yeast may be associated with increased NAD⁺ levels. However we have shown that in strains with additional copies of *NPT1,* steady-state NAD⁺ levels are unaltered. Furthermore, the NAD⁺/NADH ratios are also similar to wild type cells, indicating that total cellular redox state is not dramatically altered either.

We have also shown that *sir2* mutants have wild type NAD⁺ levels, implying that Sir2 is not a major consumer of NAD⁺. Nevertheless, by virtue of its ability to convert NAD⁺ to nicotinamide, Sir2 should be responsive to increased flux through the salvage pathway (Fig. 6). Thus, while steady-state levels of NAD⁺ remain constant, the turnover of this molecule may be elevated. Localization of GFP-tagged enzymes indicated that at least two of the enxymes in the NAD⁺ salvage pathway are concentrated in the nucleus. Consistent with this, Nma1 and Nma2 have been shown by high-throughput 2-hybrid screening to interact with Srp1, a protein that acts as a receptor for nuclear localization sequences (NLS) (54). The same 2-hybrid screen also found that Nma1 and Nma2 can interact with themselves and with each other. Perhaps Nma proteins exist as dimers, as is the case for the *Bacillus subtilis* NaMNAT (55), or as hexamers, as is the case for *Methanococcus jannaschii* (56) and *Methanobacterium thermoautotrophicum* NaMNATs (57). It is worth nothing that strains disrupted for either *NMA1* or *NMA2* are viable (58), arguing that they are functionally redundant.

In vertebrates, NaMNAT/NMNAT activity is primarily observed in the nuclear fraction of liver cell extracts (59), suggesting that nuclear compartmentalization of the pathway may be a universal property of eukaryotic cells. Having the salvage pathway in proximity to chromatin may allow NAD⁺ to be rapidly regenerated for silencing proteins. Alternatively, it may permit the coordination of a variety of nuclear activities via the alteration of nuclear NAD⁺ pools. Testing of these hypotheses will not be a simple task but one that will be greatly assisted by the development of a molecular probe for intracellular NAD⁺.

In yeast and many metazoans, a number of long-lived mutants display increased stress resistance. However, there are many examples of mutations that extend life span but provide little protection against stress, indicating that this relationship is not straightforward (4). For example, in yeast the life span extension provided by a *cdc25-10* mutation is not accompanied by heat shock resistance (19). We have shown that additional copies of *NPT1* or *SIR2* extend life span but do not provide protection against MMS, paraquat or starvation. Thus, in *S. cerevisiae* longevity is not linked to a general increase in stress resistance. The only stress-related phenotype that we found correlated with longevity was heat shock resistance. Based on genome-wide analyses of gene expression in *sir2*Δ strains, it has been proposed that Sir2 regulates genes other than those at the three silent loci (60), although this interpretation is debated (61). If the interpretation is correct, then it is plausible that the heat shock resistance we observed in *2xNPT1* and 2x*SIR2* strains results from Sir2-mediated silencing of genes that suppress heat shock resistance.

In bacteria, the Npt1 homolog PncB catalyzes a rate-limiting step in the NAD⁺ salvage pathway (35,37,38). In this study we show that additional copies of *PNC1*, *NPT1*, *NMA1* or *NMA2* all increase rDNA and telomeric silencing. The implication is that, in yeast, multiple steps can affect the rate of the pathway. Such a proposal is consistent with Metabolic Control Analysis, a theory based on the observation that flux through most metabolic pathways is controled by multiple enzymes, rather than by a single rate-liming step (62). Of all the genes in the salvage pathway, only *QNS1* had no effect on silencing, suggesting that it is the only enzyme in the pathway limited by substrate availability. This is likely due to the fact that the predicted substrate for Qns1, desamido-NAD⁺, is the only intermediate that can not be supplied from a source outside the salvage pathway (see Fig. 6).

In yeast and metazoans there are multiple members of the Sir2 family, many of which have been shown (or are predicted) to be NAD⁺-dependent deacetylases (24,63). This finding, combined with the fact that some Sir2 family members are cytoplasmic (64,65), suggests that reversible acetylation may be a much more prevalent regulatory mechanism than previously thought (66). This would place the NAD⁺ salvage pathway in a pivotal position, coordinating the activity of this group of effector proteins in response to cellular energy status

It is now widely accepted that there are conserved pathways for the regulation of longevity (4,5). The extent of this conservation is exemplified by the discovery that additional copies of *C. elegans sir-2.1* also extend life span in that organism (31). Our findings show that several *SIR2*-dependent processes can be enhanced by manipulation of the NAD⁺ salvage pathway in yeast and this may hold true for higher organisms. We have identified *NPT1* homologs in every genome we have examined and all possess a highly conserved region around a histidine residue that, in *Salmonella,* greatly stimulates catalysis when phosphorylated (67). This mode of regulation may permit the design of mutations or small molecules that increase Npt1 activity. Together, our findings show that Npt1 and other members of the salvage pathway are attractive targets for small molecules that may mimic the beneficial effects of caloric restriction.

### REFERENCES

1. Masoro, E. J. (2000) Exp Gerontol 35(3), 299-305.
2. Vanfleteren, J. R., and Braeckman, B. P. (1999) Neurobiol Aging 20(5), 487-502
3. Zainal, T. A., Oberley, T. D., Allison, D. B., Szweda, L. I., and Weindruch, R. (2000) Faseb J 14(12), 1825-36.
4. Kenyon, C. (2001) Cell 105, 165-168
5. Guarente, L., and Kenyon, C. (2000) Nature 408(6809), 255-62.
6. Kirkwood, T. B., and Rose, M. R. (1991) Philos Trans R Soc Lond B Biol Sci 332(1262), 15-24.
7. Barton, A. (1950) J Gen Microbiol 4, 84-86
8. Sinclair, D. A., Mills, K., and Guarente, L. (1997) Science 277(5330), 1313-6.
9. Mortimer, R. K., and Johnston, J. R. (1959) Nature 183, 1751-1752
10. Kennedy, B. K., Austriaco, N. R., Jr., and Guarente, L. (1994) J Cell Biol 127(6 Pt 2), 1985-93.
11. Kim, S., Villeponteau, B., and Jazwinski, S. M. (1996) Biochem Biophys Res Commun 219(2), 370-6.
12. Ashrafi, K., Lin, S. S., Manchester, J. K., and Gordon, J. I. (2000) Genes Dev 14(15), 1872-85.
13. Lin, S. S., Manchester, J. K., and Gordon, J. I. (2001) J. Biol. Chem.,
14. Longo, V. D. (1999) Neurobiol Aging 20(5), 479-86.
15. Jazwinski, S. M. (2001) Mech Ageing Dev 122(9), 865-82.
16. Sinclair, D. A., and Guarente, L. (1997) Cell 91(7), 1033-42.
17. Kaeberlein, M., McVey, M., and Guarente, L. (1999) Genes Dev 13(19), 2570-80.
18. Park, P. U., Defossez, P. A., and Guarente, L. (1999) Mol Cell Biol 19(5), 3848-56
19. Lin, S. J., Defossez, P. A., and Guarente, L. (2000) Science 289(5487), 2126-8.
20. Defossez, P. A., Prusty, R., Kaeberlein, M., Lin, S. J., Ferrigno, P., Silver, P. A., Keil, R. L., and Guarente, L. (1999) Mol Cell 3(4), 447-55
21. Tanner, K. G., Landry, J., Stemglanz, R., and Denu, J. M. (2000) Proc Natl Acad Sci U S A 97(26), 14178-82*.*
22. Imai, S., Armstrong, C. M., Kaeberlein, M., and Guarente, L. (2000) Nature 403(6771), 795-800
23. Smith, J. S., Brachmann, C. B., Celic, I., Kenna, M. A., Muhammad, S., Starai, V. J., Avalos, J. L., Escalante-Semerena, J. C., Grubmeyer, C., Wolberger, C., and Boeke, J. D. (2000) Proc Natl Acad Sci U S A 97(12), 6658-63.
24. Landry, J., Sutton, A., Tafrov, S. T., Heller, R. C., Stebbins, J., Pillus, L., and Stemglanz, R. (2000) Proc Natl Acad Sci U S A 97(11), 5807-11.
25. Laurenson, P., and Rine, J. (1992) Microbiol Rev 56(4), 543-60.
26. Straight, A. F., Shou, W., Dowd, G. J., Turck, C. W., Deshaies, R. J., Johnson, A. D., and Moazed, D. (1999) Cell 97(2), 245-56.
27. Shou, W., Seol, J. H., Shevchenko, A., Baskerville, C., Moazed, D., Chen, Z. W., Jang, J., Charbonneau, H., and Deshaies, R. J. (1999) Cell 97(2), 233-44.
28. Shou, W., Sakamoto, K. M., Keener, J., Morimoto, K. W., Traverso, E. E., Azzam, R., Hoppe, G. J., Feldman, R. M. R., DeModena, J., Moazed, D., Charbonneaux, H., Nomura, M., and Deshaies, R. J. (2001) Mol. Cell. 8(1), 45-55
29. Tanny, J. C., and Moazed, D. (2001) Proc Natl Acad Sci U S A 98(2), 415-20.
30. Smith, J. S., Caputo, E., and Boeke, J. D. (1999) Mol Cell Biol 19(4), 3184-97.
31. Tissenbaum, H. A., and Guarente, L. (2001) Nature 410(6825), 227-30.
32. Jiang, J. C., Jaruga, E., Repnevskaya, M. V., and Jazwinski, S. M. (2000) Faseb J 14(14), 2135-7.
33. Foster, J. W., Kinney, D. M., and Moat, A. G. (1979) J Bacteriol 137(3), 1165-75.
34. Ghislain, M., Talla, E., and Francois, J. M. (2002) Yeast 19(3), 215-224.
35. Wubbolts, M. G., Terpstra, P., van Beilen, J. B., Kingma, J., Meesters, H. A., and Witholt, B. (1990) J Biol Chem 265(29), 17665-72.
36. Vinitsky, A., Teng, H., and Grubmeyer, C. T. (1991) J Bacteriol 173(2), 536-40.
37. Imsande, J. (1964) Biochim. Biophys. Acta 85, 255-273
38. Grubmeyer, C. T., Gross, J. W., and Rajavel, M. (1999) Methods Enzymol 308, 28-48
39. Emanuelli, M., Carnevali, F., Lorenzi, M., Raffaelli, N., Amici, A., Ruggieri, S., and Magni, G. (1999) FEBS Lett 455(1-2), 13-7.
40. Hughes, K. T., Olivera, B. M., and Roth, J. R. (1988) J Bacteriol 170(5), 2113-20.
41. Mills, K. D., Sinclair, D. A., and Guarente, L. (1999) Cell 97(5), 609-20.
42. Smith, J. S., and Boeke, J. D. (1997) Genes Dev 11(2), 241-54.
43. Lalo, D., Carles, C., Sentenac, A., and Thuriaux, P. (1993) Proc Natl Acad Sci US A 90(12), 5524-8.
44. Becker, D. M., Fikes, J. D., and Guarente, L. (1991) Proc Natl Acad Sci U S A 88(5), 1968-72.
45. Sikorski, R. S., and Hieter, P. (1989) Genetics 122(1), 19-27.
46. Guldener, U., Heck, S., Fielder, T., Beinhauer, J., and Hegemann, J. H. (1996) Nucleic Acids Res 24(13), 2519-24.
47. De Antoni, A., and Gallwitz, D. (2000) Gene 246(1-2), 179-85.
48. Longtine, M. S., McKenzie, A., 3rd, Demarini, D. J., Shah, N. G., Wach, A., Brachat, A., Philippsen, P., and Pringle, J. R. (1998) Yeast 14(10), 953-61.
49. Keil, R. L., and McWilliams, A. D. (1993) Genetics 135(3), 711-8.
50. Ashrafi, K., Sinclair, D., Gordon, J. I., and Guarente, L. (1999) Proc Natl Acad Sci U S A 96(16), 9100-5.
51. Clancy, D. J., Gems, D., Harshman, L. G., Oldham, S., Stocker, H., Hafen, E., Leevers, S. J., and Partridge, L. (2001) Science 292(5514), 104-6.
52. Kennedy, B. K., and Guarente, L. (1996) Trends Genet 12(9), 355-9.
53. Gottschling, D. E., Aparicio, O. M., Billington, B. L., and Zakian, V. A. (1990) Cell 63(4), 751-62.
54. Uetz, P., Giot, L., Cagney, G., Mansfield, T. A., Judson, R. S., Knight, J. R., Lockshon, D., Narayan, V., Srinivasan, M., Pochart, P., Qureshi-Emili, A., Li, Y., Godwin, B., Conover, D., Kalbfleisch, T., Vijayadamodar, G., Yang, M., Johnston, M., Fields, S., and Rothberg, J. M. (2000) Nature 403(6770), 623-7.
55. Olland, A. M., Underwood, K. W., Czerwinski, R. M., Lo, M. C., Aulabaugh, A., Bard, J., Stahl, M. L., Somers, W. S., Sullivan, F. X., and Chopra, R. (2002) J Biol Chem 277(5), 3698-707.
56. D'Angelo, I., Raffaelli, N., Dabusti, V., Lorenzi, T., Magni, G., and Rizzi, M. (2000) Structure Fold Des 8(9), 993-1004.
57. Saridakis, V., Christendat, D., Kimber, M. S., Dharamsi, A., Edwards, A. M., and Pai, E. F. (2001) J Biol Chem 276(10), 7225-32.
58. Winzeler, E. A., Shoemaker, D. D., Astromoff, A., Liang, H., Anderson, K., Andre, B., Bangham, R., Benito, R., Boeke, J. D., Bussey, H., Chu, A. M., Connelly, C., Davis, K., Dietrich, F., Dow, S. W., El Bakkoury, M., Foury, F., Friend, S. H., Gentalen, E., Giaever, G., Hegemann, J. H., Jones, T., Laub, M., Liao, H., Davis, R. W., and et al. (1999) Science 285(5429), 901-6.
59. Hogeboom, G., and Schneider, W. (1950) J. Biol. Chem. 197, 611-620
60. Wyrick, J. J., Holstege, F. C., Jennings, E. G., Causton, H. C., Shore, D., Grunstein, M., Lander, E. S., and Young, R. A. (1999) Nature 402(6760), 418-21.
61. Bedalov, A., Gatbonton, T., Irvine, W. P., Gottschling, D. E., and Simon, J. A. (2001) Proc Natl Acad Sci U S A 98(26), 15113-8.
62. Fell, D. (1997) Understanding the Control of Metabolism. Frontiers in Metabolism (Snell, K., Ed.), Portland Press, London
63. Landry, J., Slama, J. T., and Sternglanz, R. (2000) Biochem Biophys Res Commun 278(3), 685-90.
64. Perrod, S., Cockell, M. M., Laroche, T., Renauld, H., Ducrest, A. L., Bonnard, C., and Gasser, S. M. (2001) Embo J 20(1-2), 197-209.
65. Afshar, G., and Murnane, J. P. (1999) Gene 234(1), 161-8.
66. Shore, D. (2000) Proc Natl Acad Sci U S A 97(26), 14030-2.
67. Rajavel, M., Lalo, D., Gross, J. W., and Grubmeyer, C. (1998) Biochemistry 37(12), 4181-8.

### Example 2: Increased genomic instability and accelerated aging by nicotinamide

The *Saccharomyces cerevisiae* Sir2 protein is an NAD⁺-dependent histone deacetylase that plays a critical role in transcriptional silencing, genome stability and longevity. A human homologue of Sir2, SIRT1, regulates the activity of the p53 tumor suppressor and inhibits apoptosis. The Sir2 deacetylation reaction generates two products: O-acetyl-ADP-ribose and nicotinamide, a precursor of nicotinic acid and a form of niacin/vitamin B3. We show here that nicotinamide completely abolishes yeast silencing and shortens replicative life span to that of a *sir2* mutant. Nicotinamide, but not nicotinic acid, strongly inhibits silencing at the telomeres, rDNA and mating type loci of yeast. Nicotinamide also increases instability of the rDNA locus and shortens yeast life span to that of a *sir2* mutant. Nicotinamide also abolishes silencing in G1-arrested cells, demonstrating that continual Sir2 activity is required to maintain silencing. In the presence of nicotinamide, Sir2 no longer associates with either telomeres or mating type loci, but remains associated with the rDNA. Sir2 no longer co-immunoprecipitates with chromatin at telomeres and mating-type loci in the presence of nicotinamide, though the Sir2 localization pattern is unaltered. We show that physiological concentrations of nicotinamide non-competitively inhibit both Sir2 and SIRT1 *in vitro.* The degree of inhibition of SIRT1 by nicotinamide (IC₅₀ < 50 µM) is equal to or better than the most effective known inhibitors of this class of proteins. We propose that nicotinamide and NAD⁺ can bind simultaneously to Sir2 preventing catalysis and discuss the possibility that inhibition of Sir2 by nicotinamide is physiologically relevant.

We discuss the possibility that nuclear nicotinamide negatively regulates Sir2 activity *in vivo.* Our findings suggest that the clinical use of nicotinamide should be given careful consideration.

### EXPERIMENTAL PROCEDURES

*Yeast assays*- All yeast strains used in this study are listed in Table 3. Cells were grown at 30°C on YPD medium (1% yeast extract, 2% bactopeptone, 2% glucose w/v) unless otherwise stated. The extent of silencing at the ribosomal DNA locus was determined by growing *RDN1::MET15* strains on Pb²⁺-containing medium (0.3% peptone, 0.5% yeast extract, 4% glucose, 0.02% (w/v) ammonium acetate, 0.07% Pb(NO₃)₂ and 2% agar). *ADE2*-based telomeric and *HM* locus silencing assays were performed as described previously (see, Example 1). Ribosomal DNA recombination frequencies were determined as previously described (44').

**Table 3. Yeast strains used in this study.**

| Strain | Genotype |
|---|---|
| W303AR5 | W303 *MAT***a***, ade2-1, leu2-3,112, can1-100, trp1-1, ura3-52, his3-11,15,* |
| | *RDN1::ADE2, RAD5* |
| YDS878 | W303 *MAT***a***,, ade2-1, leu2-3,112, can1-100, trp1-1,, ura3-52, his3-11,15,* |
| | *RDN1::ADE2, RAD5, sir2:TRP1* |
| YDS1572 | W303 *MAT***a**, *ade2-1, leu2-3,112, can1-100, trp1-1, ura3-52, his3-11,15,* |
| | *RDN1::ADE2, RAD5, LEU2*/*SIR2* |
| YDS1595 | W303 *MAT***a***, ade2-1, leu2-3,112, can1-100, trp1-1, ura3-52, his3-11,15, RAD5* |
| YDS1596 | W303 *MAT***a***, ADE2, leu2-3,112, can1-100, trp1-1, ura3-52, his3-11,15, RAD5* |
| YDS1097 | W303 *MAT***a***, ade2-1, leu2-3,112, can1-100, trp1-1, ura3-52, his3-11,15, RDN,* |
| | *RAD5, GFP-Sir4::URA3* |
| YDS1099 | W303 *MAT***a***, ade2-1, leu2-3,112, can1-100, trp1-1, ura3-52, his3-11,15, RDN,* |
| | *RAD5, GFP-Sir3::LEU2* |
| YDS1109 | W303 *MAT***a***, ade2-1, leu2-3,112, can1-100, trp1-1, ura3-52, his3-11,15, RDN,* |
| | *RAD5, GFP-Sir3::LEU2, sir2:TRP1* |
| YDS1078 | W303 *MAT***a***, ade2-1, leu2-3,112, can1-100, trp1-1, ura3-52, his3-11,15,* |
| | *RDN1::ADE2, RAD5, GFP-Sir2::LEU2, sir2:TRP1* |
| PSY316AT | *MAT*α*, ura3-53 leu2-3,112 his3-*Δ*200 ade2-1,01 can1-100 ADE2-TEL V-R* |
| YDS1594 | PSY316 *MAT*α*, ura3-53 leu2-3,112 his3-*Δ*200 ade2-1,01 can1-100 ADE2-TEL* |
| | *V-R, sir2:TRP1* |
| YDS970 | PSY316 *MAT*α*, ura3-53 leu2-3,112 his3-*Δ*200 ade2-1,01 can1-100 ADE2-TEL* |
| | *V-R, HMR::GFP* |
| YDS1005 | PSY316 *MAT*a*, ura3-53 leu2-3,112 his3-*Δ*200 ade2-1,01 can1-100 ADE2-TEL* |
| | *V-R, HMR::GFP* |
| YDS1499 | PSY316 *MAT*α*, ura3-53 leu2-3,112 his3*-Δ*200 ade2-1,01 can1-100 ADE2-TEL* |
| | *V-R, HMR::GFP, sir4:HIS3* |
| YDS1690 | PSY316 *MAT*α*, ura3-53 leu2-3,112 his3-*Δ*200 ade2-1,01 can1-100 ADE2-TEL* |
| | *V-R, HMR::GFP,* Δ*hml::LEU2* |
| JS209 | *MAT*α, *his3*Δ*200, leu2*Δ*1, met15*Δ*200, trp1*Δ*63, ura3-167* |
| JS241 | JS209 *MAT*α*, his3*Δ*200, leu2*Δ*1, met15*Δ*200, trp1*Δ*63, ura3-167, Ty1-MET15* |
| JS237 | JS209 *MAT*α*, his3*Δ*200, leu2*Δ*1, met15*Δ*200, trp1*Δ*63, ura3-167, RDN1::Ty1-* |
| | *MET15* |
| JS218 | JS237 *MAT*α*, his3*Δ*200, leu2*Δ*1, met15*Δ*200, trp1*Δ*63, ura3-167, RDN1::Ty1-* |
| | *MET15, sir2::HIS3* |
| YDS1583 | JS237 *MAT*α*, his3*Δ*200, leu2*Δ*1, met15Δ200, trp1*Δ*63, ura3-167, RDN1::Ty1-* |
| | *MET15, LEU2*/*SIR2* |

Replicative life span determination was performed by micromanipulation as described (25'). A minimum of 40 cells were examined per experiment and each experiment was performed at least twice independently. Statistical significance of life span differences was determined using the Wilcoxon rank sum test. Differences are stated to be significant when the confidence is higher than 95%.

GFP fluorescence was quantified by fluorescence-activated cell sorting (FACS) using a FACSCalibur flow cytometer (Becton Dickinson, CA) as described (45'). For G1-arrest experiments, cells were treated with 10 µg/ml alpha factor for 3 hours. DNA content was determined by FACS analysis of fixed cells stained with propidium iodide (Sigma) as described (45'). Typically 20,000 cells were analyzed per sample. Data acquisition and analysis were performed using CELLQuest software (Becton Dickenson).

*Fluorescence Microscopy and Chomatin immunoprecipitation-* GFP fluorescence was visualized in live cells grown to log phase in synthetic complete (SC) medium (1.67% yeast nitrogen base, 2% glucose, 40 mg/liter each of histidine, uridine, tryptophan, adenine and leucine). Images were captured using a Nikon Eclipse E600 microscope at a magnification of 1000X and analyzed with Scion Image software. Chromatin immunoprecipitation (ChIP) was performed as described (45') using the primer pairs listed in Table 2 (46'). PCR reactions were carried out in a 50 µl volume using a 1/5000 or a 1/12500 dilution of input DNA from precleared whole-cell extracts and a 1/50 dilution of immunoprecipitated DNA. PCR parameters were as follows. For *CUP1* and 5S rDNA primer pairs, 26 cycles of PCR were performed with an annealing temperature of 55°C. For Tel 0.6, Tel 1.4 and *HM* primer pairs 32 cycles at an annealing temperature of 50°C were used. PCR products were separated by gel electrophoresis on a 2.3% agarose gel and visualized by ethidium bromide staining.

**Table 4. Oligonucleotide Sequences**

| Oligonucleotide | Sequence |
|---|---|
| TEL-0.6.Fwd | CAGGCAGTCCTTTCTATTTC |
| TEL-0.6.Rev | GCTTGTTAACTCTCCGACAG |
| TEL-1.4.Fwd | AATGTCTTATCAAGACCGAC |
| TEL-1.4.Rev | TACAGTCCAGAAATCGCTCC |
| RDN-5S.Fwd | GAAAGGATTTGCCCGGACAGTTTG |
| RDN-5S.Rev | CTTCTTCCCAGTAGCCTGTTCCTT |
| HMR-YA/ZL.Fwd | GTGGCATTACTCCACTTCAAGTAAG |
| HMR-YA/ZL.Rev | CAAGAGCAAGACGATGGGG |
| CUP1-Fwd | TTTTCCGCTGAACCGTTCCA |
| CUP1-Rev | CATTGGCACTCATGACCTTC |

*In vitro deacetylation assays-* Recombinant GST tagged yeast Sir2p (gift of D. Moazed) and recombinant human SIRT1 (47') were assayed for deacetylase activity using the HDAC Fluorescent Activity Assay/Drug Discovery Kit (AK-500, BIOMOL Research Laboratories). This assay system allows detection of a fluorescent signal upon deacetylation of a histone substrate when treated with developer. Fluorescence was measured on a fluorometric reader (Cytofluor II 400 series PerSeptive Biosystems) with excitation set at 360 nm and emission detection set at 460 nm. Reactions consisted of either 5 µg of GST-Sir2 or 2.5 µg of SIRT1, incubated with 250 µM acetylated histone substrate, 1 mM DTT and a range of NAD⁺ concentrations as described, in 50 µl of assay buffer. Reactions with the yeast and human proteins were carried out at 30°C and 37°C respectively for 30 minutes.

For inhibitor assays, reactions were performed in the presence of 200 µM NAD⁺ and either nicotinamide (0, 50, 150 or 300 µM) (Sigma), or 50 µM of the following inhibitors; nicotinic acid (Sigma), sirtinol, M15 (Chembridge), splitomicin (47), TSA (BIOMOL).

### RESULTS

*Nicotinamide abolishes silencing at the rDNA, telomeres and mating type loci.* Nicotinamide is a product of Sir2 deacetylation and is a key substrate in the NAD⁺ salvage pathway. Based on our previous observation that manipulation of NAD⁺ biosynthesis can influence Sir2 dependent activities (see, Example 1), we wished to examine what effect NAD⁺ precursors would have on silencing. Strains with either an *ADE2* or *MET15* marker integrated at the rDNA locus (*RDN1*) were examined. Silencing of *ADE2* results in the accumulation of a red pigment on plates with limiting adenine, whereas silencing of *MET15* leads to production of a brown pigment on Pb²⁺-containing medium. We used two marker genes to ensure that the effects we observed were not simply due to changes in adenine or methionine biosynthesis. Strains with a single extra copy of *SIR2* (*2X SIR2*) or lacking *SIR2* (*sir2::TRP1*), were included as controls for increased silencing and lack of silencing, respectively. As shown in Figure 8A, when grown in the presence of 5 mM nicotinamide, silencing is completely abolished. Silencing of an *ADE2* marker at this locus was similarly abolished by addition of nicotinamide.

To test whether the effect of nicotinamide is specific to the rDNA or whether it influences other heterochromatic regions, we examined silencing at telomeres. To monitor telomeric silencing, we used a strain in which the *ADE2* gene is integrated at the subtelomeric (Y') region of the right arm of chromosome V (22'). On plates with limiting adenine, colonies have red/white sectors due to variegated expression of the *ADE2* marker. In the presence of 5 mM nicotinamide colonies were white, demonstrating a complete loss of repression (Fig, 8B). We also monitored silencing of mating type genes and found that nicotinamide completely abrogated silencing at this locus as well.

Nicotinic acid, an intermediate in the NAD⁺ salvage pathway, is structurally similar to nicotinamide (see Fig. 9B). Nicotinic acid is taken up efficiently by yeast cells and a specific transporter for this compound, Tna1, was recently identified (48',49'). In each of the above assays, we examined the effect of 5 mM nicotinic acid on Sir2-dependent silencing and in each case found that nicotinic acid had no effect.

*Nicotinamide increases genomic instability and shortens yeast life span.* We wished to determine whether the above loss of silencing was due to inhibition of Sir2 activity, in which case nicotinamide-treated cells should mimic a *sir2*Δ strain. Yeast lacking a functional Sir2 show increased frequencies of rDNA recombination. The loss of an *ADE2* marker at the rDNA locus was monitored in wild type, *2X SIR2* and *sir2* strains, in the presence and absence of nicotinamide. As shown in Figure 9A, treatment of wild type and *2X SIR2* cells with nicotinamide increased the frequency of marker loss ∼7-fold, similar to that of a *sir2* mutant. Importantly, treatment of the *sir2* strain did not further increase recombination, arguing that the observed marker loss was due to inhibition of Sir2.

Instability of the rDNA locus has been shown to be a major cause of yeast replicative aging (25',26'). We therefore examined the effect of nicotinamide on yeast life span. Cells were grown for two days on fresh yeast YPD medium to ensure that they had fully recovered from conditions of calorie restriction prior to the assay. Daughter cells that emerged from previously unbudded mother cells were then micro-manipulated away and scored. Figure 9C shows representative life span curves of both wild type (triangles) and the short-lived *sir2* mutant (circles). Cells grown on medium containing 5 mM nicotinamide (closed diamonds) had an average life span ∼45% that of wild type, which was equivalent to that of the *sir2* mutant. Treatment of the *sir2* strain with nicotinamide did not further shorten life span (squares). In contrast to these results, we observed no detrimental effect on replicative life span in the presence of either 5 or 50 mM nicotinic acid (Fig. 9D, closed and open diamonds, respectively).

*Nicotinamide inhibits silencing in non-dividing cells.* The reestablishment of silent chromatin domains requires passage through S phase (50'), although the trigger does not appear to be DNA replication (51',52'). Experiments with a temperature-sensitive *SIR3* allele suggest that the presence of the Sir2/3/4 complex is required to maintain a silenced state throughout the cell cycle (50'). We have shown that nicotinamide derepresses silent domains in cycling cells and attenuates replicative life span. We wondered whether nicotinamide treatment could have a similar effect on silencing in non-cycling, G1-arrested cells. We used a strain containing a GFP reporter integrated at the *HMR* locus allowing us to quantify the effects of nicotinamide on *HM* silencing in single cells. We first validated the system in cycling cells. As shown in Figure 10A, GFP was not expressed in untreated cells due to the high degree of silencing at this locus. However, after 60 minutes in 5 mM nicotinamide we observed a dramatic increase in the level of expression, which became even more pronounced after 90 minutes (Fig. 10A, second and third panels respectively).

To gain a more quantitative measure of silencing, cells were analyzed by fluorescence activated cell sorting (FACS). The top two panels of Figure 10B show the GFP expression profiles of asynchronous cultures of *sir4* and wild type strains. Deletion of *SIR4* disrupts the telomeric and mating type loci SIR complexes, leading to a redistribution of Sir2 away from these sites and to the rDNA locus. Thus, the profile of the *sir4* strain represents complete derepression of the *HMR* locus. Figure 10B shows that growth of wild type cells in 5 mM nicotinamide leads to complete derepression of this locus (third panel), as compared to the *sir4* mutant. Cells treated with 5 mM nicotinic acid or the structurally related quinolinic acid (a substrate in the *de novo* NAD⁺ synthesis pathway) showed no increase in GFP expression (Fig. 10B, bottom two panels) demonstrating that the desilencing effect is specific to nicotinamide.

Using this assay system we could monitor the effects of nicotinamide on heterochromatin in non-cycling cells. A *MAT***a** strain containing the *GFP* transgene was deleted for the *HML*α locus to ensure that the cells did not escape G1-arrest due to the co-expression of a and α genes. After arrest in G1 by treatment with α factor, cells were exposed to 5 mM nicotinamide and examined by FACS every 30 min. Figure 10C shows the expression profiles of arrested cells, in the presence and absence of nicotinamide. Surprisingly, cells arrested in G1 showed a loss of silencing when treated with nicotinamide. Measurement of DNA content by FACS confirmed that the cells remained in G1 for the duration of the experiment (Fig 10C, right column). These results demonstrate that exogenous nicotinamide derepresses silent chromatin even in non-dividing cells and suggests that heterochromatin is an unstable and dynamic structure. This also indicates that continued deacetylation of histones is essential for the maintenance of silencing.

*Nicotinamide causes Sir2 to dissociate from telomeres and mating type loci but not from rDNA.* We have shown that nicotinamide derepresses heterochromatin at all three silent loci in yeast. Although the most likely explanation for our observations was that Sir2 is catalytically inactivated by nicotinamide, is was also possible that Sir2 was delocalized or that its expression was down-regulated. To address the latter possibility we determined Sir2 protein levels in the presence of nicotinamide (1-5 mM) and found that they were unaltered. Next, we examined the effect of nicotinamide on the localization of a GFP-tagged Sir2. Identical log-phase cultures were grown in the presence or absence of 5 mM nicotinamide for two hours, during which the localization of GFP-Sir2 was monitored by fluorescence microscopy. Under normal conditions, Sir2 can be visualized at distinct foci near the nuclear periphery, each focal point representing a cluster of several telomeres (53'). In a *sir2* mutant background, Sir3 is released from telomeres and shows a diffuse nuclear pattern (Fig 11A). This strain served as a reference for Sir delocalization. During growth in nicotinamide we observed no change in the Sir2-GFP pattern, even after two hours, a time at which treated cells show maximal derepression of silent loci (Fig. 11C and D)=. We also examined the two other members of the Sir silencing complex, Sir3 and Sir4. Figures 5E and G show the localization pattern of Sir3-GFP and GFP-Sir4 in untreated cells, respectively. Treatment with 5 mM nicotinamide for two hours did not alter the pattern of GFP fluorescence for either of these proteins (Figs. 11F and H). These results show for the first time that inhibition of Sir2 does not result in a gross relocalization of the SIR complex.

To more closely examine the association between Sir2 and silent loci in the presence of nicotinamide, we performed chromatin immunoprecipitation (ChIP) on both treated and untreated cells. A *sir2* mutant strain and the non-silenced *CUP1* gene served as controls. Figure 12 shows PCR products from input and immunoprecipitated DNA using a 5S rDNA-specific primer pair. Treatment of cells with 5 mM nicotinamide did not alter the amount of PCR product obtained using these primers (compare lanes 5 and 6), demonstrating that Sir2 remains associated with rDNA in the presence of this compound.

Next, we examined the association of Sir2 with the silent *HMRα* locus and DNA 0.6 and 1.4 kb from the right telomere of chromosome VI. In the presence of nicotinamide, no PCR product was obtained using primers specific for *HMR.* Similarly, the amount of product from obtained from nicotinamide-treated cells using primers specific for sub-telomeric DNA was equivalent to background. These results demonstrate that Sir2 is not associated with *HMR* or sub-telomeric DNA in cells treated with nicotinamide. This presumably reflects a fundamental difference in the roles of Sir2 in the RENT complex at the rDNA and in the heterotrimeric SIR complex at telomeres and mating type loci.

*Nicotinamide is a potent non-competitive inhibitor of both yeast Sir2 and human SIRT1 in vitro.* Since Sir2 was neither delocalized nor down-regulated in response to nicotinamide, the most plausible explanation for our results was that this compound acted as a direct inhibitor of Sir2 deacetylase activity. To further explore this, and to gain more insight into the mechanism of desilencing induced by nicotinamide, we directly measured Sir2 activity *in vitro* in the presence of varying amounts of this compound. We utilized a novel class III HDAC activity assay that generates a fluorescent signal upon deacetylation of a histone substrate. When incubated with acetylated substrate and NAD⁺, recombinant GST-tagged Sir2 gives a strong fluorescent signal 10-fold greater than no enzyme and no NAD⁺ controls. Using this assay, we tested the ability of nicotinamide to inhibit deacetylation in the presence of varying concentrations of NAD⁺. A double reciprocal Lineweaver-Burk plot of the data (Fig. 13A) shows that nicotinamide is a strong non-competitive inhibitor of this reaction. A similar result has recently been obtained for Hst2, a cytoplasmic Sir2 homologue (54'). We wished to determine whether the inhibitory effects of nicotinamide could be extended to the Sir2 homologues of higher eukaryotes. Thus, we examined whether nicotinamide could also inhibit human SIRT1 *in vitro.* Using recombinant SIRT1, we monitored deacetylation of the substrate in the presence of varying amounts of nicotinamide and NAD⁺. Similar to Sir2, a Lineweaver-Burk plot of the data shows that nicotinamide also inhibits SIRT1 in a non-competitive manner (Fig. 13B). These results imply that nicotinamide does not inhibit deacetylation by competing with NAD⁺ for binding to Sir2/SIRT1 and that nicotinamide and NAD⁺ can bind the enzyme simultaneously.

Recently several groups have isolated compounds that inhibit Sir2-like proteins both *in vitro* and *in vivo* (55',56'). Among these are sirtinol, M15 and splitomycin. These compounds were isolated in high-throughput phenotypic screens of small molecule libraries as inhibitors of silencing, though none has yet been examined for its ability to inhibit SIRT1 activity. To compare the efficacy of inhibition of these compounds to that of nicotinamide we measured recombinant SIRT1 activity in the presence of 50 µM of each of these inhibitors. We also included the class I/II HDAC inhibitor TSA as a negative control. As shown in Figure 13C, nicotinamide inhibited SIRT1 with an IC₅₀<50 µM, a value that was equal to, or lower than, that of all the other inhibitors we tested. Adding further support to our *in vivo* results, we showed that the structurally related compound, nicotinic acid, had no effect on the activity of SIRT1 in *vitro* (Fig. 13C).

### DISCUSSION

We have shown that nicotinamide, a product of the Sir2 deacetylation reaction, is a potent inhibitor of Sir2 activity both *in vivo* and *in vitro.* Addition of exogenous nicotinamide to yeast cells derepresses all three silent loci, increases instability at the ribosomal DNA locus and shortens yeast life span to that of a *sir2* mutant. rDNA instability and short life span phenotypes of nicotinamide-treated cells are not augmented by a *sir2* mutation indicating that these phenotypes are the consequence of Sir2 inhibtion. Importantly, these results also indicate that rDNA instability and life span are not influenced by the other yeast Sir2 family members, the Hst proteins.

We have recently shown that strains carrying extra copies of NAD⁺ salvage pathway genes show increased silencing and are long lived, yet they do not have increased steady-state NAD⁺ or NADH levels (see, Example 1). We hypothesized that the increased longevity is mediated by local increases in NAD⁺ availability or increased flux through the salvage pathway. The latter model implies that that there may be continual recycling of NAD⁺ to nicotinamide, via Sir2 family members and/or NMN adenylyl transferases. We show that nicotinamide abrogates silencing in G1 arrested cells, arguing that Sir2 activity is required constitutively for the maintenance of heterochromatin and that Sir2 consumes NAD⁺ even in non-cycling cells. This is consistent with the recent finding of Bedelov et al. that the *MATα* gene at the silenced *HML* locus is expressed in G1 cells treated with splitomycin (56').

Addition of nicotinamide to cells does not alter the localization pattern of any of the Sir-GFP fusion proteins we examined (Fig. 11). This suggests that there are interactions that maintain the localization of Sir2 independently of its activity. Closer examination using ChIP shows that while Sir2 is still bound to the rDNA, it no longer associates with either telomeres or mating type loci in the presence of this compound (Fig. 12). It has previously been shown that Net1, the DNA binding subunit of the RENT complex, can associate with chromatin independently of Sir2 (57'). These findings indicate that this complex can assemble on ribosomal DNA in the absence of Sir2 deacetylase activity. In contrast, we show that the heterotrimeric Sir2/3/4 complex can not assemble on chromatin in the absence of Sir2 catalytic activity. These results are consistent with recent data from two other groups using catalytically inactive Sir2 mutants (46',58'). Both groups find that mutation of the conserved histidine in the catalytic domain (His-364) prevents Sir2 from interacting with telomeres and mating type loci *in vivo.* However, there remains the possibility that these mutations also affect the ability of Sir2 to interact with other proteins. Our results show conclusively that the deacetylase activity of Sir2 is required for its proper association with telomeres and mating type loci.

We have shown that nicotinamide strongly inhibits the deacetylase activity of both yeast Sir2 and the human homologue, SIRT *in vitro.* The fact that nicotinamide acts non-competitively to inhibit Sir2 suggests that this compound does not compete with NAD⁺ for binding. Examination of the reaction mechanism for Sir2 deacetylation and the crystal structure of an archeal Sir2 homologue provides clues as to a possible mechanism of inhibition. Sir2-catalyzed deacetylation consists of two hydrolysis steps which are thought to be coupled. Cleavage of the glycosidic bond connecting nicotinamide to the ADP-ribose moiety of NAD⁺ is followed by cleavage of the C-N bond between an acetyl group and lysine. A recent structural analysis indicates that Sir2 enzymes contain two spatially distinct NAD⁺ binding *sites* (the B site and the C site), both of which are involved in catalysis (59'). The authors propose that in the presence of an acetyl lysine, NAD⁺ bound to the B site can undergo a conformational change bringing the nicotinamide group in proximity to the C site where it is cleaved. The ADP-ribose product of this reaction then returns to the B site where deacetylation of the acetyl lysine occurs. We propose that at elevated concentrations, nicotinamide binds to and blocks the internal C site, which prevents the conformational change and subsequent cleavage of NAD⁺. This would explain the non-competitive nature of the mode of inhibition of this compound.

We have shown that the potency of nicotinamide rivals that of the most effective library-isolated compounds used in our assay. The fact that SIRT1 is inhibited by such low concentrations of nicotinamide *in vitro,* raises the possibility that this mode of inhibition may be physiologically relevant. Levels ofnicotinamide in mammalian tissues have been reported to lie in the range of 11-440 µM (39' ,60'-62'). Recently, levels of nicotinamide in cerebrospinal fluid were determined with high accuracy to be 54.2 µM (63'), a value which is similar to the IC₅₀ for nicotinamide reported here. We propose that fluctuations in cellular nicotinamide levels may directly control the activity of Sir2 proteins *in vivo.* These fluctuations may, in turn, be regulated by enzymes involved in nicotinamide metabolism.

The yeast *PNCI* gene encodes a nicotinamidase that is situated in a key position to regulate NAD⁺-dependent deacetylases. By converting nicotinamide into nicotinic acid, Pncl may reduce levels of this inhibitor and stimulate the rate at which NAD⁺ is regenerated (see Fig.7). Interestingly, *PNC1* is one of the most highly induced genes in response to stress and conditions that resemble calorie restriction (64',65'). Furthermore, *PNC1* encodes the only salvage pathway enzyme whose transcript undergoes cell-cycle dependent fluctuations (66'). Levels of *PNC1* are highest in MIG1 and drop off sharply in S-phase. Interestingly, this coincides with the establishment of Sir-dependent silencing (51',52',67'), These facts raise the possibility that high levels of Pnc1 induce silencing after S-phase or under conditions of stress and calorie restriction by removing the inhibitory effects of nicotinamide. Our previous finding, that a single extra copy of *PNC1* increases Sir2-dependent silencing (see, Example 1), adds further support to this model. It will be interesting to determine if intracellular nicotinamide levels change during the cell cycle, stress or calorie restriction.

Nicotinamide and nicotinic acid are used at high doses (up to 10 g/day) to self-treat a wide variety of conditions (41'). Both are considered forms of vitamin B3 and are often used interchangeably, however nicotinamide has become preferred in many cases due to an apparent lack of side effects. In addition, nicotinamide is currently in trials as a therapy to prevent cancer recurrence and insulin-dependent (type I) diabetes. Our results, which clearly demonstrate that nicotinamide can disrupt heterochromatin, even in non-cycling cells, raise the concern that there may be deleterious consequences of long-term nicotinamide therapy in humans.

### REFERENCES

1'. Courey, A. J., S. (2001) Genes Dev 15(21), 2786-96
2'. Moazed, D. (2001) Mol Cell 8(3), 489-98.
3'. Gasser, S. C. M. (2001) Gene 279(1), 1-16
4'. Eberharter, A. B., PB. (2002) Embo Reports 3(3), 224-9
5'. Kuo, M. A., CD. (1998) Bioessays 20(8), 615-26
6'. Bernstein, B. E., Tong, J. K., and Schreiber, S. L. (2000) Proc Natl Acad Sci U S A 97(25), 13708-13.
7'. Fischle, W. K., V. Dequiedt, F. Verdin, E. (2001) Biochem Cell Biol79(3), 337-48
8'. Marks, P. R., R A. Richon, V M. Breslow, R. Miller, T. Kelly, W K. (2001) Nature Rev Cancer1(3), 194-202
9'. Yoshida, M. F., R. Nishiyama, M. Komatsu, Y. Nishino, N. Horinouchi, S. (2001) Cancer Chemother Pharmacol 48(suppl), S20-6
10'. Smith, J. S., Brachmann, C. B., Celic, I., Kenna, M. A., Muhammad, S., Starai, V. J., Avalos, J. L., Escalante-Semerena, J. C., Grubmeyer, C., Wolberger, C., and Boeke, J. D. (2000) Proc Natl Acad Sci U S A 97(12), 6658-63.
11'. Tanner, K. G., Landry, J., Sternglanz, R., and Denu, J. M. (2000) Proc Natl Acad Sci U S A 97(26), 14178-82*.*
12'. Landry, J., Sutton, A., Tafrov, S. T., Heller, R. C., Stebbins, J., Pillus, L., and Sternglanz, R. (2000) Proc Natl Acad Sci US A 97(11), 5807-11.
13'. Imai, S., Armstrong, C. M., Kaeberlein, M., and Guarente, L. (2000) Nature 403(6771), 795-800
14'. Brachmann, C. B., Sherman, J. M., Devine, S. E., Cameron, E. E., Pillus, L., and Boeke, J. D. (1995) Genes Dev 9(23), 2888-902.
15'. Tanny, J. C., and Moazed, D. (2001) Proc Natl Acad Sci U SA 98(2), 415-20.
16'. Rine, J. H. I. (1987) Genetics 116(1), 9-22
17'. Wood, J. G., and Sinclair, D. A. (2002) Trends Pharmacol Sci 23(1), 1-4.
18'. Strahl-Bolsinger S, H. A., Luo K, Grunstein M. (1997) Genes Dev (11), 1
19'. Hecht, A. S.-B. S., Grunstein M. (1996) Nature 383(6595), 92-6
20'. Ghidelli, S. D. D., Dhillon N, Kamakaka RT. (2001) EMBO 20(16), 4522-35 5
21'. Shou, W., Sakamoto, K. M., Keener, J., Morimoto, K. W., Traverso, E. E., Azzam,
R., Hoppe, G. J., Feldman, R. M. R., DeModena, J., Moazed, D., Charbonneaux, H., Nomura, M., and Deshaies, R. J. (2001) Mol. Cell. 8(1), 45-55
22'. Gottschling, D. E., Aparicio, O. M., Billington, B. L., and Zakian, V. A. (1990) Cell 63(4), 751-62.
23'. Smith, J. S., and Boeke, J. D. (1997) Genes Dev 11(2), 241-54.
24'. Bryk, M., Banerjee, M., Murphy, M., Knudsen, K. E., Garfinkel, D. J., and Curcio, M. J. (1997) Genes Dev 11(2), 255-69.
25'. Sinclair, D. A., and Guarente, L. (1997) Cell 91(7), 1033-42.
26'. Kaeberlein, M., McVey, M., and Guarente, L. (1999) Genes Dev 13(19), 2570-80.
27'. Park, P. U., Defossez, P. A., and Guarente, L. (1999) Mol Cell Biol 19(5), 3848-56
28'. Sinclair, D. A., Mills, K., and Guarente, L. (1998) Trends Biochem Sci 23(4), 131-4.
29'. Gottlieb, S., and Esposito, R. E. (1989) Cell 56(5), 771-6.
30'. Kennedy, B. K., Austriaco, N. R., Jr., Zhang, J., and Guarente, L. (1995) Cell 80(3), 485-96.
31'. Lin, S. J., Defossez, P. A., and Guarente, L. (2000) Science 289(5487), 2126-8.
32'. Anderson, R. M., Bitterman, K. J., Wood, J. G., Medvedik, O., Cohen, H., Lin, S. S., Manchester, J. K., Gordon, J. I., and Sinclair, D. A. (2002) J Biol Chem 277(21), 18881-90.
33'. Tissenbaum, H. A., and Guarente, L. (2001) Nature 410(6825), 227-30.
34'. Vaziri, H., Dessain, S. K., Eaton, E. N., Imai, S. I., Frye, R. A., Pandita, T. K., Guarente, L., and Weinberg, R. A. (2001) Cell 107(2), 149-59*.*
35'. Luo, J., Nikolaev, A. Y., Imai, S., Chen, D., Su, F., Shiloh, A., Guarente, L., and Gu, W. (2001) Cell 107(2), 137-48.
36'. Moazed, D. (2001) Curr Opin Cell Biol 13(2), 232-8.
37'. Sauve, A. A., Celic, I., Avalos, J., Deng, H., Boeke, J. D., and Schramm, V. L. (2001) Biochemistry 40(51), 15456-63.
38'. Borra, M. T., O'Neill, F. J., Jackson, M. D., Marshall, B., Verdin, E., Foltz, K. R., and Denu, J. M. (2002) J Biol Chem 277(15), 12632-41.
39'. Dietrich, L. S. (1971) AmerJClin Nut 24, 800-804
40'. Kaanders, J. P. L., Marres HA, Bruaset I, van den Hoogen FJ, Merkx MA, van der Kogel AJ. (2002) Int J Radiat Oncol Biol Phys 52(3), 769-78
41'. Knip, M. D. L, Moore WP, Gillmor HA, McLean AE, Bingley PJ, Gale EA. (2000) Diabetologia 43(11), 1337-45
42'. Foster, J. W., Park, Y. K., Penfound, T., Fenger, T., and Spector, M. P. (1990) J Bacteriol 172(8), 4187-96.
43'. Ghislain, M., Talla, E., and Francois, J. M. (2002) Yeast 19(3), 215-224.
44'. Keil, R. L., and Mc Williams, A. D. (1993) Genetics 135(3), 711-8.
45'. Mills, K. D., Sinclair, D. A., and Guarente, L. (1999) Cell 97(5), 609-20.
46'. Hoppe, G. T. J., Rudner AD, Gerber SA, Danaie S, Gygi SP, Moazed D. (2002) Mol Cell Biol 22(12), 4167-80
47'. Langley, E. P. M., Faretta M, Bauer UM, Frye RA, Minucci S, Pelicci PG, Kouzarides T. (2002) EMBO J 21(10), 2383-2396
48'. Llorente, B., and Dujon, B. (2000) FEBS Lett 475(3), 237-41.
49'. Sandmeier, J. J., Celic, I., Boeke, J. D., and Smith, J. S. (2002) Genetics 160(3), 877-89.
50'. Miller, A. N. K. (1984) Nature 312(5991), 247-51
51'. Kirchmaier, A. L., and Rine, J. (2001) Science 291 (5504), 646-50.
52'. Li, Y. C., Cheng, T. H., and Gartenberg, M. R. (2001) Science 291 (5504), 650-3.
53'. Gasser, S. M., Gotta, M., Renauld, H., Laroche, T., and Cockell, M. (1998) Novartis Found Symp 214, 114-26
54'. Landry, J., Slama, J. T., and Sternglanz, R. (2000) Biochem Biophys Res Commun 278(3), 685-90.
55'. Grozinger, C. C. E., Blackwell HE, Moazed D, Schreiber SL. (2001) JBiol Chem 276(42), 38837-43
56'. Bedalov, A., Gatbonton, T., Irvine, W. P., Gottschling, D. E., and Simon, J. A. (2001) Proc Natl Acad Sci US A 98(26), 15113-8.
57'. Straight, A. F., Shou, W., Dowd, G. J., Turck, C. W., Deshaies, R. J., Johnson, A. D., and Moazed, D. (1999) Cell 97(2), 245-56.
58'. Armstrong, C. K. M., Imai SI, Guarente L. (2002) Mol Biol Cell 13(4), 1427-38
59'. Min, J. L. J., Sternglanz R, Xu RM. (2001) Cell 105(2), 269-79
60'. Hoshino, J., Schluter, U., and Kroger, H. (1984) Biochim Biophys Acta 801(2), 250-8.
61'. Ijichi, H. A. I., A. Hataishi, O. (1966) J Biol Chem 241, 3701
62'. Hagino, Y. L., J. Henderson, M. (1968) J Biol Chem 243, 4980
63'. Smythe, G. A., Braga, O., Brew, B. J., Grant, R. S., Guillemin, G. J., Kerr, S. J., and Walker, D. W. (2002) Anal Biochem 301(1), 21-6.
64'. Gasch, A. P., Spellman, P. T., Kao, C. M., Carmel-Harel, O., Eisen, M. B., Storz, G., Botstein, D., and Brown, P. O. (2000) Mol Biol Cell 11 (12), 4241-57.
65'. Moskvina, E. S. C., Maurer CT, Mager WH, Ruis H. (1998) Yeast 14(11), 1041-50
66'. Spellman, P. T., Sherlock, G., Zhang, M, Q., Iyer, V. R., Anders, K., Eisen, M. B., Brown, P.O., Botstein, D., and Futcher, B. (1998) Mol Biol Cell 9(12), 3273-97.
67'. Laurenson, P., and Rine, J. (1992) Microbiol Rev 56(4), 543-60. 68'. Barton, A. (1950) J Gen Microbiol 4, 84-86
69'. Kennedy, B. K., Austriaco, N. R., Jr., and Guarente, L. (1994) J Cell Biol 127(6 Pt 2), 1985-93.

### Example 3: Nicotinamide, but not nicotinic acid, bind to the C pocket of Sir2

The nicotinamide was docked in the crystal structure of Sir2 from Archaeoglobus fulgidus (Sir2-Afl) bound to NAD⁺ (Protein Data Bank ID code 1ICI, Min et al. (2001). Crystal structure of a SIR2 homolog-NAD complex. Cell 105, 269-279). It was first manually docked in the C site of Sir2-Afl using QUANTA (MSI, Inc.). Subsequently, an energy minimization calculation was done with CHARMM (Brooks et al, (1983) J. Comput. Chem. 4, 187-217) with harmonic constraint on Sir2-Afl and NAD⁺ (F = 2.4 Kcal/mol Å²). Fig. 14A-C were made with PYMOL (DeLano, W.L. The PyMOL Molecular Graphics System (2002) DeLano Scientific, San Carlos, CA, USA).

These studies indicate that nicotinamide inhibits Sir2 (see Figs. 14 A-C) and that nicotinic acid does not inhibit Sir2 because the presence of residue D101 (i.e., acidic) prevents nicotinic acid to dock into the C pocket of Sir2.

### Example 4: PNC1 mediates lifespan extension

As shown in Fig. 17A, *PNCI* catalyzes an amide hydrolysis, converting nicotinamide to nicotinic acid in the NAD⁺ salvage pathway (Fig. 17B). Most wild-type yeast strains have an average lifespan of 21-23 divisions, with a maximum lifespan of ~40 divisions. A wild-type strain that was calorie restricted (0.5% glucose) or heat stressed (37°C) exhibited a longer lifespan than an untreated control (2.0% glucose or 30°C, respectively) (Fig. 17C and D). The *sir2Δ* strain had a short lifespan, consistent with previous reports^{12,13}, and neither calorie restriction nor heat extended lifespan in this strain (Fig. 17C and D). The *pnc1Δ* strain did not exhibit a lifespan extension under either of these conditions, demonstrating that *PNC1* is necessary for lifespan extension.

Strikingly, under non-stressing conditions (2% glucose, 30°C), a strain with additional copies of *PNC1 (5xPNC1)* lived 70% longer than the wild-type and some cells lived over 70 divisions, which is the longest reported lifespan extension in this organism (Fig. 17E). Neither calorie restriction nor heat stress further increased the lifespan of the *5xPNC1* strain. Deletion of *SIR2* in the *5xPNC1* background shortened lifespan to that of the *sir2Δ* strain (Fig. 17E). The *pnc1Δ sir2Δ* double mutant had a lifespan similar to the *sir2Δ* mutant as well (Fig. 17E) and its lifespan was unaffected by glucose restriction. This indicates that *PNC1* and *SIR2* function in the same pathway and that *PNC1* increases lifespan via *SIR2.*

Thus, these results demonstrate that *PNC1* is necessary for lifespan extension by both calorie restriction and heat stress, and that additional *PNC1* is sufficient to mimic these stimuli. According to our model, additional copies of *PNC1* extend lifespan by depleting nicotinamide, thus relieving inhibition of Sir2.

### Example 5: PNC1 expression is increased in response to stress conditions

*S. cerevisiae* were incubated in different stress conditions and the level of expression of PNC was measured by conducted Western blots. The amount of PNC1 measured in yeast cells grown in 2.0% glucose complete medium (YPD) was set at 1. The

Table below and Fig. 18 show the fold induction in different growth conditions relative to this reference level of expression:

| **Culture conditions** | **Fold comparison** |
|---|---|
| 2.0% glucose complete meidum (YPD) | 1 |
| 0.5% glucose complete medium (YPD) | 15 |
| 0.1 % glucose complete medium (YPD) | 25 |
| Defined complete medium (SD) + amino acids | 5 |
| Defined complete medium (SD) - amino acids | 20 |
| Heat shocked in 2% YPD (37 degrees for 4 hours) | 20 |
| Osmotic stress (0.1 M NaCl) | 15 |

It was also shown that nitrogen restriction greatly induced PNC1 expression. Since all of the above "stress conditions," i.e., not 2.0% glucose complete medium (YPD) extend the life span of *S*. *cerevisiae* (caloric restriction), an increase in PNC1 correlates with an extended life span in every condition tested and known to extend yeast lifespan, including amino acid restriction, salt stress and heat stress (Fig. 18C). Analysis of genome-wide mRNA profiles of the stress response (Gash) showed that PNC 1 is one of the most highly responsive genes in response to stress and starvation in this organism. PNC1 levels were also greatly induced in cells carrying a cdc25-10 allele that mimics calorie restriction by lowering cAMP (Fig. 18B).

To test whether this response was specific to environmental stress, we examined Pncl levels in a strain deleted for *BNA6*/*QPT1,* which is required for the *de novo* synthesis of NAD⁺ but not life span extension by calorie restriction , In this mutant Pnc1 levels were unaltered (Fig. 18B). Pnc1 activity in extracts from treated cells correlated with Pnc1 levels in Western blots (Fig. 18D), demonstrating that these cells have increased rates of nicotinamide hydrolysis. Thus, *PNC1* is the first yeast longevity gene whose expression is modulated by stimuli that extend lifespan.

Accordingly, methods in which the level of PNC1 is increased to extend the life span of cells or protect them against stresses, as further described herein, mimics the natural events in cells.

### Example 6: Additional PNC1 confers resistance to acute stress

Given the strong link between longevity and stress resistance in other species, we tested whether additional *PNC1* could also confer resistance to a range of stresses. A well-characterized test of stress resistance in yeast is the ability of cells to tolerate high concentrations of salt²⁶. We found that the *5xPNC1* strain was dramatically more resistant than wild-type to high levels of both NaCl (600 mM) and LiCl (200 mM) (Fig. 19A). We also tested survival following DNA damage by UV irradiation (5 mJ/cm²) and found again that additional *PNC1* conferred resistance (Fig. 19B). Because mitochondrial DNA damage has been implicated in mammalian aging²⁷, we also examined the ability of additional *PNC1* to protect against this type of stress. Under conditions of obligate respiration (3% glycerol as carbon source), *5xPNC1* cells were more resistant than wild-type to mitochondrial mutagenesis by ethidium bromide (Fig. 19C). The increased resistance of the *5xPNC1* strain to LiC1 was dependent on *SIR2.* Strikingly, the resistance of this strain to NaCl, UV and ethidium bromide was independent of *SIR2* (Figs. 19A-C). These results demonstrate that *PNC1* promotes both longevity and stress resistance, and suggests that *SIR2* is not the only downstream effector of this gene. It is thus likely that nicotinamide regulates proteins other than Sir2.

### Example 7: Cellular localization of PNC1 under a variety of stress conditions

We have previously shown that two enzymes in the NAD⁺ salvage pathway, Nptl (nicotinamide phosphoribosyltransferase) and Nma2 (nicotinate mononucleotide adenylyltransferase), are concentrated in the nucleus²³. We investigated whether Pnc1, another salvage pathway enzyme, had a similar cellular distribution. Surprisingly, on complete 2% glucose medium, Pncl-GFP was observed in the cytoplasm, the nucleus and in 3 - 6 discrete cytoplasmic foci per cell (Fig. 20A). Calorie-restricted or stressed cells showed a dramatic increase in the intensity of fluorescence, consistent with the Western data. Interestingly, under conditions of amino acid restriction or salt stress, this pattern was altered, with the fluorescence being predominately localized to the foci (Fig 20B). This suggests that Pnc1 localization is regulated in distinct ways by different stresses.

To determine the identity of the foci, we searched for cellular markers that co-localized with Pnc1-GFP. We observed significant overlap with a peroxisomally-targeted red fluorescent protein (RFP) (Fig. 20C). Furthermore, the Pnc1-GFP foci were no longer observed in a *pex6Δ* mutant, which is unable to form peroxisomes (Fig 20D). Because our stress studies indicated that the localisation of Pnc1 to peroxisomes might be regulated, we sought to identify the transporter responsible for its import into this organelle. Although Pex5 imports the vast majority of peroxisomal proteins, the localisation of Pnc1 to peroxisomes required the lesser-utilised transporter Pex7 (Fig. 20D). The localisation of Pnc 1 to sites outside the nucleus is consistent with our stress results demonstrating that nicotinamide regulates proteins other than Sir2. The peroxisomal localisation is of particular interest because these organelles are a major source of reactive oxygen species and have been implicated in mammalian aging^{28,29}. In addition, a number of crucial steps in lipid metabolism occur in peroxisomes and lipid signaling has recently been linked to salt tolerance²⁶. The salt resistance of additional *PNC1* maybe the result of a peroxisomal function of Pnc1.

### Example 8: Life span and stress resistance are negatively regulated by intracellular nicotinamide

One prediction of our model is that any manipulation of intracelluar nicotinamide levels should be sufficient to alter Sir2 activity. A common indicator of Sir2 activity is the extent to which a reporter gene inserted at the rDNA *(RDN1)* is silenced. To exclude the possibility that NAD⁺ levels were responsible for any silencing effect, we sought to manipulate intracellular nicotinamide levels using a gene outside the NAD⁺ salvage pathway. In humans, the major route of nicotinamide metabolism is through nicotinamide N-methlytransferase (NNMT)³⁰ . NNMT converts nicotinamide to N'-methylnicotinamide, which is excreted via the kidneys³¹. By homology we identified the *S. cerevisiae* NNMT gene, which we have named *NNT1.* Nntl is 23% identical to a mammalian NNMT core domain³⁰ and contains the four signature motifs of 5-adenosylmethionine(SAM)-dependent methyltranferases³².

Deletion of *NNT1* caused a desilencing phenotype similar to deletion of *PNC1³³* (Fig. 21A). These results are consistent with our finding that rDNA silencing is abrogated in the presence of exogenous nicotinamide (Example 2). As predicted, strains with additional *NNT1* showed increased silencing, similar to strains with additional *PNC1*²³. We conclude that lifespan, stress resistance and Sir2 activity can be regulated by changes in intracellular nicotinamide and levels of NNT1 . It is worth noting that although *NNT1* can mimic *PNC1* phenotypes, unlike *PNC1,* its expression is not apparently modulated by stimuli that extend lifespan²⁵.

We have identified *PNC1* as a calorie restriction- and stress-responsive gene that increases lifespan and stress resistance of cells by depleting intracellular nicotinamide (Figure 21 B). We show that lifespan extension by calorie restriction is the result of an active cellular defense response coordinated by a specific regulatory gene. An attractive feature of this mechanism is that it is not based on the modulation of NAD⁺, an essential cofactor involved in cellular homeostasis.

We do not yet know how a gene involved in nicotinamide metabolism confers resistance to numerous acute stresses. Presumably the benefits of increased Pnc 1 come at an evolutionary cost but we have yet to identify any selective disadvantage. Both our stress and localisation results imply the existence of multiple nicotinamide-regulated effectors. Based on the enzymology of Sir2 inhibition by nicotinamide (Example 2 and ³⁴), proteins that cleave NAD⁺ in a two-step reaction are plausible candidates. Examples include the homologues of Sir2 (Hst1-4) and Tpt1, an NAD⁺-dependent 2'-phosphotransferase that facilitates the unfolded protein response³⁵. Expression profiling of cells with altered nicotinamide metabolism should help identify these effectors and the downstream pathways of stress resistance.

In mammals, there is evidence for a link between nicotinamide metabolism and stress resistance. Poly(adenosine diphosphate-ribose) polymerase-1 (PARP) is a nuclear enzyme that cleaves NAD⁺ to covalently attach poly(ADP-ribose) to acceptor proteins. This two-step reaction generates nicotinamide, which exerts an inhibitory effect on PARP-1 I allowing for autoregulation³⁶. PARP enzymes have been implicated in numerous cellular functions including DNA break repair, telomere-length regulation, histone modification, and the regulation at the transcriptional level of key proteins including ICAM-1 and nitric oxide synthase³⁶. Our results suggest that PARP enzymes might be regulated by nicotinamide metabolism as part of a general stress response. Nicotinamide also inhibits human *SIRTI* both *in vitro* (Example 2) and *in vivo*¹⁷. *SIRTI* negatively controls p53 activity, indicating that nicotinamide levels may regulate apoptosis and DNA repair^{17,18}. Consistent with this, the expression of NNMT in human cells and tissues correlates with tumorigenesis³⁷ and radioresistance³⁸.

### Example 9: Materials and Methods for Examples 4-8

Media and Strains: All strains were grown at 30°C in complete 2.0% (w/v) glucose (YPD) medium except where stated otherwise. In all experiments, we ensured that auxotrophic markers were matched between strains by integrating empty vector. All deletions were generated using a kanMX6 PCR-based technique³⁹ and confirmed by PCR. Additional copies of *PNC1* were integrated as previously described²³. The entire open reading frame and 700 bases of upstream sequence of *NNT1 (YLR285w)* were cloned from genomic DNA by PCR into pSP400 ⁴⁰, sequenced, and integrated into the yeast genome as described previously²³. The copy number of integrated genes was determined by Southern blotting. A GFP cassette was introduced in-frame at the 3' end of the native *PNC1* gene as previously described ³⁹. The RFP-PTS1 plasmid (pSG421) was a gift from S.J. Gould (Johns Hopkins U.). PSY316AT-derived strains were used for lifespan analysis and stress resistance assays. Strains derived from PSY316AT *(MATα, ura3-53 leu2-3,112 his3-Δ200 ade2-1,01 canl-100ADE2-TEL V-R): pnc1Δ* (YDS1741), *sir2Δ* (YDS1750), *5xPNC1* (YDS1853), 5*xPNCl sir2Δ* (YDS1851), *pnc1Δ sir2Δ* (YDS1853). W303-derived strains were used for Western blot analysis, fluorescence microscopy and *SIR2* dependent silencing assays. Strains derived from W303 *(MATa, ade2-1, leu2-3,1l2, canl-100, trpl1l, ura3-52, his3-11,15, RDNI::ADE2, RAD5)* include: *PNCI-GFP* (YDS1742), *pne1 Δ* (YDS1911), *nnt1Δ (YDS1747), 2xPNC1* (YDS1588), *2xNNT1* (YDS1926), *ADE2* (YDS1596). The following strains were derived from *PNCI-GFP* (YDS1742): *bna6Δ* (YDS1857), pSG421 (YDS1916),*pex6Δ* (YDS1869),*pex5Δ* (YDS1870) and *pex7Δ* (YDS1871). The *cdc25-10* strain was a gift from L Guarente (M.LT.).

Yeast assays were conducted as follows. Life span measurements were performed as previously described²³ except for the heat stress experiments where strains were incubated after each dissection at 37°C. Stress resistance assays were performed using mid-log phase cells. Silencing was assayed as previously described²³.

Protein expression analysis were conducted as follows. Strains were pretreated under the indicated conditions and grown to mid-log phase. Western blots were performed as described²³ using whole cell extracts (75 µg). Proteins were detected using anti-GFP antibodies (Santa Cruz) or anti-actin antibodies (Chemicon). Fluorescent microscopy images were captured at the same exposure (1 s) at 100x magnification with a Hamamatsu Orca100 CCD camera and processed with Openlab software.

Nicotinamidase activity assay was conducted as follows. Activity of Pnc1 in extracts obtained from pretreated mid-log phase cultures was determined as previously described ⁴¹. Briefly, 0.16 mg of protein were incubated with either 0 or 8 mM nicotinamide for 45 min at 30°C in a final volume of 400 µl consisting of 10 mM Tris pH 7.5, 150 mM - NaCl and 1 mM MgCl₂, Pnc1 activity was determined by measuring the final concentration of the reaction product, ammonia, using the Sigma ammonia diagnostic kit. Baseline ammonia was accounted for by subtracting a no nicotinamide control. Nicotinamidase activity was expressed as nmol ammonia produced/min/mg total protein. Pnc1 activity was obtained by subtracting the background value for the *pnc1 Δ* strain (0.06 ± 0.004 nmol/min/mg).

### References for Examples 4-9:

1. Masoro, E. J. Exp Gerontol 35, 299-305. (2000).
2. Masoro, E. J. Exp Gerontol 33, 61-6. (1998),
3. Kirkwood, T. B. & Holliday, R. Proc R Soc Lond B Biol Sci 205, 531-46. (1979).
4. Holliday, R. Food Bioessays 10, 125-7. (1989).
5. Kenyon, C. Cell 105, 165-168 (2001).
6. Guarente, L. & Kenyon, C. Nature 408, 255-62. (2000).
7. Kaeberlein, M. & Guarente, Genetics 160, 83-95. (2002).
8. Jiang et al. Faseb J 14, 2135-7. (2000).
9. Swiecilo et al. Acta Biochim Pol 47, 355-64 (2000).
10. Sinclair, D. A. Mech Ageing Dev in press. (2002).
11. Moazed, D. Curr Opin Cell Biol 13, 232-8. (2001).
12. Lin et al. Science 289, 2126-8. (2000).
13. Kaeberlein et al. Genes Dev 13, 2570-80. (1999).
14. Sinclair, D. A. & Guarente, L. Cell 91, 1033-42. (1997).
15. Tissenbaum, H. A. & Guarente, L. Nature 410, 227-30. (2001).
16. Rogina et al. Science, in press (2002).
17. Vaziri, H. et al. Cell 107, 149-59. (2001).
18. Luo, J. et al. Cell 107, 137-48. (2001).
19. Smith, J. S. et al. Proc Natl Acad Sci U S A 97, 6658-63. (2000).
20. Imai et al. Nature 403, 795-800 (2000).
21. Tanny, J. C. & Moazed, D. Proc Natl Acad Sci USA 98, 415-20. (2001).
22. Landry, J. et al. Proc Natl Acad Sci U S A 97, 5807-11. (2000).
23. Anderson, R. M. et al. J Biol Chem 277, 18881-90. (2002).
24. Bitterman et al. J. Biol, Chem. in press (2002).
25. Gasch, A. P. et al. Mol Biol Cell 11, 4241-57. (2000).
26. Betz et al. EurJBiochem 269, 4033-9. (2002).
27. Melov, S. Ann N YAcad Sci 908, 219-25. (2000).
28. Masters, C. J. & Crane, D. I. Mech Ageing Dev 80, 69-83. (1995).
29. Perichon et al. Cell Mol Life Sci 54, 641-52. (1998).
30. Aksoy et al. J Biol Chem 269, 14835-40. (1994).
31. Matsubara et al. Neurotoxicol Teratol 24, 593. (2002).
32. Niewmierzycka, A. & Clarke, S.J Biol Chem 274, 814-24. (1999).
33. Sandmeier et al. Genetics 160, 877-89. (2002).
34. Landry et al. Biochem Biophys Res Commun 278, 685-90. (2000).
35. Spinelli et al. J Biol Chem 274, 2637-44. (1999).
36. Virag, L. & Szabo, C. Pharmacol Rev 54, 375-429. (2002).
37. Lal, A. et al. Cancer Res 59, 5403-7. (1999).
38. Kassem et al. Int J cancer 101, 454-60. (2002).
39. Longtine, M. S. et al. Yeast 14, 953-61. (1998).
40. Mills et al. Cell 97, 609-20. (1999).
41. Ghislain et al. Yeast 19, 215-224. (2002).

### Example 10: Human nicotinamide methyltransferase (NMNAT) confers radioresistance in human cells

NMNAT (EC 2.1.1.1; CAS registry number 9029-74-7), which is also referred to as nicotinamide N-methyltransferase, is an enzyme that catalyzes the reaction S-adenosyl-L-methionine + nicotinamide = S-adenosyl-L-homocysteine + 1-methylnicotinamide (see also, Cantoni (1951) J. Biol. Chem. 203-216). Overexpression of human NMNAT in radiosensitive human cells was found to increase the radioresistance of the cells.

Furthermore, the present invention comprises the following embodiments:
1. A method for modulating the life span of a cell or its resistance to stress, comprising modulating the flux through the NAD+ salvage pathway in the cell.
2. The method of 1, wherein modulating the NAD+ salvage pathway comprises modulating the level or activity of a protein selected from the group consisting of NPT 1, PNC 1, NMA 1 and NMA2.
3. The method of 2, wherein modulating is increasing and the method comprises increasing the level or activity of a protein selected from the group consisting of NPT1, PNC1, NMA and NMA2.
4. The method of 3, comprising introducing into the cell at least one nucleic acid encoding a protein selected from the group consisting of NPT1, PNC1, NMA1 and NMA2 or at least a portion thereof sufficient for increasing the flux through the NAD+ salvage pathway in a cell.
5. The method of 4, comprising introducing into the cell at least one nucleic acid comprising at least 5 nucleotide sequences encoding one or more proteins selected from the group consisting of NPT1, PNC1, NMA1 and NMA2 or at least a portion thereof sufficient for increasing the flux through the NAD+ salvage pathway in a cell.
6. The method of 3, comprising introducing into the cell at least one protein selected from the group consisting of NPT1, PNC1, NMA1 and NMA2 or at least a portion thereof sufficient for increasing the flux through the NAD+ salvage pathway in a cell.
7. A method for modulating the life span of a cell or its resistance to stress, comprising modulating the level of nicotinamide in the cell.
8. The method of 7, wherein modulating is increasing and the method comprises contacting the cell with nicotinamide or an analog thereof.
9. The method of 2 or 7, wherein the lifespan of the cell is extended by at least about 40%.
10. The method of 1 or 7, wherein the cell is in vitro.
11. The method of 1 or 7, wherein the cell is a eukaryotic cell.
12. The method of 11, wherein the cell is a mammalian cell.
13. The method of 1 or 7, wherein the cell is a yeast cell.
14. The method of 1 or 7, wherein stress is a heatshock osmotic stress; a DNA damaging agent; inadequate salt level; inadequate nitrogen levels; or inadequate nutrient level.
15. The method of 1 or 7, wherein modulating the flux through the NAD+ salvage pathway occurs essentially without changing steady state levels of NAD+ and NADH.
16. A method for identifying a compound that modulates the life span of a cell or its resistance to stress, comprising (i) contacting a protein selected from the group consisting of NPT1, PNC1, NMA1, NMA2, NNMT, NAMPRT, NMNAT-1, and NMNAT-2 with a test compound for an amount of time that would be sufficient to affect the activity of the protein; and (ii) determining the activity of the enzyme, wherein a difference in the activity of the enzyme in the presence of the test compound relative to the absence of the test compound indicates that the test compound is a compound that modulates the life span of the cell or its resistance to stress.
17. A method for identifying a compound that modulates the life span of a cell or its resistance to stress, comprising (i) contacting a cell or a lysate comprising a transcriptional regulatory nucleic acid of a gene selected from the group consisting of NPT 1, PNC1, NMA 1, NMA2, NNMT, NAMPRT, NMNAT-1, and NMNAT-2 operably linked to a reporter gene, with a test compound for an amount of time that would be sufficient to affect the transcriptional regulatory nucleic acid; and (ii) determining the level or activity of the reporter gene, wherein a difference in the level or activity of the reporter gene in the presence of the test compound relative to the absence of the test compound indicates that the test compound is a compound that modulates the life span of the cell or its resistance to stress.
18. The method of 17 or 18, further comprising contacting a cell with the test compound and determining whether the life span of the cell or its resistance to stress has been modulated.
19. A method for identifying an inhibitor of the activity of a Sir2 family member comprising: (i) supplying a computer modeling application with a set of structure coordinates of a molecule or molecular complex, the molecule or molecular complex including at least a portion of a Sir2 family member comprising a C pocket; (ii) supplying the computer modeling application with a set of structure coordinates of a chemical entity; and (iii) determining whether the chemical entity is an inhibitor expected to bind to or interfere with the molecule or molecular complex, wherein binding to or interfering with the molecule or molecular complex is indicative of potential inhibition of the activity of the Sir2 family member.
20. The method of 19, wherein the chemical entity is an analog of nicotinamide.
21. A method for identifying an inhibitor of the activity of a Sir2 family member comprising (i) contacting a protein of the Sir2 family comprising at least the C pocket with a test compound for a time sufficient for the test compound to potentially bind to the C pocket of the protein of the Sir2 family; and (ii) determining the activity of protein; wherein a lower activity of the protein in the presence of the test compound relative to the absence of the test compound indicates that the test compound is an inhibitor of the activity of a Sir2 family member.
22. A method for treating or preventing a disorder that is associated with cell death or aging in a subject, comprising administering to a subject in need thereof an agent that increases the flux through the NAD+ salvage pathway or reduces nicotinamide levels in the cells susceptible to or subject to cell death or aging.
23. A method for treating or preventing a disorder in which reducing the life span of cells or rendering cells sensitive to stress is beneficial, comprising administering to a subject in need thereof an agent that decreases the flux through the NAD+ salvage pathway or increases nicotinamide levels in cells of the subject.

### Equivalents

Those skilled in the art will recognize, or be able to ascertain using no more than routine experimentation, many equivalents of the specific embodiments of the invention described herein. Such equivalents are intended to be encompassed by the following claims.

## Claims

1. An agent for use in increasing resistance to stress in a cell, wherein said agent increases the level or activity of an enzyme that metabolizes, degrades or inhibits nicotinamide in the cell.

2. The agent of claim 1, wherein the enzyme is a nicotinamidase enzyme or a nicotinamide phosphoribosyltransferase enzyme.

3. The agent of claim 1 or claim 2 wherein the enzyme is one or more selected from PNC1, NMA1, NMA2, NNMT, NAMPRT, NMNAT-1 or NMNAT-2.

4. The agent of claim 1, wherein the agent is at least one nucleic acid encoding an enzyme as claimed in any one of claims 1 to 3.

5. The agent of claim 1, wherein the agent is at least one protein selected from the group consisting of an enzyme as claimed in any one of claims 1 to 3.

6. An agent for use in increasing resistance to stress in a cell, wherein said agent decreases the level of nicotinamide in the cell, thereby increasing resistance to stress in the cell.

7. The agent of claim 1 or 6, wherein the cell is a eukaryotic cell.

8. The agent of claim 7, wherein the cell is a mammalian cell.

9. The agent of claim 1 or 6, wherein the cell is a yeast cell.

10. The agent of claim 1 or 6, wherein stress is a heatshock; osmotic stress; a DNA damaging agent; inadequate salt level; inadequate nitrogen levels; or inadequate nutrient level.

11. The agent of claim 1 or 6, wherein modulating the flux through the NAD+ salvage pathway occurs without changing steady state levels of NAD+ and NADH.

12. An *in vitro* method for increasing resistance to stress in a cell, comprising increasing the level or activity of an enzyme that metabolizes, degrades or inhibits nicotinamide in the cell.

13. A method for identifying a compound that modulates resistance to stress, comprising (i) contacting an enzyme selected from the group consisting of PNC1, NMA2, NMA2, NNMT, NAMPRT, NMNAT-1 or NMNAT-2 with a test compound for an amount of time that would be sufficient to affect the activity of the enzyme; and (ii) determining the activity of the enzyme, wherein a difference in the activity of the enzyme in the presence of the test compound relative to the absence of the test compound indicates that the test compound is a compound that modulates resistance to stress.

14. A method for identifying a compound that modulates resistance to stress, comprising (i) contacting a cell or a lysate comprising a transcriptional regulatory nucleic acid of a gene encoding an enzyme selected from the group consisting of PNC1, NMA1, NMA2, NNMT, NAMPRT, NMNAT-1 or NMNAT-2 operably linked to a reporter gene, with a test compound for an amount of time that would be sufficient to affect the transcriptional regulatory nucleic acid; and (ii) determining the expression level or activity of the reporter gene, wherein a difference in the expression level or activity of the reporter gene in the presence of the test compound relative to the absence of the test compound indicates that the test compound is a compound that modulates resistance to stress.

15. The method of claim 13 or 14, further comprising contacting a cell with the test compound and determining whether its resistance to stress has been modulated.

16. A pharmaceutical composition for increasing resistance to stress in a cell, comprising an agent which increases the level or activity of an enzyme that metabolizes, degrades or inhibits nicotinamide in the cell.
